# EUROPEAN PATENT APPLICATION

(11) **EP 3 978 605 A1**
(43) Date of publication of application: **06.04.2022**
(21) Application number: 21199536.0
(22) Date of filing: 28.04.2017
(51) Int. Cl.: C12N 9/50, C12N 9/36, C07K 14/46, C12N 15/62

(54) **ANTIMICROBIAL AGENTS AGAINST SALMONELLA BACTERIA**

(30) Priority: 28.04.2016 EP 16167543
(62) Divisional of application: 17723031.5
(71) Applicant: Lysando AG, 9497 Triesenberg (LI)
(72) Inventor: GRIESSL, Martin, 93155 Hemau (DE); BIEBL, Manfred, 93083 Obertraubling (DE); SCHIRMEIER, Eva, 93053 Regensburg (DE)
(74) Representative: Rückerl, Florian

(57) **Abstract**

The present invention relates to the field of antimicrobial agents active against *Salmonella* bacteria. In particular, the present invention relates to polypeptides comprising a first and a second amino acid sequence, wherein the first amino acid sequence is an endolysin, and wherein the second amino acid sequence is an antimicrobial peptide, cationic peptide, hydrophobic peptide, amphipathic peptide or sushi peptide, and wherein the polypeptide comprises at least one sequence selected from the group consisting of SEQ ID NO:1, SEQ ID NO:3, SEQ ID NO:4 and derivatives thereof. In addition, the present invention relates to nucleic acids encoding such polypeptides, vectors comprising such nucleic acids, and corresponding host cells. Finally, the present invention relates to applications of the inventive polypeptides, nucleic acids, vectors, and/or host cells, in particular in the pharmaceutical field and in the field of food and feed.

## Description

The present invention relates to the field of antimicrobial agents active against *Salmonella* bacteria. In particular, the present invention relates to polypeptides comprising a first and a second amino acid sequence, wherein the first amino acid sequence is an endolysin, and wherein the second amino acid sequence is an antimicrobial peptide, cationic peptide, hydrophobic peptide, amphipathic peptide or sushi peptide, and wherein the polypeptide comprises at least one sequence selected from the group consisting of SEQ ID NO:1, SEQ ID NO:3, SEQ ID NO:4 and derivatives thereof. In addition, the present invention relates to nucleic acids encoding such polypeptides, vectors comprising such nucleic acids, and corresponding host cells. Finally, the present invention relates to applications of the inventive polypeptides, nucleic acids, vectors, and/or host cells, in particular in the pharmaceutical field and in the field of food and feed.

Bacterial pathogens represent a significant threat for human health. Although various types of agents having bactericidal or bacteriostatic activity are known in the art (e.g. antibiotics), microbial resistance to these, in particular to antibiotics, is steadily increasing. One of the pathogens representing a health concern are bacteria of the genus *Salmonella.* A large number of infections caused by *Salmonella* bacteria are due to ingestion of contaminated food. Since increasing resistance renders the use of antibiotics in, e.g., feed additives problematic, there is a constant demand for new antimicrobial agents to control the number of *Salmonella* bacteria, e.g. in food and feed.

Bacteria of the genus *Salmonella* are Gram-negative bacteria. Gram-negative bacteria possess an outer membrane, with its characteristic asymmetric bilayer as a hallmark. The outer membrane bilayer consists of an inner monolayer containing phospholipids (primarily phosphatidyl ethanolamine) and an outer monolayer that is mainly composed of a single glycolipid, lipopolysaccharide (LPS). There is an immense diversity of LPS structures in the bacterial kingdom and the LPS structure may be modified in response to prevailing environmental conditions. The stability of the LPS layer and interaction between different LPS molecules is mainly achieved by the electrostatic interaction of divalent ions (Mg²⁺, Ca²⁺) with the anionic components of the LPS molecule (phosphate groups in the lipid A and the inner core and carboxyl groups of KDO). Furthermore, the dense and ordered packing of the hydrophobic moiety of lipid A, favoured by the absence of unsaturated fatty acids, forms a rigid structure with high viscosity. This makes it less permeable for lipophilic molecules and confers additional stability to the outer membrane (OM).

The present invention makes use of endolysins. Endolysins are peptidoglycan hydrolases typically encoded by bacteriophages (or bacterial viruses). They are synthesized during late gene expression in the lytic cycle of phage multiplication and mediate the release of progeny virions from infected cells through degradation of the bacterial peptidoglycan. They are either β(1,4)-glycosyl hydrolases (lysozymes), transglycosylases, amidases or endopeptidases. Antimicrobial application of endolysins was already suggested in 1991 by Gasson (GB2243611). Although the killing capacity of endolysins has been known for a long time, the use of these enzymes as antibacterials was ignored due to the success and dominance of antibiotics. Only after the appearance of multiple antibiotic resistant bacteria this simple concept of combating human pathogens with endolysins received interest. A compelling need to develop totally new classes of antibacterial agents emerged and endolysins used as 'enzybiotics' - a hybrid term of 'enzymes' and 'antibiotics' - perfectly met this need. In 2001, Fischetti and coworkers demonstrated for the first time the therapeutic potential of bacteriophage Cl endolysin towards group A streptococci (Nelson et al., 2001, Proc. Natl. Acad. Sci. U. S. A. 98:4107-4112; herewith incorporated by reference). Since then many publications have established endolysins as an attractive and complementary alternative to control bacterial infections of Gram-positive bacteria. Subsequently different endolysins against other Gram-positive pathogens such as *Streptococcus pneumoniae* (Loeffler et al., 2001, Science 294:2170-2172), *Bacillus anthracis* (Schuch et al., 2002; Nature 418:884-889), *S*. *agalactiae* (Cheng et al., 2005; Antimicrob Agents Chemother. 2005 Jan;49(1):111-117) and *Staphylococcus aureus* (Rashel et al, 2007; J Infect Dis. 2007 Oct 15;196(8):1237-1247) have proven their efficacy as enzybiotics (all references incorporated herewith by reference). Nowadays, the most important challenge of endolysin therapy lies in the insensitivity of Gram-negative bacteria towards the exogenous action of endolysins, since the outer membrane shields the access of endolysins from the peptidoglycan. This currently prevents the expansion of the range of effective endolysins to important Gram-negative pathogens, such as bacteria of the genus *Salmonella.*

In the art combinations of endolysins with further amino acid sequence stretches have been described to create new antimicrobial agents. WO 2015/071436 (herewith incorporated by reference) discloses fusions of peptides with derivatives of endolysin KZ144, which show activity towards *E. coli*, *P. aeruginosa*, and *C. jejuni.* Briers et al. (MBio; 2014;5(4):e01379-14; herewith incorporated by reference) report that fusion proteins of the endolysins OBPgp279 and PVP-SE1gp146 with certain peptides yield antimicrobial agents with moderate activity against *Salmonella* Typhimurium LT 2 bacteria.

Nonetheless, there is still a constant need for new antibacterial agents active against Gram-negative bacteria. In particular, there is a need for antibacterial agents active against bacteria of the Genus *Salmonella.* Preferably, said agents are active against a diverse set of *Salmonella* strains, exhibit an increased activity and/or are sufficiently (heat) stable for technical applications.

This object is solved by the subject matter defined in the claims and set forth below.

The term "polypeptide" as used herein refers in particular to a polymer of amino acids linked by peptide bonds in a specific sequence. The amino acid residues of a polypeptide may be modified by e.g. covalent attachments of various groups such as carbohydrates and phosphate. Other substances may be more loosely associated with the polypeptide, such as heme or lipid, giving rise to conjugated polypeptides which are also comprised by the term "polypeptide" as used herein. The term as used herein is intended to encompass also proteins. Thus, the term "polypeptide" also encompasses for example complexes of two or more amino acid polymer chains. The term "polypeptide" does encompass embodiments of polypeptides which exhibit optionally modifications typically used in the art, e.g. biotinylation, acetylation, pegylation, chemical changes of the amino-, SH- or carboxyl-groups (e.g. protecting groups) etc.. As will become apparent from the description below, the polypeptide according to the present invention may also be a fusion protein, i.e. linkage of at least two amino acid sequences which do not occur in this combination in nature. The term "polypeptide", as used herein, is not limited to a specific length of the amino acid polymer chain, but typically the polypeptide will exhibit a length of more than about 50 amino acids, more than about 100 amino acids or even more than about 150 amino acids. Usually, but not necessarily, a typical polypeptide of the present invention will not exceed about 750 amino acids in length.

The term "endolysin" as used herein refers to a bacteriophage-derived enzyme which is suitable to hydrolyse bacterial cell walls. Endolysins comprise at least one "enzymatically active domain" (EAD) having at least one of the following activities: endopeptidase, chitinase, T4 like muraminidase, lambda like muraminidase, N-acetyl-muramoyl-L-alanine-amidase (amidase), muramoyl-L-alanine-amidase, muramidase , lytic transglycosylase (C), lytic transglycosylase (M), N-acetyl-muramidase (lysozyme), N-acetyl-glucosaminidase or transglycosylases as e.g. KZ144. In addition, the endolysins may contain also regions which are enzymatically inactive, and bind to the cell wall of the host bacteria, the so-called CBDs (cell wall binding domains). The term "endolysin" also encompasses enzymes which comprise modifications and/or alterations vis-a-vis naturally occurring endolysins. Such alterations and/or modifications may comprise mutations such as deletions, insertions and additions, substitutions or combinations thereof and/or chemical changes of the amino acid residues. Particularly preferred chemical changes are biotinylation, acetylation, pegylation, chemical changes of the amino-, SH- or carboxyl- groups. Said endolysins exhibit on a general level the lytic activity of the respective wild-type endolysin. However, said activity can be the same, higher or lower as the activity of the respective wild-type endolysin. Said activity can be for example at least about 50, 60, 70, 80, 90, 100, 110, 120, 130, 140, 150, 160, 170, 180, 190 or at least about 200 % of the activity of the respective wild-type endolysin or even more. The activity can be measured by assays well known in the art by a person skilled in the art as e.g. the plate lysis assay or the liquid lysis assay which are e.g. described in Briers et al. (J. Biochem. Biophys Methods; 2007; 70: 531-533) or Donovan et al. (J. FEMS Microbiol Lett. 2006 Dec;265(1) and similar publications.

The term "fragment" as used herein refers to an amino acid sequence which is N-terminally, C-terminally, and/or on both termini truncated with respect to the respective reference sequence, for example a given SEQ ID NO. Thus, a fragment of an amino acid sequence as used herein is an amino acid sequence which is at least one amino acid shorter than the respective reference sequence. A fragment of an amino acid sequence as used herein is preferably an amino acid sequence which is at most 20, more preferably at most 19, more preferably at most 18, more preferably at most 17, more preferably at most 16, more preferably at most 15, more preferably at most 14, more preferably at most 13, more preferably at most 12, more preferably at most 11, more preferably at most 10, more preferably at most 9, more preferably at most 8, more preferably at most 7, more preferably at most 6, more preferably at most 5, more preferably at most 4, more preferably at most 3, more preferably at most 2, most preferably 1 amino acid residue shorter than the respective reference amino acid sequence. The fragment may for example exhibit vis-a-vis the reference sequence a truncation of 1, 2, 3, 4, 5, 6, 7, 8, 9, 10, 11, 12, 13, 14, 15, 16, 17, 18, 19 or 20 amino acids at the N-terminus, the C-terminus or both. It is understood that a polypeptide comprising a fragment of a given amino acid sequence does not comprise the full length of said reference amino acid sequence.

The term "derivative", as used herein, refers to an amino acid sequence which exhibits, in comparison to the respective reference sequence, one or more additions, deletions, insertions, and/or substitutions and combinations thereof. This includes for example combinations of deletions/insertions, insertions/deletions, deletions/additions, additions/deletions, insertion/ additions, additions/insertions etc. A person skilled in the art will however understand that the presence of an amino acid residue at a certain position of the derivative sequence which is different from the one that is present at the respective same position in the reference sequence is not a combination of, for example, a deletion and a subsequent insertion at the same position but is a substitution as defined herein. Rather, if reference is made herein to combinations of one or more of additions, deletions, insertions, and substitutions, then combination of changes at distinct positions in the sequence are intended, e.g. an addition at the N-terminus and an intrasequential deletion. Such derived sequence will exhibit a certain level of sequence identity with the respective reference sequence, for example a given SEQ ID NO, which is preferably at least 60%, such as at least 75%, at least 80%, at least 85%, at least 90%, at least 95%, at least 96%, at least 97%, at least 98% or at least 99%. Preferred derivatives are fragments of the parent molecule, for example a given SEQ ID NO, retaining the activity of the parent molecule, i.e. exhibiting on a general level same activity as the respective parent molecule. However, said activity can be the same, higher or lower as the respective parent molecule. Also preferred derivatives are those resulting from conservative amino acid substitutions within the parent sequence, for example a given SEQ ID NO, again retaining the activity of the parent molecule on a general level.

As used herein, the term "% sequence identity", has to be understood as follows: Two sequences to be compared are aligned to give a maximum correlation between the sequences. This may include inserting "gaps" in either one or both sequences, to enhance the degree of alignment. A % identity may then be determined over the whole length of each of the sequences being compared (so-called global alignment), that is particularly suitable for sequences of the same or similar length, or over shorter, defined lengths (so-called local alignment), that is more suitable for sequences of unequal length. In the above context, an amino acid sequence having a "sequence identity" of at least, for example, 95% to a query amino acid sequence, is intended to mean that the sequence of the subject amino acid sequence is identical to the query sequence except that the subject amino acid sequence may include up to five amino acid alterations per each 100 amino acids of the query amino acid sequence. In other words, to obtain an amino acid sequence having a sequence of at least 95% identity to a query amino acid sequence, up to 5% (5 of 100) of the amino acid residues in the subject sequence may be inserted or substituted with another amino acid or deleted. Methods for comparing the identity and homology of two or more sequences are well known in the art. The percentage to which two sequences are identical can for example be determined by using a mathematical algorithm. A preferred, but not limiting, example of a mathematical algorithm which can be used is the algorithm of Karlin et al. (1993), PNAS USA, 90:5873-5877. Such an algorithm is integrated in the BLAST family of programs, e.g. BLAST or NBLAST program (see also Altschul et al., 1990, J. Mol. Biol. 215, 403-410 or Altschul et al. (1997), Nucleic Acids Res, 25:3389-3402), accessible through the home page of the NCBI at world wide web site ncbi.nlm.nih.gov) and FASTA (Pearson (1 990), Methods Enzymol. 83, 63-98; Pearson and Lipman (1988), Proc. Natl. Acad. Sci. U. S. A 85, 2444-2448.). Sequences which are identical to other sequences to a certain extent can be identified by these programmes. Furthermore, programs available in the Wisconsin Sequence Analysis Package, version 9.1 (Devereux et al, 1984, Nucleic Acids Res., 387-395), for example the programs BESTFIT and GAP, may be used to determine the % identity between two polypeptide sequences. BESTFIT uses the "local homology" algorithm of (Smith and Waterman (1981), J. Mol. Biol. 147, 195-197.) and finds the best single region of similarity between two sequences. If herein reference is made to an amino acid sequence sharing a particular extent of sequence identity to a reference sequence, then said difference in sequence is preferably due to conservative amino acid substitutions. Preferably, such sequence retains the activity of the reference sequence, e.g. albeit maybe at a slower rate. In addition, if reference is made herein to a sequence sharing "at least" at certain percentage of sequence identity, then 100% sequence identity are preferably not encompassed.

"Conservative amino acid substitutions", as used herein, may occur within a group of amino acids which have sufficiently similar physicochemical properties, so that a substitution between members of the group will preserve the biological activity of the molecule (see e.g. Grantham, R. (1974), Science 185, 862-864). Particularly, conservative amino acid substitutions are preferably substitutions in which the amino acids originate from the same class of amino acids (e.g. basic amino acids, acidic amino acids, polar amino acids, amino acids with aliphatic side chains, amino acids with positively or negatively charged side chains, amino acids with aromatic groups in the side chains, amino acids the side chains of which can enter into hydrogen bridges, e.g. side chains which have a hydroxyl function, etc.). Conservative substitutions are in the present case for example substituting a basic amino acid residue (Lys, Arg, His) for another basic amino acid residue (Lys, Arg, His), substituting an aliphatic amino acid residue (Gly, Ala, Val, Leu, lie) for another aliphatic amino acid residue, substituting an aromatic amino acid residue (Phe, Tyr, Trp) for another aromatic amino acid residue, substituting threonine by serine or leucine by isoleucine. Further conservative amino acid exchanges will be known to the person skilled in the art.

The term "deletion" as used herein refers preferably to the absence of 1, 2, 3, 4, 5 (or even more than 5) continuous amino acid residues in the derivative sequence in comparison to the respective reference sequence, either intrasequentially or at the N- or C-terminus. A derivative of the present invention may exhibit one, two or more of such deletions.

The term "insertion" as used herein refers preferably to the additional intrasequential presence of 1, 2, 3, 4, 5 (or even more than 5) continuous amino acid residues in the derivative sequence in comparison to the respective reference sequence. A derivative of the present invention may exhibit one, two or more of such insertions.

The term "addition" as used herein refers preferably to the additional presence of 1, 2, 3, 4, 5 (or even more than 5) continuous amino acid residues at the N- and/or C-terminus of the derivative sequence in comparison to the respective reference sequence.

The term "substitution" as used herein refers to the presence of an amino acid residue at a certain position of the derivative sequence which is different from the amino acid residue which is present or absent at the corresponding position in the reference sequence. A derivative of the present invention may exhibit 1, 2, 3, 4, 5, 6, 7, 8, 9, 10, 11, 12, 13, 14, 15, 16, 17, 18, 19, 20, or more of such substitutions. As mentioned above, preferably such substitutions are conservative substitutions.

The term "cell wall" as used herein refers to all components that form the outer cell enclosure of Gram-negative bacteria and thus guarantee their integrity. In particular, the term "cell wall" as used herein refers to peptidoglycan, the outer membrane of the Gram-negative bacteria with the lipopolysaccharide, the bacterial cell membrane, but also to additional layers deposited on the peptidoglycan as e.g. capsules, outer protein layers or slimes.

The term "second amino acid sequence", as used herein refers an amino acid subsequence within the amino acid sequence of the polypeptide of the invention. Said sequence may be the sequence of a cationic peptide, a polycationic peptide, an amphipathic peptide, a hydrophobic peptide, a sushi peptide and/or an antimicrobial peptide. The term does not refer to conventional tags like His-tags, such as His5-tags, His6-tags, His7-tags, His8-tags, His9-tags, His10-tags, His11-tags, His12-tags, His16-tags and His20-tags, Strep-tags, Avi-tags, Myc-tags, Gst-tags, JS-tags, cystein-tags, FLAG-tags or other tags known in the art, thioredoxin or maltose binding proteins (MBP). Preferably, the second amino acid sequence has as a length of at least about 6 to at most about 50, preferably at most about 39 amino acid residues.

The terms "first amino acid sequence" and "second amino acid sequence", as used herein, do not imply an inherent order of the sequences within the inventive polypeptide, i.e. the second amino acid sequence may be N-terminal of the first amino acid sequence or C-terminal of the first amino acid sequence.

As used herein, the term "cationic peptide" refers preferably to a peptide having positively charged amino acid residues. Preferably a cationic peptide has a pKa-value of 9.0 or greater. Typically, at least four of the amino acid residues of the cationic peptide can be positively charged, for example, lysine or arginine. "Positively charged" refers to the side chains of the amino acid residues which have a net positive charge at about physiological conditions. The term "cationic peptide" as used herein refers also to polycationic peptides, but also includes cationic peptides which comprise for example less than 20%, preferably less than 10% positively charged amino acid residues.

The term "polycationic peptide" as used herein refers preferably to a peptide composed of mostly positively charged amino acid residues, in particular lysine and/or arginine residues. A peptide is composed of mostly positively charged amino acid residues if at least about 20, 30, 40, 50, 60, 70, 75, 80, 85, 90, 95 or about 100 % of the amino acid residues are positively charged amino acid residues, in particular lysine and/or arginine residues. The amino acid residues being not positively charged amino acid residues can be neutrally charged amino acid residues and/or negatively charged amino acid residues and/or hydrophobic amino acid residues. Preferably the amino acid residues being not positively charged amino acid residues are neutrally charged amino acid residues, in particular serine and/or glycine.

The term, "antimicrobial peptide" (AMP) as used herein refers preferably to any naturally occurring peptide that has microbicidal and/or microbistatic activity on, for example, bacteria, viruses, fungi, yeasts, mycoplasma and protozoa. Thus, the term "antimicrobial peptide" as used herein refers in particular to any peptide having anti-bacterial, anti-fungal, anti-mycotic, anti-parasitic, anti-protozoal, anti-viral, anti-infectious, anti-infective and/or germicidal, algicidal, amoebicidal, microbicidal, bactericidal, fungicidal, parasiticidal, protozoacidal, protozoicidal properties. Preferred are anti-bacterial peptides. The antimicrobial peptide may be a member of the RNase A super family, a defensin, cathelicidin, granulysin, histatin, psoriasin, dermicidine or hepcidin. The antimicrobial peptide may be naturally occurring in insects, fish, plants, arachnids, vertebrates or mammals. Preferably the antimicrobial peptide may be naturally occurring in radish, silk moth, wolf spider, frog, preferably in Xenopus laevis, Rana frogs, more preferably in Rana catesbeiana, toad, preferably Asian toad Bufo bufo gargarizans, fly, preferably in Drosophila, more preferably in Drosophila melanogaster, in Aedes aegypti, in honey bee, bumblebee, preferably in Bombus pascuorum, flesh fly, preferably in Sarcophaga peregrine, scorpion, horseshoe crab, catfish, preferably in Parasilurus asotus, cow, pig, sheep, porcine, bovine, monkey and human. As used herein, an "antimicrobial peptide" (AMP) may in particular be a peptide which is not a cationic peptide, polycationic peptide, amphipathic peptide, sushi peptide, defensins, and hydrophobic peptide, but nevertheless exhibits antimicrobial activity.

The term "sushi peptide" as used herein refers to complement control proteins (CCP) having short consensus repeats. The sushi module of sushi peptides functions as a protein-protein interaction domain in many different proteins. Peptides containing a Sushi domain have been shown to have antimicrobial activities. Preferably, sushi peptides are naturally occurring peptides.

The term "defensin" as used herein refers to a peptide present within animals, preferably mammals, more preferably humans, wherein the defensin plays a role in the innate host defense system as the destruction of foreign substances such as infectious bacteria and/or infectious viruses and/or fungi. A defensin is a non-antibody microbicidal and/or tumoricidal protein, peptide or polypeptide. Examples for "defensins" are "mammalian defensins," alpha-defensins, beta-defensins, indolicidin and magainins. The term "defensins" as used herein refers both to an isolated form from animal cells or to a synthetically produced form, and refers also to variants which substantially retain the cytotoxic activities of their parent proteins, but whose sequences have been altered by insertion or deletion of one or more amino acid residues.

The term "amphipathic peptide" as used herein refers to synthetic peptides having both hydrophilic and hydrophobic functional groups. Preferably, the term "amphipathic peptide" as used herein refers to a peptide having a defined arrangement of hydrophilic and hydrophobic groups e.g. amphipathic peptides may be e.g. alpha helical, having predominantly non polar side chains along one side of the helix and polar residues along the rest of its surface.

The term "hydrophobic group" as used herein refers preferably to chemical groups such as amino acid side chains which are substantially water insoluble, but soluble in an oil phase, with the solubility in the oil phase being higher than that in water or in an aqueous phase. In water, amino acid residues having a hydrophobic side chain interact with one another to generate a non-aqueous environment. Examples of amino acid residues with hydrophobic side chains are valine, isoleucine, leucine, methionine, phenylalanine, tryptophan, cysteine, alanine, tyrosine, and proline residues

The term "hydrophobic peptide" as used herein refers to a hydrophobic peptide, which is preferably composed of mostly amino acid residues with hydrophobic groups. Such peptide is preferably composed of mostly hydrophobic amino acid residues, i.e. at least about 20, 30, 40, 50, 60, 70, 75, 80, 85, 90, 95 or at least about 100 % of the amino acid residues are hydrophobic amino acid residues. The amino acid residues being not hydrophobic are preferably neutral and preferably not hydrophilic.

As used herein, the term "tag" refers to an amino acid sequence, which is typically in the art fused to or included in another amino acid sequence for a) improving expression of the overall amino acid sequence or polypeptide, b) facilitating purification of the overall amino acid sequence or polypeptide, c) facilitating immobilisation of the overall amino acid sequence or polypeptide, and/or d) facilitating detection of the overall amino acid sequence or polypeptide. Examples for tags are His tags, such as His5-tags, His6-tags, His7-tags, His8-tags, His9-tags, His10-tags, His11-tags, His12-tags, His16-tags and His20-tags, Strep-tags, Avi-tags, Myc-tags, GST-tags, JS-tags, cystein-tags, FLAG-tags, HA-tags, thioredoxin or maltose binding proteins (MBP), CAT, GFP, YFP, etc. The person skilled in the art will know a vast number of tags suitable for different technical applications. The tag may for example make such tagged polypeptide suitable for e.g. antibody binding in different ELISA assay formats or other technical applications.

The term "comprising" as used herein shall not be construed as being limited to the meaning "consisting of" (i.e. excluding the presence of additional other matter). Rather, "comprising" implies that optionally additional matter may be present. The term "comprising" encompasses as particularly envisioned embodiments falling within its scope "consisting of' (i.e. excluding the presence of additional other matter) and "comprising but not consisting of' (i.e. requiring the presence of additional other matter), with the former being more preferred.

The inventors of the present invention have surprisingly found that endolysins according to SEQ ID NO:1 and SEQ ID NO:2, and their derivatives, as well as antimicrobial peptides according to SEQ ID NO:3 and SEQ ID NO:4, and their derivatives, are extremely useful components when designing antimicrobial agents against bacteria of the Genus *Salmonella.* Such compounds show increased activity, for example against *Salmonella* Typhimurium bacteria.

Therefore, the present invention relates in a first aspect to a polypeptide comprising a first and a second amino acid sequence, wherein the first amino acid sequence is an endolysin, and wherein the second amino acid sequence is an antimicrobial peptide, amphiphatic peptide, cationic peptide, hydrophobic peptide, sushi peptide or defensin, and wherein the polypeptide comprises at least one sequence selected from the following group of sequences:
i) an amino acid sequence according to SEQ ID NO:1;
ii) a derivative of SEQ ID NO:1 exhibiting at least 80% sequence identity with SEQ ID NO:1, with the proviso that the polypeptide may not comprise the sequence of SEQ ID NO:5 if the polypeptide comprises ii), but none of iii), iv), v) or vi),
iii) an amino acid sequence according to SEQ ID NO:3;
iv) a derivative of SEQ ID NO:3 exhibiting at least 77% sequence identity with SEQ ID NO:3;
v) an amino acid sequence according to SEQ ID NO:4, with the proviso that the polypeptide may in this case not comprise the sequence of SEQ ID NO:6 in parallel; and
vi) a derivative of SEQ ID NO:4 exhibiting at least 80% sequence identity with SEQ ID NO:4.

The amino acid sequence according to SEQ ID NO:1 and derivatives thereof are examples for the first amino acid sequence, i.e. the endolysin, SEQ ID NO:3, SEQ ID NO:4 and respective derivatives are examples for the second amino acid sequence.

In preferred embodiments of the invention the first amino acid sequence is selected from the group consisting of:
i) an amino acid sequence according to SEQ ID NO:1;
ii) a derivative of SEQ ID NO:1 exhibiting at least 80% sequence identity with SEQ ID NO:1,
iii) an amino acid sequence according to SEQ ID NO:2; and
iv) a derivative of SEQ ID NO:2 exhibiting at least 80% sequence identity with SEQ ID NO:2.

Derivatives of SEQ ID NO:1 exhibit preferably at least 85%, at least 87,5%, at least 90%, at least 91%, at least 92%, at least 93%, at least 94%, at least 95%, at least 96%, at least 97%, at least 98%, at least 99%, or more than 99% sequence identity with SEQ ID NO:1. An example for a derivative of SEQ ID NO:1 is the amino acid sequence according to SEQ ID NO:7. In other embodiments, the derivative is not an amino acid sequence according to SEQ ID NO:7. Particularly preferred derivatives exhibit at least 98%, at least 99%, or more than 99% sequence identity with SEQ ID NO:1. As mentioned before, a polypeptide of the present invention comprising a derivative of SEQ ID NO:1 may not comprise the sequence of SEQ ID NO:5, if the polypeptide of the invention does not comprise in parallel an amino acid sequence according to SEQ ID NO:3, a derivative of SEQ ID NO:3 as defined herein, an amino acid sequence according to SEQ ID NO:4, and/or a derivative of SEQ ID NO:4 as defined herein. This proviso applies for the polypeptides of the invention per se, but not necessarily for other aspects of the present invention, e.g. methods and uses employing such polypeptide, in particular when dealing with Salmonella bacteria. However, said proviso may of course also apply to the other aspects, e.g. the methods and uses of such polypeptide.

Derivatives of SEQ ID NO:2 exhibit preferably at least 85%, at least 87,5%, at least 90%, at least 91%, at least 92%, at least 93%, at least 94%, at least 95%, at least 96%, at least 97%, at least 98%, at least 99%, or more than 99% sequence identity with SEQ ID NO:2. Suitable derivatives of SEQ ID NO:2 according to SEQ ID NO:8 have been described extensively in WO 2015/071436 (incorporated herein by reference).

The derivative of SEQ ID NO:2, e.g. based on the consensus sequence according to SEQ ID NO:8, may deviate from SEQ ID NO:2 in particular at those positions in SEQ ID NO:8, which are known to be non-critical for the enzymatic activity, i.e. X1, X14, X23, X50, X82, X122, X149; X160, X167, X179, X180, X186; X206; X212; X224; X230 and/or X232. Such derivative may exhibit at least one (e.g. 1, 2, 3, 4, 5, 6, 7, 8, 9, 10, 11, 12, 13, 14, 15, 16 or even all 17) of the following: X14 is not C; X23 is not C; X50 is not C; X82 is I; X122 is M; X149 is P; X154 is T, X160 is T; X167 is L; X179 is F; X180 is E; X186 is Y; X206 is N or V, X212 is N; X224 is Q; X230 is Y and/or X232 is T. It is understood that the number indicating the position of the respective amino acid residue indicates the relative position in the sequence corresponding to SEQ ID NO:8, and not to the overall amino acid sequence of the polypeptide according to the present invention, which may be longer.

Preferably, any derivative of SEQ ID NO:2 exhibits a glutamic acid residue at the position corresponding to position 114 in SEQ ID NO:2. As shown in the publication Briers et al. (Molecular Microbiology; 2007; 65(5), 1334-1344), the mutation E115A (position 114 in SEQ ID NO:2) led to a loss in activity of about 70% of the enzyme. Thus, while an inventive polypeptide comprising said mutation will thus not be a loss of function polypeptide and may still serve various technical purposes, it is certainly preferred if such mutation is not present in the sequence stretch corresponding to SEQ ID NO:8 within the inventive polypeptide.

In a particular preferred embodiment according to the present invention the derivative of SEQ ID NO:2 comprises the sequence of SEQ ID NO:8.

The authors of WO 2015/071436 have found out that three cysteine residues in the amino acid sequence of SEQ ID NO:2 (KZ144 endolysin sequence) are not essential for the enzymatic activity. Thus, in the sequence corresponding to SEQ ID NO:8 (consensus sequence of selected KZ144 derivatives), in some embodiments X14 is not C, X23 is not C, or X50 is not C. Combinations are possible, e.g. X14 and X23 are not C, X14 and X50 are not C, or X23 and X50 are not C. Likewise, it is also possible that neither X14 nor X23 nor X50 are C. In principle said amino acid residues can be deleted or substituted by any other amino acid. Examples for such other amino acids are S, R and N. Thus, X14 may for example be S, N, or R; more preferably S or R; most preferably R; X23 may for example be S, N, or R, more preferably S; and X50 may for example be S, N, or R, more preferably S or N; most preferably N. X14, X23 and X50 may of course exhibit different amino acid substitutions, for example X14 may be R while X23 and X50 are S; or X14 and X23 are S, while X50 is N; X14 may be R while X23 is S and X50 is N etc.. Any other combination conceivable is also contemplated by the present invention. Conservative amino acid substitutions are preferred. Particularly preferred is a substitute of a serine residue for the cysteine residue. Thus, in particularly preferred examples of the present invention X14 is S, X23 is S or X50 is S. Of course, it is also possible that X14 and X23 are S, or that X14 and X50 are S, or that X23 and X50 are S. X14, X23 and X50 may also all three be S. Absence of one or more or even of all of these cysteine residue has the advantage that the risk of aggregation of the polypeptide according to the present invention, e.g. by undesired disulfide bridge formation, is reduced, and is thus an preferred embodiment of the present invention.

Aside of the dispensability of the above referenced cysteine residues, the authors of WO 2015/071436 have also elucidated that various other residues in the sequence of SEQ ID NO:8 are also not essential and, moreover, may be replaced by other residues, thereby increasing for instance the temperature stability of the inventive polypeptide. Examples for such substitutions are X82I, X122M, X149P; X154T, X160T, X167L, X179F, X180E, X186Y, X206V, X206N, X212N, X230Y and X232T. These substitutions may be present alone or in any combination. A typical combination is the combination of X122M and X160T. Other examples of combinations are, without being limited thereto, X82I, X206V plus X232T; X82I, X122M, X160T, X206V, plus X232T; X82I, X122M, X160T, X206N, plus X232T; X82I, X122M, X206V, plus X232T; X82I, X122M, X149P, X160T, X206V, plus X232T; X82I, X122M, X160T, X180E, X206V, plus X232T; X82I, X122M, X160T, X186Y, X206V, plus X232T; X82I, X122M, X160T, X206V, X230Y, plus X232T; X82I, X122M, X149P, X206V, plus X232T; X82I, X122M, X149P, X160T, X206V, plus X232T; X82I, X122M, X149P, X206V, plus X232T; X82I, X122M, X149P, X167L, X206V, plus X232T; X82I, X122M, X149P, X179F, X206V, plus X232T; X82I, X122M, X149P, X206V, X212N plus X232T; X82I, X122M, X149P, X206V, X224Q plus X232T; X82I, X122M, X149P, X154T, X206V, plus X232T etc.. Of course, this second type of amino acid modifications may be combined with the above mentioned cysteine replacements in any type of combination conceivable. Examples of such combinations are, without being limited thereto, X14S, X50S, X122M and X160T; X14S, X50S, X82I, X122M, X160T, X206V, and X232T; X14S, X50S, X82I, X122M, X160T, X206N, and X232T; X14S, X50S, X82I, X122M, X206V, and X232T; X14S, X50S, X82I, X122M, X149P, X160T, X206V, and X232T; X14S, X50S, X82I, X122M, X160T, X180E, X206V, and X232T; X14S, X50S, X82I, X122M, X160T, X186Y, X206V, and X232T; X14S, X50S, X82I, X122M, X160T, X206V, X230Y, and X232T; X14R, X50S, X82I, X122M, X160T, X206V, and X232T; X14S, X50N, X82I, X122M, X160T, X206V, and X232T; X14R, X50S, X82I, X122M, X149P, X206V, and X232T; X14R, X50S, X82I, X122M, X149P, X160T, X206V, and X232T; X14R, X50N, X82I, X122M, X149P, X206V, and X232T; X14R, X50N, X82I, X122M, X149P, X167L, X206V, and X232T; X14R, X50N, X82I, X122M, X149P, X179F, X206V, and X232T; X14R, X50N, X82I, X122M, X149P, X206V, X212N, and X232T; X14R, X50N, X82I, X122M, X149P, X206V, X224Q and X232T; X14R, X50N, X82I, X122M, X149P, X154T, X206V, and X232T; etc..

Examples of particularly preferred derivatives of SEQ ID NO:2 are for instance SEQ ID NO:9; SEQ ID NO:10; SEQ ID NO:11; SEQ ID NO:12; SEQ ID NO:13; SEQ ID NO:14; SEQ ID NO:15; SEQ ID NO:16; SEQ ID NO:17; SEQ ID NO:18; SEQ ID NO:19; SEQ ID NO:20; SEQ ID NO:21; SEQ ID NO:22; SEQ ID NO:23; SEQ ID NO:24; SEQ ID NO:25; SEQ ID NO:26; SEQ ID NO:27; SEQ ID NO:28; SEQ ID NO:29; and SEQ ID NO:30 (and corresponding sequences with N-terminal methionine). A particularly preferred derivative of SEQ ID NO:2 is SEQ ID NO:13.

Suitable sequences for the first amino acid sequence of the polypeptide according to the present invention are for example SEQ ID NO:31, SEQ ID NO:32, and SEQ ID NO:33. Other examples for the first amino acid sequence of the polypeptide according to the present invention include SEQ ID NO:34 or SEQ ID NO:35. A further example is the amino acid sequence of SEQ ID NO:7. In certain embodiments of the invention, in particular where the polypeptide comprises an amino acid sequence according to SEQ ID NO:3, a derivative of SEQ ID NO:3 as defined herein, an amino acid sequence according to SEQ ID NO:4, and/or a derivative of SEQ ID NO:4 as defined herein, the amino acid sequence according to SEQ ID NO:5 is also a suitable sequence for the first amino acid sequence of the polypeptide according to the present invention.

In preferred embodiments of the invention the the second amino acid sequence is selected from the group consisting of:
i) an amino acid sequence according to SEQ ID NO:3;
ii) a derivative of SEQ ID NO:3 exhibiting at least 77% sequence identity with SEQ ID NO:3;
iii) an amino acid sequence according to SEQ ID NO:4; and
iv) a derivative of SEQ ID NO:4 exhibiting at least 80% sequence identity with SEQ ID NO:4.

As mentioned previously, a polypeptide of the present invention comprising the amino acid sequence according to SEQ ID NO:4 may not comprise in parallel the sequence of SEQ ID NO:6. Preferably, such polypeptide according to the present invention does not comprise the sequence of SEQ ID NO:2. In particular, a polypeptide according to the present invention does not comprise the sequence of SEQ ID NO:36. This proviso applies for the polypeptides of the invention per se, but not necessarily for other aspects of the present invention, e.g. methods and uses employing such polypeptide, in particular when dealing with Salmonella bacteria. However, said proviso may of course also apply to the other aspects, e.g. the methods and uses of such polypeptide.

Derivatives of SEQ ID NO:3 exhibit preferably at least 81%, at least 86%, at least 90%, or at least 95% sequence identity with SEQ ID NO:3. Derivatives of SEQ ID NO:3 are preferably fragments of SEQ ID NO:3 and/or exhibit conservative amino acid substitutions vis-a-vis SEQ ID NO:3. Particularly preferred derivatives of SEQ ID NO:3 are fragments of SEQ ID NO:3. For example, a 17-mer fragment (SEQ ID NO:37) of SEQ ID NO:3 has been described in the art (Monteiro et al, Mol Pharm., 2015; 12(8):2904-11) which exhibits improved selectivity.

Derivatives of SEQ ID NO:4 exhibit preferably at least 83%, at least 86%, at least 90%, at least 93%, or at least 96% sequence identity with SEQ ID NO:4. Derivatives of SEQ ID NO:4 are preferably fragments of SEQ ID NO:4 and/or exhibit conservative amino acid substitutions vis-a-vis SEQ ID NO:4.

Preferred combinations of first and second amino acid sequence in the polypeptide according to the present invention are polypeptides, wherein the first amino acid sequence is SEQ ID NO:1 or said derivative thereof, and wherein the second amino acid sequence is SEQ ID NO:3 or said derivative thereof. A preferred combination is also wherein the first amino acid sequence is SEQ ID NO:1 or said derivative thereof, and wherein the second amino acid sequence is SEQ ID NO:4 or said derivative thereof. A further preferred combination is where the first amino acid sequence is SEQ ID NO:2 or said derivative thereof, and wherein the second amino acid sequence is SEQ ID NO:3 or said derivative thereof. Preferred are also combinations, wherein the first amino acid sequence is SEQ ID NO:2 and wherein the second amino acid sequence is said derivative of SEQ ID NO:4,or wherein the first amino acid sequence is said derivative of SEQ ID NO:2, and the second amino acid sequence is SEQ ID NO:4 or said derivative thereof. Similarly preferred combinations are those, wherein the first amino acid sequence is SEQ ID NO:2 or said derivative thereof, and wherein the second amino acid sequence is SEQ ID NO:4 or said derivative thereof, in particular a derivative according to SEQ ID NO:13.

Particularly preferred combinations are wherein
i) the first amino acid sequence is SEQ ID NO:1 and the second amino acid sequence is SEQ ID NO:3,
ii) the first amino acid sequence is SEQ ID NO:1 and the second amino acid sequence is SEQ ID NO:4, and
iii) wherein the first amino acid sequence is SEQ ID NO:13 and the second amino acid sequence is SEQ ID NO:4.

In other embodiments of the invention, where the second amino acid sequence of the inventive polypeptide is neither SEQ ID NO:3 nor SEQ ID NO:4, the second amino acid sequence is selected from the group consisting of an antimicrobial peptide, amphiphatic peptide, cationic peptide, polycationic peptide, hydrophobic peptide, sushi peptide or defensin.

Examples for cationic/ polycationic amino acid sequences are listed in the following table.

**Table 1:**

| **Amino acid sequence** | **Length** | **SEQ ID NO:** |
|---|---|---|
| KRKKRK | 6 | 38 |
| KRXKR | 5 | 39 |
| KRSKR | 5 | 40 |
| KRGSG | 5 | 41 |
| KRKKRKKRK | 9 | 42 |
| RRRRRRRRR | 9 | 43 |
| KKKKKKKK | 8 | 44 |
| KRKKRKKRKK | 10 | 45 |
| KRKKRKKRKKRK | 12 | 46 |
| KRKKRKKRKKRKKR | 14 | 47 |
| KKKKKKKKKKKKKKKK | 16 | 48 |
| KRKKRKKRKKRKKRKKRK | 18 | 49 |
| KRKKRKKRKKRKKRKKRKK | 19 | 50 |
| RRRRRRRRRRRRRRRRRRR | 19 | 51 |
| KKKKKKKKKKKKKKKKKKK | 19 | 52 |
| KRKKRKKRKRSKRKKRKKRK | 20 | 53 |
| KRKKRKKRKRSKRKKRKKRKK | 21 | 54 |
| KRKKRKKRKKRKKRKKRKKRK | 21 | 55 |
| KRKKRKKRKRGSGKRKKRKKRK | 22 | 56 |
| KRKKRKKRKRGSGSGKRKKRKKRK | 24 | 57 |
| KRKKRKKRKKRKKRKKRKKRKKRKK | 25 | 58 |
| KRKKRKKRKRSKRKKRKKRKRSKRKKRKKRK | 31 | 59 |
| KRKKRKKRKRGSGSGKRKKRKKRKGSGSGKRKKRKKRK | 38 | 60 |
| KRKKRKKRKKRKKRKKRKKRKKRKKRKKRKKRKKRKKRK | 39 | 61 |
| KRKKRKKRKRSKRKKRKKRKRSKRKKRKKRKRSKRKKRKKRK | 42 | 62 |

Examples for antimicrobial amino acid sequences which may be used in carrying out the present invention are listed in the following table.

**Table 2:**

| **Peptide** | **Sequence** | **SEQ ID NO** |
|---|---|---|
| LL-37 | LLGDFFRKSKEKIGKEFKRIVQRIKDFLRNLVPR TES | 63 |
| SMAP-29 | RGLRRLGRKIAHGVKKYGPTVLRIIRIAG | 64 |
| Indolicidin | ILPWKWPWWPWRR | 65 |
| Protegrin | RGGRLCYCRRRFCVCVGR | 66 |
| Cecropin P1 | SWLSKTAKKLENSAKKRISEGIAIAIQGGPR | 67 |
| Magainin | GIGKFLHSAKKFGKAFVGEIMNS | 68 |
| Pleurocidin | GWGSFFKKAAHVGKHVGKAALTHYL | 69 |
| Cecropin A (A.aegypti) | GGLKKLGKKLEGAGKRVFNAAEKALPVVAGAKAL RK | 70 |
| Cecropin A (D. melanogaster) | GWLKKIGKKIERVGQHTRDATIQGLGIPQQAANV AATARG | 71 |
| Buforin II | TRSSRAGLQFPVGRVHRLLRK | 72 |
| Sarcotoxin IA | GWLKKIGKKIERVGQHTRDATIQGLGIAQQAANV AATAR | 73 |
| Apidaecin | ANRPVYIPPPRPPHPRL | 74 |
| Ascaphine 5 | GIKDWIKGAAKKLIKTVASHIANQ | 75 |
| Nigrocine 2 | GLLSKVLGVGKKVLCGVSGLVC | 76 |
| Pseudin 1 | GLNTLKKVFQGLHEAIKLINNHVQ | 77 |
| Ranalexin | FLGGLIVPAMICAVTKKC | 78 |
| Melittin | GIGAVLKVLTTGLPALISWIKRKRQQ | 79 |
| Lycotoxin 1 | IWLTALKFLGKHAAKKLAKQQLSKL | 80 |
| Parasin 1 | KGRGKQGGKVRAKAKTRSS | 81 |
| Buforin I | AGRGKQGGKVRAKAKTRSSRAGLQFPVGRVHRLL RKGNY | 82 |
| Dermaseptin 1 | ALWKTMLKKLGTMALHAGKAALGAAADTISQGTQ | 83 |
| Bactenecin 1 | RLCRIVVIRVCR | 84 |
| Thanatin | GSKKPVPIIYCNRRTGKCQRM | 85 |
| Brevinin 1T | VNPIILGVLPKVCLITKKC | 86 |
| Ranateurin 1 | SMLSVLKNLGKVGLGFVACKINIKQC | 87 |
| Esculentin 1 | GIFSKLGRKKIKNLLISGLKNVGKEVGMDVVRTG IKIAGCKIKGEC | 88 |
| Tachyplesin | RWCFRVCYRGICYRKCR | 89 |
| Androctonin | RSVCRQIKICRRRGGCYYKCTNRPY | 90 |
| alpha-defensin | DCYCRIPACIAGERRYGTCIYQGRLWAFCC | 91 |
| beta-defensin | NPVSCVRNKGICVPIRCPGSMKQIGTCVGRAVKC CRKK | 92 |
| theta-defensin | GFCRCLCRRGVCRCICTR | 93 |
| defensin (sapecin A) | ATCDLLSGTGINHSACAAHCLLRGNRGGYCNGKA VCVCRN | 94 |
| Thionin (crambin) | TTCCPSIVARSNFNVCRIPGTPEAICATYTGCII IPGATCPGDYAN | 95 |
| defensin from radish | QKLCQRPSGTWSGVCGNNNACKNQCIRLEKARHG SCNYVFPAHCICYFPC | 96 |
| Drosomycin | DCLSGRYKGPCAVWDNETCRRVCKEEGRSSGHCS PSLKCWCEGC | 97 |
| Hepcidin | DTHFPICIFCCGCCHRSKCGMCCKT | 98 |
| Bac 5 | RFRPPIRRPPIRPPFYPPFRPPIRPPIFPPIRPP FRPPLGRPFP | 99 |
| PR-39 | RRRPRPPYLPRPRPPPFFPPRLPPRIPPGFPPRF PPRFP | 100 |
| Pyrrhocoricin | VDKGSYLPRPTPPRPIYNRN | 101 |
| Histatin 5 | DSHAKRHHGYKRKFHEKHHSHRGY | 102 |
| ECP19 | RPPQFTRAQWFAIQHISLN | 103 |
| MSI-594 | GIGKFLKKAKKGIGAVLKVLTTG | 104 |
| TL-ColM | METLTVHAPSPSTNLPSYGNGAFSLSAPHVPGAG P | 105 |
| SBO | KLKKIAQKIKNFFAKLVA | 106 |
| Macedocin | GKNGVFKTISHECHLNTWAFLATCCS | 107 |
| Macedocin (Trunc) | GKNGVFKTISHECHLNTWAFLA | 108 |
| D16 | ACKLKSLLKTLSKAKKKKLKTLLKALSK | 109 |
| CPF-C1 | GFGSLLGKALRLGANVL | 110 |
| TL-ColM(-Met) | ETLTVHAPSPSTNLPSYGNGAFSLSAPHVPGAGP | 111 |
| TM-174E | LISKGWPYLLVVVLGATIYFWGNSNG | 112 |
| ECP45 | RPPQFTRAQWFAIQHISLNPPRCTIAMRAINNYR WRCKNQNTFLR | 113 |
| ColicinE3_1-51 (S37F) | SGGDGRGHNTGAHSTSGNINGGPTGLGVGGGASD GFGWSSENNPWGGGSG | 114 |
| ColicinE3_1-69 (S37F) | SGGDGRGHNTGAHSTSGNINGGPTGLGVGGGASD GFGWSSENNPWGGGSGSGIHWGGGSGHGNGGGNG | 115 |
| ColicinD_1-53 | SDYEGSGPTEGIDYGHSMVVWPSTGLISGGDVKP GGSSGIAPSMPPGWGDYS | 116 |
| MSI-78 | GIGKFLKKAKKFGKAFVKILKK | 3 |
| Cathelicidin-BF | KFFRKLKKSVKKRAKEFFKKPRVIGVSIPF | 4 |

The second amino acid sequence stretch may be a sushi peptide which is described by Ding JL, Li P, Ho B Cell Mol Life Sci. 2008 Apr;65(7-8):1202-19. The Sushi peptides: structural characterization and mode of action against Gram-negative bacteria. Especially preferred is the sushi 1 peptide according to SEQ ID NO: 117. Other preferred sushi peptides are sushi peptides S1 and S3 and multiples thereof (Tan et al, FASEB J. 2000 Sep;14(12):1801-13).

Preferred hydrophobic peptides are Walmagh1 having the amino acid sequence according to SEQ ID NO: 118 and the hydrophobic peptide having the amino acid sequence Phe-Phe-Val-Ala-Pro (SEQ ID NO: 119).

Preferred amphiphatic peptides are α4-helix of T4 lysozyme according to SEQ ID NO: 120 and WLBU2-Variant having the amino acid sequence according to SEQ ID NO: 121 and Walmagh 2 according to SEQ ID NO: 122.

With respect to the arrangement of first and second amino acid sequence within the inventive polypeptide it is preferred if the second amino acid sequence is situated N-terminal of the first amino acid sequence. Preferably, the first and second amino acid sequence are linked to each other directly or via a short linker of 1 to 10 amino acid residues, preferably 1 to 5 amino acid residues, even more preferably 1 to 2 amino acids. Linker sequences are preferably flexible sequences, comprising one or more glycine residues. An example for such linker is the sequence GGGGS (SEQ ID NO: 123).

In particular in cases where the inventive polypeptide is to be recombinantly expressed by a host cell, it is preferred if the inventive polypeptide comprises a methionine residue at the N-terminus.

The inventive polypeptide may comprise additionally one or more tag sequences. Such tag sequence may for example be located at the N- or C-terminus of the inventive polypeptide. In a preferred embodiment, the one or more tag sequence is located on the C-terminal side of the polypeptide, e.g. directly at the C-terminus. The one or more tag sequences may be linked for example directly or via a short linker to the rest of the inventive polypeptide (see above). Numerous examples for tags are known in the art, some of which have already been mentioned above. In the context of the present invention a particularly preferred tag sequence is a His-tag, preferably a His tag according to SEQ ID NO: 124.

The length of the polypeptide according to present invention is in principle not limited, but preferably the length will not be excessively large. Preferably, a polypeptide according to the present invention has an overall length not exceeding about 320 amino acids, preferably not exceeding about 310 amino acids.

A preferred polypeptide according to the invention comprises SEQ ID NO: 125, such as a polypeptide comprising SEQ ID NO: 126 or comprising SEQ ID NO: 127. Also contemplated are derivatives of SEQ ID NO: 125, SEQ ID NO: 126 and SEQ ID NO: 127, e.g. each exhibiting at least 80% sequence identity to the respective reference sequence.

A further preferred polypeptide according to the invention comprises SEQ ID NO: 128, such as a polypeptide comprising SEQ ID NO: 129 or comprising SEQ ID NO: 130. Also contemplated are derivatives of SEQ ID NO: 128, SEQ ID NO: 129 and SEQ ID NO: 130, e.g. each exhibiting at least 80% sequence identity to the respective reference sequence.

A further preferred polypeptide according to the invention comprises SEQ ID NO: 131, such as a polypeptide comprising SEQ ID NO: 132 or comprising SEQ ID NO: 133. Also contemplated are derivatives of SEQ ID NO: 131, SEQ ID NO: 132 and SEQ ID NO: 133, e.g. each exhibiting at least 80% sequence identity to the respective reference sequence.

A further polypeptide according to the invention comprises SEQ ID NO: 134, such as a polypeptide comprising SEQ ID NO: 135 or comprising SEQ ID NO: 136. Also contemplated are derivatives of SEQ ID NO: 134, SEQ ID NO: 135 and SEQ ID NO: 136, e.g. each exhibiting at least 80% sequence identity to the respective reference sequence.

Further examples of inventive polypeptides are polypeptides comprising any of the sequences selected from the group consisting of SEQ ID NO: 137, SEQ ID NO: 138, SEQ ID NO: 139, SEQ ID NO: 140, SEQ ID NO: 141, SEQ ID NO: 142, SEQ ID NO: 143, SEQ ID NO: 144, SEQ ID NO: 145, SEQ ID NO: 146, SEQ ID NO: 147, SEQ ID NO: 148 and derivatives of these sequences, e.g. each exhibiting at least 80% sequence identity to the respective reference sequence

A polypeptide according to the present invention is preferably characterized by the ability to degrade the peptidoglycan of *Salmonella* bacteria. The peptidoglycan degrading activity can be measured by assays well known in the art, e.g. by muralytic assays in which the outer membrane of gram negative bacteria such as *Salmonella* bacteria is permeabilized or removed (e.g. with chloroform) to allow the putative enzyme access to the peptidoglycan layer. If the enzyme is active, degradation of the peptidoglycan layer will lead to a drop of turbidity, which can be measured photometrically (see for example Briers et al., J. Biochem. Biophys Methods 70: 531-533, (2007).

The present invention does also relate to nucleic acids encoding one or more inventive polypeptides of the present invention. The inventive nucleic acid may take all forms conceivable for a nucleic acid. In particular the nucleic acids according to the present invention may be RNA, DNA or hybrids thereof. They may be single-stranded or doublestranded. The may have the size of small transcripts or of entire genomes, such as a bacteriophage genome. As used herein, a nucleic acid encoding one or more inventive polypeptides of the present invention may be a nucleic acid reflecting the sense strand. Likewise, the antisense strand is also encompassed. The nucleic acid may encompass a heterologous promotor for expression of the inventive polypeptide.

A nucleic acid according to the present invention comprises a first nucleic acid sequence encoding an endolysin, and a second nucleic acid sequence, wherein the second nucleic acid sequence encodes an antimicrobial peptide, cationic peptide, hydrophobic peptide, amphiphatic peptide or sushi peptide, and wherein the nucleic acid according to the present invention comprises at least one sequence selected from the following group of sequences:
i) a nucleic acid sequence according to SEQ ID NO:149;
ii) a derivative of SEQ ID NO:149 encoding the same polypeptide as SEQ ID NO:149,
iii) a nucleic acid sequence according to SEQ ID NO:150;
iv) a derivative of SEQ ID NO:150 encoding the same polypeptide as SEQ ID NO:150;
v) a nucleic acid sequence according to SEQ ID NO:151; and vi) a derivative of SEQ ID NO:151 encoding the same polypeptide as SEQ ID NO:151.

Preferred nucleic acids according to the present invention comprise the nucleic acid sequence according to SEQ ID NO:149 as first nucleic acid sequence and SEQ ID NO:150 as second nucleic acid sequence, or comprise SEQ ID NO:152 as first nucleic acid sequence and SEQ ID NO:151 as second nucleic acid sequence. Particularly preferred nucleic acid sequences comprise SEQ ID NO:153 or SEQ ID NO:154.

In a further aspect, the present invention relates to a vector, which comprises a nucleic acid according to the present invention. Such vector may for example be an expression vector allowing for expression of an inventive polypeptide. Said expression may be constitutive or inducible. The vector may also be a cloning vector comprising the nucleic acid sequence of an inventive polypeptide for cloning purposes.

In a further aspect, the present invention relates to a host cell comprising a polypeptide according to the present invention, a nucleic acid according to the present invention, and/or a vector according to the present invention. The host cells may be selected in particular from the group consisting of bacterial cells and yeast cells. Particularly preferred host cells are *E. coli* cells.

In a further aspect, the present invention relates to composition comprising a polypeptide according to the present invention, a nucleic acid according to the present invention, a vector according to the present invention, and/or a host cell according to the present invention. Preferred compositions comprise the polypeptide according to the present invention. Preferably, a composition according to the present invention comprises a pharmaceutical acceptable diluent, excipient or carrier. Such composition may be a pharmaceutical composition. A composition according to the present invention may also be a feed additive.

In a further aspect the present invention relates to a polypeptide according to the present invention, a nucleic acid according to the present invention, a vector according to the present invention, a host cell according to the present invention, and/or a composition according the present invention for use in a method for treatment of the human or animal body by surgery or therapy or in diagnostic methods practiced on the human or animal body.

The present invention also relates to a polypeptide (and likewise a nucleic acid encoding such polypeptide, a vector comprising such nucleic acid, a host cell comprising such nucleic acid or vector, and/or a composition comprising such polypeptide, nucleic acid, vector, and/or host cell) for use in a method of treatment or prevention of infections caused by bacteria of the genus *Salmonella,* wherein the polypeptide comprises a first and a second amino acid sequence, wherein the first amino acid sequence is an endolysin, and wherein the second amino acid sequence is an antimicrobial peptide, amphiphatic peptide, cationic peptide, hydrophobic peptide, sushi peptide or defensin, and wherein the polypeptide comprises at least one sequence selected from the following group of sequences:
i) an amino acid sequence according to SEQ ID NO:1;
ii) a derivative of SEQ ID NO:1 exhibiting at least 80% sequence identity with SEQ ID NO:1,
iii) an amino acid sequence according to SEQ ID NO:2;
iv) a derivative of SEQ ID NO:2 exhibiting at least 80% sequence identity with SEQ ID NO:2,
v) an amino acid sequence according to SEQ ID NO:3;
vi) a derivative of SEQ ID NO:3 exhibiting at least 77% sequence identity with SEQ ID NO:3;
vii) an amino acid sequence according to SEQ ID NO:4; and
viii) a derivative of SEQ ID NO:4 exhibiting at least 80% sequence identity with SEQ ID NO:4.

Disclosure set out above for the inventive polypeptide is contemplated for the polypeptide (and the nucleic acid encoding such polypeptide, the vector comprising such nucleic acid, the host cell comprising such nucleic acid or vector, and/or the composition comprising such polypeptide, nucleic acid, vector, and/or host cell) for use in a method of treatment or prevention of infections caused by bacteria of the genus *Salmonella* as well. In particular, disclosure detailing SEQ ID NO:2 and its derivatives is contemplated as embodiments for the polypeptide for use in a method of treatment or prevention of infections caused by bacteria of the genus *Salmonella* (see for instance examples of particularly preferred derivatives of SEQ ID NO:2 above, such as SEQ ID NO:13). As mentioned previously, the provisos regarding the polypeptide of the invention (polypeptide may not comprise the sequence of SEQ ID NO:5, if the polypeptide of the invention does not comprise in parallel an amino acid sequence according to SEQ ID NO:3, a derivative of SEQ ID NO:3 as defined herein, an amino acid sequence according to SEQ ID NO:4, and/or a derivative of SEQ ID NO:4 as defined herein; polypeptide may not comprise in parallel the sequence of SEQ ID NO:6, if the polypeptide comprises SEQ ID NO:4) do not necessarily but preferably apply for this aspect of the present invention. Thus, in some embodiments, the polypeptide for use is an inventive polypeptide as set forth above, i.e. the present invention relates to a polypeptide according to the present invention, a nucleic acid according to the present invention, a vector according to the present invention, a host cell according to the present invention, and/or a composition according the present invention for use in a method of treatment or prevention of infections caused by bacteria of the genus *Salmonella.*

The present invention also relates to a method of treatment or prevention of infections caused bacteria of the genus *Salmonella* in a subject, the method comprising contacting said subject with a polypeptide (or likewise a nucleic acid encoding such polypeptide, a vector comprising such nucleic acid, a host cell comprising such nucleic acid or vector, and/or a composition comprising such polypeptide, nucleic acid, vector, and/or host cell), wherein the polypeptide comprises a first and a second amino acid sequence, wherein the first amino acid sequence is an endolysin, and wherein the second amino acid sequence is an antimicrobial peptide, amphiphatic peptide, cationic peptide, hydrophobic peptide, sushi peptide or defensin, and wherein the polypeptide comprises at least one sequence selected from the following group of sequences:
i) an amino acid sequence according to SEQ ID NO:1;
ii) a derivative of SEQ ID NO:1 exhibiting at least 80% sequence identity with SEQ ID NO:1,
iii) an amino acid sequence according to SEQ ID NO:2;
iv) a derivative of SEQ ID NO:2 exhibiting at least 80% sequence identity with SEQ ID NO:2,
v) an amino acid sequence according to SEQ ID NO:3;
vi) a derivative of SEQ ID NO:3 exhibiting at least 77% sequence identity with SEQ ID NO:3;
vii) an amino acid sequence according to SEQ ID NO:4; and
viii) a derivative of SEQ ID NO:4 exhibiting at least 80% sequence identity with SEQ ID NO:4.

Again, disclosure set out above for the inventive polypeptide is contemplated for the polypeptide (and the nucleic acid encoding such polypeptide, the vector comprising such nucleic acid, the host cell comprising such nucleic acid or vector, and/or the composition comprising such polypeptide, nucleic acid, vector, and/or host cell) to be used in said method of treatment as well. In particular, disclosure detailing SEQ ID NO:2 and its derivatives is contemplated as embodiments for the polypeptide to be used in the method of treatment or prevention of infections caused by bacteria of the genus *Salmonella* (see for instance examples of particularly preferred derivatives of SEQ ID NO:2 above, such as SEQ ID NO:13). As mentioned previously, the provisos regarding the polypeptide of the invention (polypeptide may not comprise the sequence of SEQ ID NO:5, if the polypeptide of the invention does not comprise in parallel an amino acid sequence according to SEQ ID NO:3, a derivative of SEQ ID NO:3 as defined herein, an amino acid sequence according to SEQ ID NO:4, and/or a derivative of SEQ ID NO:4 as defined herein; polypeptide may not comprise in parallel the sequence of SEQ ID NO:6, if the polypeptide comprises SEQ ID NO:4) do not necessarily but preferably apply for this aspect of the present invention. Thus, in some embodiments, the polypeptide to be used in the inventive method of treatment is an inventive polypeptide as set forth above, i.e. the present invention relates to a method of treatment or prevention of infections caused bacteria of the genus *Salmonella* in a subject, the method comprising contacting said subject with a polypeptide according to the present invention, a nucleic acid according to the present invention, a vector according to the present invention, a host cell according to the present invention, and/or a composition according the present invention.

In a further aspect the present invention relates to the use of a polypeptide (or likewise a nucleic acid encoding such polypeptide, a vector comprising such nucleic acid, a host cell comprising such nucleic acid or vector, and/or a composition comprising such polypeptide, nucleic acid, vector, and/or host cell) as an antimicrobial in food, as an antimicrobial in feed, as an antimicrobial in cosmetics, or as disinfecting agent, wherein the polypeptide comprises a first and a second amino acid sequence, wherein the first amino acid sequence is an endolysin, and wherein the second amino acid sequence is an antimicrobial peptide, amphiphatic peptide, cationic peptide, hydrophobic peptide, sushi peptide or defensin, and wherein the polypeptide comprises at least one sequence selected from the following group of sequences:
i) an amino acid sequence according to SEQ ID NO:1;
ii) a derivative of SEQ ID NO:1 exhibiting at least 80% sequence identity with SEQ ID NO:1,
iii) an amino acid sequence according to SEQ ID NO:2;
iv) a derivative of SEQ ID NO:2 exhibiting at least 80% sequence identity with SEQ ID NO:2,
v) an amino acid sequence according to SEQ ID NO:3;
vi) a derivative of SEQ ID NO:3 exhibiting at least 77% sequence identity with SEQ ID NO:3;
vii) an amino acid sequence according to SEQ ID NO:4; and
viii) a derivative of SEQ ID NO:4 exhibiting at least 80% sequence identity with SEQ ID NO:4.

Again, the provisos regarding the polypeptide of the invention (see above) do not necessarily but preferably apply for this aspect of the present invention. Thus, the present invention does also relate to a polypeptide according to the present invention, a nucleic acid according the present invention, a vector according to the present invention, a host cell according to the present invention, and/or a composition according to the present invention as an antimicrobial in food, as an antimicrobial in feed, as an antimicrobial in cosmetics, or as disinfecting agent.

In a further aspect the present invention relates to the use of a polypeptide (and likewise a nucleic acid encoding such polypeptide, a vector comprising such nucleic acid, a host cell comprising such nucleic acid or vector, and/or a composition comprising such polypeptide, nucleic acid, vector, and/or host cell) for controlling (e.g. non-therapeutically) the growth of bacteria of the genus *Salmonella* in animals, in particular in livestock, companion animal and/or aquaculture, wherein the polypeptide comprises a first and a second amino acid sequence, wherein the first amino acid sequence is an endolysin, and wherein the second amino acid sequence is an antimicrobial peptide, amphiphatic peptide, cationic peptide, hydrophobic peptide, sushi peptide or defensin, and wherein the polypeptide comprises at least one sequence selected from the following group of sequences:
i) an amino acid sequence according to SEQ ID NO:1;
ii) a derivative of SEQ ID NO:1 exhibiting at least 80% sequence identity with SEQ ID NO:1,
iii) an amino acid sequence according to SEQ ID NO:2;
iv) a derivative of SEQ ID NO:2 exhibiting at least 80% sequence identity with SEQ ID NO:2,
v) an amino acid sequence according to SEQ ID NO:3;
vi) a derivative of SEQ ID NO:3 exhibiting at least 77% sequence identity with SEQ ID NO:3;
vii) an amino acid sequence according to SEQ ID NO:4; and
viii) a derivative of SEQ ID NO:4 exhibiting at least 80% sequence identity with SEQ ID NO:4.

The provisos regarding the polypeptide of the invention (see above) do not necessarily but preferably apply for this aspect of the present invention as well. Disclosure set out above for the inventive polypeptide is contemplated for the polypeptide (and the nucleic acid encoding such polypeptide, the vector comprising such nucleic acid, the host cell comprising such nucleic acid or vector, and/or the composition comprising such polypeptide, nucleic acid, vector, and/or host cell) to be used for controlling (e.g. non-therapeutically) the growth of bacteria of the genus *Salmonella* in animals (in particular in livestock, companion animal and/or aquaculture) as well. In particular, disclosure detailing SEQ ID NO:2 and its derivatives is contemplated as embodiments for the polypeptide to be used for controlling (non-therapeutically) the growth of bacteria of the genus *Salmonella* in animals such as livestock, companion animal and/or aquaculture (see for instance examples of particularly preferred derivatives of SEQ ID NO:2 above, such as SEQ ID NO:13). In some embodiments, the polypeptide to be used for controlling (e.g. non-therapeutically) the growth of bacteria of the genus *Salmonella* in animals is an inventive polypeptide as set forth above, i.e. the present invention relates to the use of a polypeptide according to the present invention, a nucleic acid according to the present invention, a vector according to the present invention, a host cell according to the present invention, and/or a composition according the present invention for controlling (e.g. non-therapeutically) the growth of bacteria of the genus *Salmonella* in animals, in particular in livestock, companion animal and/or aquaculture.

In a further aspect the present invention relates to a method of controlling the growth of bacteria of the genus *Salmonella* in animals, in particular in livestock, companion animal and/or aquaculture, the method comprising contacting livestock, companion animal and/or aquaculture with a polypeptide (or a nucleic acid encoding such polypeptide, a vector comprising such nucleic acid, a host cell comprising such nucleic acid or vector, and/or a composition comprising such polypeptide, nucleic acid, vector, and/or host cell), wherein the polypeptide comprises a first and a second amino acid sequence, wherein the first amino acid sequence is an endolysin, and wherein the second amino acid sequence is an antimicrobial peptide, amphiphatic peptide, cationic peptide, hydrophobic peptide, sushi peptide or defensin, and wherein the polypeptide comprises at least one sequence selected from the following group of sequences:
i) an amino acid sequence according to SEQ ID NO:1;
ii) a derivative of SEQ ID NO:1 exhibiting at least 80% sequence identity with SEQ ID NO:1,
iii) an amino acid sequence according to SEQ ID NO:2;
iv) a derivative of SEQ ID NO:2 exhibiting at least 80% sequence identity with SEQ ID NO:2,
v) an amino acid sequence according to SEQ ID NO:3;
vi) a derivative of SEQ ID NO:3 exhibiting at least 77% sequence identity with SEQ ID NO:3;
vii) an amino acid sequence according to SEQ ID NO:4; and
viii) a derivative of SEQ ID NO:4 exhibiting at least 80% sequence identity with SEQ ID NO:4.

The provisos regarding the polypeptide of the invention (see above) do not necessarily but preferably apply for this aspect of the present invention as well. Again, disclosure set out above for the inventive polypeptide is contemplated for the polypeptide (and the nucleic acid encoding such polypeptide, the vector comprising such nucleic acid, the host cell comprising such nucleic acid or vector, and/or the composition comprising such polypeptide, nucleic acid, vector, and/or host cell) to be used in said method of controlling the growth of bacteria of the genus *Salmonella* in animals, in particular in livestock, companion animal and/or aquaculture, as well. In particular, disclosure detailing SEQ ID NO:2 and its derivatives is contemplated as embodiments for the polypeptide to be used in the method of controlling the growth of bacteria of the genus *Salmonella* in animals (see for instance examples of particularly preferred derivatives of SEQ ID NO:2 above, such as SEQ ID NO:13). In some embodiments, the polypeptide to be used in the inventive method of treatment is an inventive polypeptide as set forth above, i.e. the present invention relates to a method of controlling the growth of bacteria of the genus *Salmonella* in animals, e.g. in livestock, companion animal and/or aquaculture, the method comprising contacting the animal (e.g. livestock, companion animal and/or aquaculture) with a polypeptide according to the present invention, a nucleic acid according to the present invention, a vector according to the present invention, a host cell according to the present invention, and/or the composition according to the present invention. Said inventive method of controlling the growth of bacteria of the genus *Salmonella* is preferably a non-therapeutic method, in particular where bacteria of the genus *Salmonella* are non-pathogenic for the respective animal (e.g. livestock, companion animal and/or aquaculture) or are only present in (for the animal) sub-pathogenic concentration, i.e. would not require medical treatment of said animal (i.e. livestock, companion animal and/or aquaculture).

### Examples

In the following, specific examples illustrating various embodiments and aspects of the invention are presented. However, the present invention shall not to be limited in scope by the specific embodiments described herein. Indeed, various modifications of the invention in addition to those described herein will become readily apparent to those skilled in the art from the foregoing description, accompanying figures and the examples below. All such modifications fall within the scope of the appended claims.

### Example 1: Data on antibacterial activity on S. Typhimurium LT2 from prior art polypeptides published in Briers et al, MBio. 2014 Jul 1:5(4):e01379-14.

Briers et al. published antibacterial activity of various fusion proteins for *S*. Typhimurium LT2 (see supplemental table 5). The data are summarized in table 3 (fusion variants of endolysin OBPgp279) and in table 4 (fusion variants of endolysin PVP-SE1gp146).

**Table 3: Fusion variants of endolysin OBPgp279**

| **Fusion** | **Polypeptide** | **w/o EDTA** | **0.5 mM EDTA** |
|---|---|---|---|
| | | | 0.05 ± 0.05 |
| **PCNP** | **LoGT-001** | 0.23 ± 0.03 | 0.91 ± 0.04 |
| **MW1** | **LoGT-002** | 0.15 ± 0.07 | 0.41 ± 0.08 |
| **HPP** | **LoGT-003** | 0.09 ± 0.01 | 0.52 ± 0.04 |
| **MW2** | **LoGT-004** | 0.12 ± 0.06 | 0.36 ± 0.07 |
| **α₄** | **LoGT-005** | 0.17 ± 0.08 | 0.43 ± 0.09 |
| **Parasinl** | **LoGT-006** | 0.20 ± 0.14 | 0.60 ± 0.05 |
| **Lycotoxinl** | **LoGT-007** | 0.01 ± 0.11 | 0.51 ± 0.04 |

**Table 4: Fusion variants of endolysin PVP-SE1gp146**

| **Fusion** | **Polypeptide** | ***S*. Typhimurium LT2** | **0.5 mM EDTA** |
|---|---|---|---|
| | | | 0.05 ± 0.05 |
| **PCNP** | **LoGT-008** | 0.30 ± 0.10 | 0.73 ± 0.30 |
| **MW1** | **LoGT-009** | 0.10 ± 0.07 | 0.27 ± 0.15 |
| **HPP** | **LoGT-010** | 0.24 ± 0.16 | 0.47 ± 0.07 |
| **MW2** | **LoGT-011** | 0.12 ±0.12 | 0.41 ± 0.25 |
| **α₄** | **LoGT-012** | 0.14 ± 0.12 | 0.47 ± 0.24 |
| **Parasinl** | **LoGT-013** | 0.04 ± 0.03 | 0.59 ± 0.38 |
| **Lycotoxinl** | **LoGT-014** | 0.20 ± 0.22 | 0.87 ± 0.28 |

### Example 2: Data on antibacterial activity of selected inventive polypeptides on S. Typhimurium LT2

The inventors of the present invention have tested antibacterial activity of polypeptides comprising SEQ ID NO: 126 or SEQ ID NO: 129 (and further comprising a His-tag for purification reasons) on *S*. Typhimurium LT2. A first set of experiments was conducted in PBS buffer.

Briefly, exponentially growing cells of *Salmonella* Typhimurium LT2 were diluted 1:100 in 5 mM HEPES pH 7.4. Subsequently, 100 µl of cells were incubated for 30 min at room temperature with 50 µl protein solution dissolved in 1 x PBS (170 mM NaCl, 3 mM KCl, 12.7 mM Na₂HPO₄, 2.2 mM KH₂PO₄, pH 7.4) and 50 µl of 5 mM HEPES pH 7.4 or 50 µL of EDTA (final concentration 0.5 mM) yielding a final protein concentration of 1.313 µM. Cells were serially diluted in 1 x PBS after incubation and plated on LB agar. As a negative control, cells were incubated in the same buffer without any protein and additionally plated. Cell colonies were counted after incubation over night at 37°C and the antibacterial activity was calculated in logarithmic units (=log10No/Ni with NO = number of untreated colonies and Ni = number of treated colonies).

The results are shown in table 5 below.

**Table 5: Antibacterial activity of inventive polypeptides in PBS buffer**

| **SEQ ID NO** | **w/o EDTA** | **0.5 mM EDTA** |
|---|---|---|
| SEQ ID NO: 126 | 2.75 | ≥ 4.00 |
| SEQ ID NO: 129 | 1.61 | 2.29 |

In a further set of experiments, antibacterial activity in HEPES-buffer on *S*. Typhimurium LT2 was determined.

Briefly, exponentially growing cells of *Salmonella* Typhimurium LT2 were diluted 1:100 in 5 mM HEPES pH 7.4. Subsequently, 100 µl of cells were incubated for 30 min at room temperature with 50 µl protein solution dissolved in 20 mM HEPES, 500 mM NaCl pH 7.4 and 50 µl of 5 mM HEPES pH 7.4 or 50 µL of EDTA (final concentration 0.5 mM) yielding a final protein concentration of 1.313 µM. Cells were serially diluted in 1 x PBS after incubation and plated on LB agar. As a negative control, cells were incubated in the same buffer without any protein and additionally plated. Cell colonies were counted after incubation over night at 37°C and the antibacterial activity was calculated in logarithmic units (=log10No/Ni with NO = number of untreated colonies and Ni = number of treated colonies).

The results are shown in table 6 below.

**Table 6: Antibacterial activity of inventive polypeptides in HEPES buffer**

| **SEQ ID NO** | **w/o EDTA** | **0.5 mM EDTA** |
|---|---|---|
| SEQ ID NO: 126 | 0.95 | 3.62 |
| SEQ ID NO: 129 | 1.24 | 1.61 |

In summary, antibacterial activity of the inventive polypeptides comprising the amino acids sequence of SEQ ID NO: 126 or SEQ ID NO: 129 on *S*. Typhimurium LT2 surprisingly exceeded the antibacterial activity achieved with similar compounds in the art significantly.

### Example 3: Data on antibacterial activity of further inventive polypeptides on S. Typhimurium LT2

In another set of experiments, the inventors of the present invention have also tested antibacterial activity of further fusion proteins comprising the components of the polypeptides of the present invention. The polypeptides tested did comprise the sequences of SEQ ID NO: 138, SEQ ID NO: 141, SEQ ID NO: 132, SEQ ID NO: 144, and SEQ ID NO: 147 (and further comprising a His-tag for purification reasons) and were tested on S. Typhimurium LT2. The tested compounds all comprise at least one component which can be advantageously used according to the present invention (SEQ ID NO:1, SEQ ID NO:3 and (SEQ ID NO:4) for treating in particular *Salmonella* bacteria.

The experiment was done as set out in Example 2.

The results are shown in table 7 below.

**Table 7: Antibacterial activity of inventive polypeptides in HEPES buffer**

| **SEQ ID NO** | **w/o EDTA** | **0.5 mM EDTA** |
|---|---|---|
| SEQ ID NO: 138 | 0.76 | 0.88 |
| SEQ ID NO: 141 | 1.71 | 1.47 |
| SEQ ID NO: 132 | 2.74 | >5 |
| SEQ ID NO: 144 | 0.15 | 3.24 |
| SEQ ID NO: 147 | 0.33 | 0.98 |

In summary, antibacterial activity of the inventive polypeptides comprising the amino acids sequence of SEQ ID NO: 138, SEQ ID NO: 141, SEQ ID NO: 132, SEQ ID NO: 144, and SEQ ID NO: 147 on S. Typhimurium LT2 reliably exceeded the antibacterial activity achieved with similar compounds in the art, indicating that the components used (SEQ ID NO:1, SEQ ID NO:3 and SEQ ID NO:4) all can provide for superior activity against Salmonella bacteria in comparison to the compounds of the prior art. Noteworthy, the polypeptide comprising two components of the invention (SEQ ID NO: 132) yielded again by far the best results.

### Example 4: Data on antibacterial activity of selected inventive polypeptide on S. Typhimurium (DSM 17058)

The inventors of the present invention have tested antibacterial activity of an inventive polypeptide comprising SEQ ID NO: 135 (and further comprising a His-tag for purification reasons) on S. Typhimurium (DSM 17058).

The experiment was done as set out in Example 2.

The results are shown in table 8 below.

**Table 8: Antibacterial activity of inventive polypeptides in HEPES buffer**

| **SEQ ID NO** | **w/o EDTA** | **0.5 mM EDTA** |
|---|---|---|
| SEQ ID NO: 135 | 3.91 | 3.88 |

Hence, antibacterial activity of the inventive polypeptide comprising the amino acid sequence of SEQ ID NO: 135 on S. Typhimurium (DSM 17058) exceeded the antibacterial activity achieved with similar compounds in the art, indicating that the endolysin component used (SEQ ID NO:13) provides for superior activity against Salmonella bacteria in comparison to the respective components of the prior art polypeptides (i.e. endolysin components OBPgp279 and PVP-SE1gp146).

### Example 5: Data on minimal inhibitory concentration of selected inventive polypeptide on S. Typhimurium (DSM 17058)

The inventors of the present invention have tested antibacterial activity in terms of minimal inhibitory concentration (MIC) of an inventive polypeptide comprising SEQ ID NO: 132 (and further comprising a His-tag for purification reasons) on *S*. Typhimurium (DSM 17058).

Briefly, Bacteria were grown in (Luria-Bertani) medium and diluted 1:10 in Mueller-Hinton medium. At optical density OD₆₀₀ of about 0.6 bacteria were diluted in the same medium 1:10 followed by a 1:500 dilution in the same medium. Protein solutions were pipetted into a 96 well plate, using different concentrations of proteins and an end volume of 20 µl including 500 µM EDTA final concentration. 180 µl of bacterial cells or a medium (Mueller-Hinton) control were given to the 96 well plate and mixed. The plate was incubated for 18-22 hours at 37°C and the bacterial growth was determined measuring the OD₆₀₀ values of the wells. The MIC which is the protein concentration of the well which showed the same OD₆₀₀ value as determined for the no-bacteria control.

The results in form of minimal inhibitory concentration (MIC) are shown in table 9 below.

**Table 9: Antibacterial activity of inventive polypeptides in PBS buffer**

| **SEQ ID NO** | **0.5 mM EDTA** |
|---|---|
| SEQ ID NO: 132 | ≤5 |

### Example 6: Data on antibacterial activity of selected inventive polypeptide on various Salmonella Enteritidis serovars in presence of salt

The inventors of the present invention have tested antibacterial activity of the polypeptide comprising SEQ ID NO: 132 (and further comprising a His-tag for purification reasons) on various Salmonella serovars in presence of physiological salt levels.

Briefly, exponentially growing cells of Salmonella Enteritidis were diluted 1:10 in 20 mM HEPES pH 7.4, 150 mM NaCl. Subsequently, 50 µl of cells were incubated for 1 h at 37°C with 50 µl protein solution dissolved in 20 mM HEPES, 150 mM NaCl pH 7.4 yielding a final protein concentration of 100 µg/ml. Cells were serially diluted in 1 x PBS after incubation and plated on LB agar. As a negative control, cells were incubated in the same buffer without any protein and additionally plated. Cell colonies were counted after incubation over night at 37 °C and the antibacterial activity was calculated in logarithmic units (=log10No/Ni with NO = number of untreated colonies and Ni = number of treated colonies).

The results in form of minimal inhibitory concentration (MIC) are shown in table 10 below.

**Table 10: Antibacterial activity of inventive polypeptides under physiological conditions**

| **SEQ ID NO** | **Serovar** | **w/o EDTA** |
|---|---|---|
| SEQ ID NO: 132 | Salmonella Enteritidis RKI 14-00409 | 3.5 |
| SEQ ID NO: 132 | Salmonella Enteritidis LGL-246 | 4.5-5.5 |
| SEQ ID NO: 132 | Salmonella Enteritidis 29/2014 | 6 |

The polypeptide comprising SEQ ID NO: 132 thus shows significant antibacterial activity in presence of physiological salt conditions.

### Example 7: Thermostability

The inventors of the present invention have tested antibacterial activity of the polypeptide comprising SEQ ID NO: 126 on *S*. Typhimurium (DSM 17058) with increasing temperatures.

Briefly, prior to the determination of the MIC, the protein solution is heated to given temperatures for 20 min. Subsequently, the protein solution is stored on ice to reach a temperature of 4°C. Bacteria were grown in (Luria-Bertani) medium and diluted 1:10 in Mueller-Hinton medium. At optical density OD₆₀₀ of about 0.6 bacteria were diluted in the same medium 1:10 followed by a 1:500 dilution in the same medium. Protein solutions were pipetted into a 96 well plate, using different concentrations of proteins and an end volume of 20 µl including 500 µM EDTA final concentration. 180 µl of bacterial cells or a medium (Mueller-Hinton) control were given to the 96 well plate and mixed. The plate was incubated for 18-22 hours at 37°C and the bacterial growth was determined measuring the OD₆₀₀ values of the wells. The MIC which is the protein concentration of the well which showed the same OD₆₀₀ value as determined for the no-bacteria control.

The results in form of minimal inhibitory concentration (MIC) are shown in table 11 below.

**Table 11: Antibacterial activity of inventive polypeptide with increasing temperature**

| | MIC [µg/ml] |
|---|---|
| Temp. [°C] | SEQ ID NO: 126 |
| RT | 6 |
| 70 | 12 |
| 74 | 10 |
| 78 | 10 |
| 82 | 10 |
| 86 | 10 |
| 90 | 8-10 |
| 94 | 8 |

The polypeptide comprising SEQ ID NO: 126 thus shows extreme thermostability with significant antibacterial activity even at very high temperatures.

Similar results were obtained when using the same endolysin with another peptide or when using a variant of the endolysin (e.g. SEQ ID NO:7) with such peptides.

Particularly preferred embodiments of the invention are set forth once more in items 1 to 43 below:
1. Polypeptide comprising a first and a second amino acid sequence, wherein the first amino acid sequence is an endolysin, and wherein the second amino acid sequence is an antimicrobial peptide, cationic peptide, hydrophobic peptide, amphiphatic peptide or sushi peptide, and wherein the polypeptide comprises at least one sequence selected from the following group of sequences:
   i) an amino acid sequence according to SEQ ID NO:1;
   ii) a derivative of SEQ ID NO:1 exhibiting at least 80% sequence identity with SEQ ID NO:1,
   iii) an amino acid sequence according to SEQ ID NO:3;
   iv) a derivative of SEQ ID NO:3 exhibiting at least 77% sequence identity with SEQ ID NO:3;
   v) an amino acid sequence according to SEQ ID NO:4; and
   vi) a derivative of SEQ ID NO:4 exhibiting at least 80% sequence identity with SEQ ID NO:4.
2. The polypeptide according to item 1, wherein the first amino acid sequence is selected from the group consisting of:
   i) an amino acid sequence according to SEQ ID NO:1;
   ii) a derivative of SEQ ID NO:1 exhibiting at least 80% sequence identity with SEQ ID NO:1,
   iii) an amino acid sequence according to SEQ ID NO:2; and
   iv) a derivative of SEQ ID NO:2 exhibiting at least 80% sequence identity with SEQ ID NO:2,
3. The polypeptide according to item 2, wherein the derivative of SEQ ID NO:2 exhibits at least 85%, at least 87,5%, at least 90%, at least 91%, at least 92%, at least 93%, at least 94%, at least 95%, at least 96%, at least 97%, at least 98%, at least 99%, or more than 99% sequence identity with SEQ ID NO:2.
4. The polypeptide according to item 2 or item 3, wherein said derivative of SEQ ID NO:2 comprises a sequence according to SEQ ID NO:8, in particular wherein said derivative comprises a sequence according to SEQ ID NO:13.
5. The polypeptide according any one of items 1 to 4, wherein the derivative of SEQ ID NO:1 exhibits at least 85%, at least 87,5%, at least 90%, at least 91%, at least 92%, at least 93%, at least 94%, at least 95%, at least 96%, at least 97%, at least 98%, at least 99%, or more than 99% sequence identity with SEQ ID NO:1.
6. The polypeptide according to anyone of the preceding items, wherein the second amino acid sequence is selected from the group consisting of:
   i) an amino acid sequence according to SEQ ID NO:3;
   ii) a derivative of SEQ ID NO:3 exhibiting at least 77% sequence identity with SEQ ID NO:3;
   iii) an amino acid sequence according to SEQ ID NO:4; and
   iv) a derivative of SEQ ID NO:4 exhibiting at least 80% sequence identity with SEQ ID NO:4.
7. The polypeptide according to item 6, wherein the derivative of SEQ ID NO:3 exhibits at least 81%, at least 86%, at least 90%, or at least 95% sequence identity with SEQ ID NO:3, or wherein the derivative of SEQ ID NO:4 exhibits at least 83%, at least 86%, at least 90%, at least 93%, or at least 96% sequence identity with SEQ ID NO:4.
8. The polypeptide according to anyone of items 1 to 7, wherein the first amino acid sequence is SEQ ID NO:1 or said derivative thereof, and wherein the second amino acid sequence is SEQ ID NO:3 or said derivative thereof, or wherein the first amino acid sequence is SEQ ID NO:2 or said derivative thereof, and wherein the second amino acid sequence is SEQ ID NO:4 or said derivative thereof.
9. The polypeptide according to anyone of items 1 to 8, wherein said derivative of SEQ ID NO:3 is a fragment of SEQ ID NO:3, in particular a fragment according to SEQ ID NO:37.
10. The polypeptide according to item 1, wherein
   i) the first amino acid sequence is SEQ ID NO:1 and the second amino acid sequence is SEQ ID NO:3, or
   ii) wherein the first amino acid sequence is SEQ ID NO:13 and the second amino acid sequence is SEQ ID NO:4.
11. The polypeptide according to item 13, wherein the polypeptide comprises an amino acid sequence according to SEQ ID NO: 126, SEQ ID NO: 127, SEQ ID NO: 129 or SEQ ID NO: 130.
12. The polypeptide according to any one of items 1 to 11, wherein the polypeptide comprises an amino acid sequence as encoded by a nucleic acid sequence according to SEQ ID NO:153 or according to SEQ ID NO:154.
13. The polypeptide according to any one of the preceding items, wherein the polypeptide degrades the peptidoglycan of *Salmonella* bacteria.
14. Use of the polypeptide according to any one of items 1 to 13 as an antimicrobial in food, as an antimicrobial in feed, as an antimicrobial in cosmetics, or as disinfecting agent.
15. Method of controlling the growth of bacteria of the genus *Salmonella* in animals, in particular in livestock, companion animal and/or aquaculture, the method comprising contacting said animal, in particular the livestock, companion animal and/or aquaculture, with a polypeptide, wherein the polypeptide comprises a first and a second amino acid sequence, wherein the first amino acid sequence is an endolysin, and wherein the second amino acid sequence is an antimicrobial peptide, amphiphatic peptide, cationic peptide, hydrophobic peptide, sushi peptide or defensin, and wherein the polypeptide comprises at least one sequence selected from the following group of sequences:
   i) an amino acid sequence according to SEQ ID NO:1;
   ii) a derivative of SEQ ID NO:1 exhibiting at least 80% sequence identity with SEQ ID NO:1,
   iii) an amino acid sequence according to SEQ ID NO:2;
   iv) a derivative of SEQ ID NO:2 exhibiting at least 80% sequence identity with SEQ ID NO:2,
   v) an amino acid sequence according to SEQ ID NO:3;
   vi) a derivative of SEQ ID NO:3 exhibiting at least 77% sequence identity with SEQ ID NO:3;
   vii) an amino acid sequence according to SEQ ID NO:4; and
   viii) a derivative of SEQ ID NO:4 exhibiting at least 80% sequence identity with SEQ ID NO:4.
16. The method according to item 15, wherein the polypeptide is a polypeptide according to any one of items 1 to 13.
17. Polypeptide comprising a first and a second amino acid sequence, wherein the first amino acid sequence is an endolysin, and wherein the second amino acid sequence is an antimicrobial peptide, cationic peptide, hydrophobic peptide, amphiphatic peptide or sushi peptide, and wherein the polypeptide comprises at least one sequence selected from the following group of sequences:
   i) an amino acid sequence according to SEQ ID NO:1;
   ii) a derivative of SEQ ID NO:1 exhibiting at least 80% sequence identity with SEQ ID NO:1, with the proviso that the polypeptide may not comprise the sequence of SEQ ID NO:5, if the polypeptide comprises ii), but none of iii), iv), v) or vi),
   iii) an amino acid sequence according to SEQ ID NO:3;
   iv) a derivative of SEQ ID NO:3 exhibiting at least 77% sequence identity with SEQ ID NO:3;
   v) an amino acid sequence according to SEQ ID NO:4; with the proviso that the polypeptide may in this case not comprise the sequence of SEQ ID NO:6 in parallel; and
   vi) a derivative of SEQ ID NO:4 exhibiting at least 80% sequence identity with SEQ ID NO:4.
18. The polypeptide according to item 17, wherein the first amino acid sequence is selected from the group consisting of:
   i) an amino acid sequence according to SEQ ID NO:1;
   ii) a derivative of SEQ ID NO:1 exhibiting at least 80% sequence identity with SEQ ID NO:1,
   iii) an amino acid sequence according to SEQ ID NO:2; and
   iv) a derivative of SEQ ID NO:2 exhibiting at least 80% sequence identity with SEQ ID NO:2,
19. The polypeptide according to item 18, wherein the derivative of SEQ ID NO:2 exhibits at least 85%, at least 87,5%, at least 90%, at least 91%, at least 92%, at least 93%, at least 94%, at least 95%, at least 96%, at least 97%, at least 98%, at least 99%, or more than 99% sequence identity with SEQ ID NO:2.
20. The polypeptide according to item 18 or item 19, wherein said derivative of SEQ ID NO:2 comprises a sequence according to SEQ ID NO:8, in particular wherein said derivative comprises a sequence according to SEQ ID NO:13.
21. The polypeptide according any one of items 17 to 20, wherein the derivative of SEQ ID NO:1 exhibits at least 85%, at least 87,5%, at least 90%, at least 91%, at least 92%, at least 93%, at least 94%, at least 95%, at least 96%, at least 97%, at least 98%, at least 99%, or more than 99% sequence identity with SEQ ID NO:1.
22. The polypeptide according any one of items 17 to 21, wherein the derivative of SEQ ID NO:1 exhibits at least 98%, at least 99%, or more than 99% sequence identity with SEQ ID NO:1.
23. The polypeptide according any one of items 17 to 21, wherein the derivative of SEQ ID NO:1 is an amino acid sequence according to SEQ ID NO:7.
24. The polypeptide according to anyone of the preceding items 17 to 23, wherein the second amino acid sequence is selected from the group consisting of:
   i) an amino acid sequence according to SEQ ID NO:3;
   ii) a derivative of SEQ ID NO:3 exhibiting at least 77% sequence identity with SEQ ID NO:3;
   iii) an amino acid sequence according to SEQ ID NO:4; and
   iv) a derivative of SEQ ID NO:4 exhibiting at least 80% sequence identity with SEQ ID NO:4.
25. The polypeptide according to item 24, wherein the derivative of SEQ ID NO:3 exhibits at least 81%, at least 86%, at least 90%, or at least 95% sequence identity with SEQ ID NO:3, or wherein the derivative of SEQ ID NO:4 exhibits at least 83%, at least 86%, at least 90%, at least 93%, or at least 96% sequence identity with SEQ ID NO:4.
26. The polypeptide according anyone of the preceding items 17 to 25, wherein the polypeptide comprises an amino acid sequence according to SEQ ID NO:4; but does not comprise the amino acid sequence according to SEQ ID NO:2.
27. The polypeptide according to anyone of items 17 to25, wherein the first amino acid sequence is SEQ ID NO:1 or said derivative thereof, and wherein the second amino acid sequence is SEQ ID NO:3 or said derivative thereof, or wherein the first amino acid sequence is SEQ ID NO:1 or said derivative thereof, and wherein the second amino acid sequence is SEQ ID NO:4 or said derivative thereof, or wherein the first amino acid sequence is SEQ ID NO:2 or said derivative thereof, and wherein the second amino acid sequence is SEQ ID NO:3 or said derivative thereof or wherein the first amino acid sequence is SEQ ID NO:2, and wherein the second amino acid sequence is said derivative of SEQ ID NO:4, or wherein the first amino acid sequence is said derivative of SEQ ID NO:2, and wherein the second amino acid sequence is SEQ ID NO:4 or said derivative thereof.
28. The polypeptide according to anyone of items 17 to 27, wherein said derivative of SEQ ID NO:3 is a fragment of SEQ ID NO:3, in particular a fragment according to SEQ ID NO:37.
29. The polypeptide according to item 17, wherein
   i) the first amino acid sequence is SEQ ID NO:1 and the second amino acid sequence is SEQ ID NO:3,
   ii) the first amino acid sequence is SEQ ID NO:1 and the second amino acid sequence is SEQ ID NO:4,
   iii) the first amino acid sequence is SEQ ID NO:13 and the second amino acid sequence is SEQ ID NO:3, or
   iii) wherein the first amino acid sequence is SEQ ID NO:13 and the second amino acid sequence is SEQ ID NO:4.
30. The polypeptide according to item 29, wherein the polypeptide comprises an amino acid sequence according to SEQ ID NO: 126, SEQ ID NO: 127, SEQ ID NO: 129, SEQ ID NO: 130, SEQ ID NO: 132, SEQ ID NO: 133, SEQ ID NO: 135 or SEQ ID NO: 136.
31. The polypeptide according to any one of items 17 to 27, wherein the polypeptide comprises an amino acid sequence as encoded by a nucleic acid sequence according to SEQ ID NO:153 or according to SEQ ID NO:154.
32. The polypeptide according to any one of the preceding items 17 to 31, wherein the polypeptide degrades the peptidoglycan of *Salmonella* bacteria.
33. Nucleic acid encoding a polypeptide according to any one of items 17 to 32.
34. Vector comprising a nucleic acid according to item 33.
35. Host cell comprising a polypeptide according to any one of items 17 to 32, a nucleic acid according to item 33, and/or a vector according to item 34.
36. Composition comprising a polypeptide according to any one of items 17 to 32, a nucleic acid according to item 33, a vector according to item 34 and/or a host cell according to item 35.
37. Composition according to item 36, wherein the composition is a pharmaceutical composition comprising a pharmaceutical acceptable diluent, excipient or carrier.
38. A polypeptide according to any one of items 17 to 32, a nucleic acid according to item 33, a vector according to item 34, a host cell according to item 35 or a composition according to item 36 or item 37 for use in a method for treatment of the human or animal body by surgery or therapy or in diagnostic methods practiced on the human or animal body.
39. A polypeptide, a nucleic acid encoding such polypeptide, a vector comprising such nucleic acid, a host cell comprising such polypeptide, nucleic acid and/or vector, or a composition comprising such polypeptide, nucleic acid, vector, and/or host cell for use in a method for treatment or prevention of infections caused by bacteria of the genus *Salmonella*, wherein the polypeptide comprises a first and a second amino acid sequence, wherein the first amino acid sequence is an endolysin, and wherein the second amino acid sequence is an antimicrobial peptide, amphiphatic peptide, cationic peptide, hydrophobic peptide, sushi peptide or defensin, and wherein the polypeptide comprises at least one sequence selected from the following group of sequences:
   i) an amino acid sequence according to SEQ ID NO:1;
   ii) a derivative of SEQ ID NO:1 exhibiting at least 80% sequence identity with SEQ ID NO:1,
   iii) an amino acid sequence according to SEQ ID NO:2;
   iv) a derivative of SEQ ID NO:2 exhibiting at least 80% sequence identity with SEQ ID NO:2,
   v) an amino acid sequence according to SEQ ID NO:3;
   vi) a derivative of SEQ ID NO:3 exhibiting at least 77% sequence identity with SEQ ID NO:3;
   vii) an amino acid sequence according to SEQ ID NO:4; and
   viii) a derivative of SEQ ID NO:4 exhibiting at least 80% sequence identity with SEQ ID NO:4.
40. The polypeptide, nucleic acid, vector, host cell or composition for use in a method for treatment or prevention of infections caused by bacteria of the genus *Salmonella* according to item 39, wherein the polypeptide is a polypeptide according to any one of items 17 to 32, wherein the nucleic acid is a nucleic acid according to item 33, wherein the vector is a vector according to item 34, wherein the host cell is a host cell according to item 35 or wherein the composition is a composition according to item 36 or item 37.
41. Use of the polypeptide according to any one of items 17 to 32, a nucleic acid according to item 33, a vector according to item 34, a host cell according to item 35 or a composition according to item 36 or item 37 as an antimicrobial in food, as an antimicrobial in feed, as an antimicrobial in cosmetics, or as disinfecting agent.
42. Non-therapeutic method of controlling the growth of bacteria of the genus *Salmonella* in animals, in particular in livestock, companion animal and/or aquaculture, the method comprising contacting said animal, in particular the livestock, companion animal and/or aquaculture, with a polypeptide, a nucleic acid encoding such polypeptide, a vector comprising such nucleic acid, a host cell comprising such polypeptide, nucleic acid and/or vector, or a composition comprising such polypeptide, nucleic acid, vector, and/or host cell, wherein the polypeptide comprises a first and a second amino acid sequence, wherein the first amino acid sequence is an endolysin, and wherein the second amino acid sequence is an antimicrobial peptide, amphiphatic peptide, cationic peptide, hydrophobic peptide, sushi peptide or defensin, and wherein the polypeptide comprises at least one sequence selected from the following group of sequences:
   i) an amino acid sequence according to SEQ ID NO:1;
   ii) a derivative of SEQ ID NO:1 exhibiting at least 80% sequence identity with SEQ ID NO:1,
   iii) an amino acid sequence according to SEQ ID NO:2;
   iv) a derivative of SEQ ID NO:2 exhibiting at least 80% sequence identity with SEQ ID NO:2,
   v) an amino acid sequence according to SEQ ID NO:3;
   vi) a derivative of SEQ ID NO:3 exhibiting at least 77% sequence identity with SEQ ID NO:3;
   vii) an amino acid sequence according to SEQ ID NO:4; and
   viii) a derivative of SEQ ID NO:4 exhibiting at least 80% sequence identity with SEQ ID NO:4.
43. The method according to item 42, wherein the polypeptide is a polypeptide according to any one of items 17 to 32.

## Claims

1. Polypeptide comprising a first and a second amino acid sequence, wherein the first amino acid sequence is an endolysin, and wherein the second amino acid sequence is an antimicrobial peptide, cationic peptide, hydrophobic peptide, amphiphatic peptide or sushi peptide, and wherein the polypeptide comprises at least one sequence selected from the following group of sequences:
i) an amino acid sequence according to SEQ ID NO:4; with the proviso that the polypeptide may in this case not comprise the sequence of SEQ ID NO:6 in parallel; and
ii) a derivative of SEQ ID NO:4 exhibiting at least 80% sequence identity with SEQ ID NO:4.

2. The polypeptide according to claim 1, wherein the derivative of SEQ ID NO:4 exhibits at least 83%, at least 86%, at least 90%, at least 93%, or at least 96% sequence identity with SEQ ID NO:4.

3. The polypeptide according anyone of the preceding claims, wherein the polypeptide comprises an amino acid sequence according to SEQ ID NO:4; but does not comprise the amino acid sequence according to SEQ ID NO:2.

4. The polypeptide according to anyone of claims 1 to 3, wherein the first amino acid sequence is SEQ ID NO:1 or a derivative thereof exhibiting at least 80% sequence identity with SEQ ID NO:1, or wherein the first amino acid sequence is SEQ ID NO:2, and wherein the second amino acid sequence is said derivative of SEQ ID NO:4, or wherein the first amino acid sequence is said derivative of SEQ ID NO:2, and wherein the second amino acid sequence is SEQ ID NO:4 or said derivative thereof.

5. The polypeptide according to claim 1, wherein
i) the first amino acid sequence is SEQ ID NO:1 and the second amino acid sequence is SEQ ID NO:4, or
ii) wherein the first amino acid sequence is SEQ ID NO:13 and the second amino acid sequence is SEQ ID NO:4.

6. The polypeptide according to claim 1, wherein the polypeptide comprises an amino acid sequence according to SEQ ID NO: 128, SEQ ID NO: 129, SEQ ID NO: 130, SEQ ID NO: 131,SEQ ID NO: 132, SEQ ID NO: 133, SEQ ID NO: 137, SEQ ID NO: 138, SEQ ID NO: 139, SEQ ID NO: 140, SEQ ID NO: 141, or SEQ ID NO: 142.

7. The polypeptide according to any one of the preceding claims, wherein the polypeptide degrades the peptidoglycan of *Salmonella* bacteria.

8. Nucleic acid encoding a polypeptide according to any one of claims 1 to 7.

9. Vector comprising a nucleic acid according to claim 8.

10. Host cell comprising a polypeptide according to any one of claims 1 to 7, a nucleic acid according to claim 8, and/or a vector according to claim 9.

11. A polypeptide according to any one of claims 1 to 7, a nucleic acid according to claim 8, a vector according to claim 9, or a host cell according to claim 10 for use in a method for treatment of the human or animal body by surgery or therapy or in diagnostic methods practiced on the human or animal body.

12. A polypeptide, a nucleic acid encoding such polypeptide, a vector comprising such nucleic acid, a host cell comprising such polypeptide, nucleic acid and/or vector, or a composition comprising such polypeptide, nucleic acid, vector, and/or host cell for use in a method for treatment or prevention of infections caused by bacteria of the genus *Salmonella*, wherein the polypeptide comprises a first and a second amino acid sequence, wherein the first amino acid sequence is an endolysin, and wherein the second amino acid sequence is an antimicrobial peptide, amphiphatic peptide, cationic peptide, hydrophobic peptide, sushi peptide or defensin, and wherein the polypeptide comprises at least one sequence selected from the following group of sequences:
i) an amino acid sequence according to SEQ ID NO:4; and
ii) a derivative of SEQ ID NO:4 exhibiting at least 80% sequence identity with SEQ ID NO:4.

13. Use of the polypeptide according to any one of claims 1 to 7, a nucleic acid according to claim 8, a vector according to claim 9, or a host cell according to claim 10 as an antimicrobial in food, as an antimicrobial in feed, as an antimicrobial in cosmetics, or as disinfecting agent.

14. Non-therapeutic method of controlling the growth of bacteria of the genus *Salmonella* in animals, in particular in livestock, companion animal and/or aquaculture, the method comprising contacting said animal, in particular the livestock, companion animal and/or aquaculture, with a polypeptide, a nucleic acid encoding such polypeptide, a vector comprising such nucleic acid, a host cell comprising such polypeptide, nucleic acid and/or vector, or a composition comprising such polypeptide, nucleic acid, vector, and/or host cell, wherein the polypeptide comprises a first and a second amino acid sequence, wherein the first amino acid sequence is an endolysin, and wherein the second amino acid sequence is an antimicrobial peptide, amphiphatic peptide, cationic peptide, hydrophobic peptide, sushi peptide or defensin, and wherein the polypeptide comprises at least one sequence selected from the following group of sequences:
i) an amino acid sequence according to SEQ ID NO:4;
ii) a derivative of SEQ ID NO:4 exhibiting at least 80% sequence identity with SEQ ID NO:4.

15. The method according to claim 14, wherein the polypeptide is a polypeptide according to any one of claims 1 to 7.


SEQUENCE LISTING

<110>  Lysando AG

<120>  New antimicrobial agents against Salmonella bacteria

<130>  LYS-034 PCT

<150>  EP16167543
<151>  2016-04-28

<160>  154

<170>  PatentIn version 3.5

<210>  1
<211>  136
<212>  PRT
<213>  Unknown

<220>
<223>  S394 endolysin without N-terminal methionine

<400>  1

Ser Phe Lys Phe Gly Lys Asn Ser Glu Lys Gln Leu Ala Thr Val Lys
1               5                   10                  15

Pro Glu Leu Gln Lys Val Ala Arg Arg Ala Leu Glu Leu Ser Pro Tyr
            20                  25                  30

Asp Phe Thr Ile Val Gln Gly Ile Arg Thr Val Ala Gln Ser Ala Gln
        35                  40                  45

Asn Ile Ala Asn Gly Thr Ser Phe Leu Lys Asp Pro Ser Lys Ser Lys
    50                  55                  60

His Val Thr Gly Asp Ala Ile Asp Phe Ala Pro Tyr Ile Asn Gly Lys
65                  70                  75                  80

Ile Asp Trp Lys Asp Leu Glu Ala Phe Trp Ala Val Lys Lys Ala Phe
                85                  90                  95

Glu Gln Ala Gly Lys Glu Leu Gly Ile Lys Leu Arg Phe Gly Ala Asp
            100                 105                 110

Trp Asn Ser Ser Gly Asp Tyr His Asp Glu Ile Asp Arg Gly Thr Tyr
        115                 120                 125

Asp Gly Gly His Val Glu Leu Val
    130                 135

<210>  2
<211>  259

<212>  PRT
<213>  unknown

<220>
<223>  KZ144 endolysin without N-terminal methionine

<400>  2

Lys Val Leu Arg Lys Gly Asp Arg Gly Asp Glu Val Cys Gln Leu Gln
1               5                   10                  15

Thr Leu Leu Asn Leu Cys Gly Tyr Asp Val Gly Lys Pro Asp Gly Ile
            20                  25                  30

Phe Gly Asn Asn Thr Phe Asn Gln Val Val Lys Phe Gln Lys Asp Asn
        35                  40                  45

Cys Leu Asp Ser Asp Gly Ile Val Gly Lys Asn Thr Trp Ala Glu Leu
    50                  55                  60

Phe Ser Lys Tyr Ser Pro Pro Ile Pro Tyr Lys Thr Ile Pro Met Pro
65                  70                  75                  80

Thr Ala Asn Lys Ser Arg Ala Ala Ala Thr Pro Val Met Asn Ala Val
                85                  90                  95

Glu Asn Ala Thr Gly Val Arg Ser Gln Leu Leu Leu Thr Phe Ala Ser
            100                 105                 110

Ile Glu Ser Ala Phe Asp Tyr Glu Ile Lys Ala Lys Thr Ser Ser Ala
        115                 120                 125

Thr Gly Trp Phe Gln Phe Leu Thr Gly Thr Trp Lys Thr Met Ile Glu
    130                 135                 140

Asn Tyr Gly Met Lys Tyr Gly Val Leu Thr Asp Pro Thr Gly Ala Leu
145                 150                 155                 160

Arg Lys Asp Pro Arg Ile Ser Ala Leu Met Gly Ala Glu Leu Ile Lys
                165                 170                 175

Glu Asn Met Asn Ile Leu Arg Pro Val Leu Lys Arg Glu Pro Thr Asp
            180                 185                 190

Thr Asp Leu Tyr Leu Ala His Phe Phe Gly Pro Gly Ala Ala Arg Arg
        195                 200                 205

Phe Leu Thr Thr Gly Gln Asn Glu Leu Ala Ala Thr His Phe Pro Lys
    210                 215                 220

Glu Ala Gln Ala Asn Pro Ser Ile Phe Tyr Asn Lys Asp Gly Ser Pro
225                 230                 235                 240

Lys Thr Ile Gln Glu Val Tyr Asn Leu Met Asp Gly Lys Val Ala Ala
                245                 250                 255

His Arg Lys

<210>  3
<211>  22
<212>  PRT
<213>  Artificial sequence

<220>
<223>  MSI-78 peptide

<400>  3

Gly Ile Gly Lys Phe Leu Lys Lys Ala Lys Lys Phe Gly Lys Ala Phe
1               5                   10                  15

Val Lys Ile Leu Lys Lys
            20

<210>  4
<211>  30
<212>  PRT
<213>  Bungarus fasciatus

<220>
<221>  misc_feature
<222>  (1)..(30)
<223>  Cathelicidin-BF peptide

<400>  4

Lys Phe Phe Arg Lys Leu Lys Lys Ser Val Lys Lys Arg Ala Lys Glu
1               5                   10                  15

Phe Phe Lys Lys Pro Arg Val Ile Gly Val Ser Ile Pro Phe
            20                  25                  30

<210>  5
<211>  137
<212>  PRT
<213>  Escherichia virus T5

<400>  5

Met Ser Phe Lys Phe Gly Lys Asn Ser Glu Lys Gln Leu Ala Thr Val
1               5                   10                  15

Lys Pro Glu Leu Gln Lys Val Ala Arg Arg Ala Leu Glu Leu Ser Pro
            20                  25                  30

Tyr Asp Phe Thr Ile Val Gln Gly Ile Arg Thr Val Ala Gln Ser Ala
        35                  40                  45

Gln Asn Ile Ala Asn Gly Thr Ser Phe Leu Lys Asp Pro Ser Lys Ser
    50                  55                  60

Lys His Ile Thr Gly Asp Ala Ile Asp Phe Ala Pro Tyr Ile Asn Gly
65                  70                  75                  80

Lys Ile Asp Trp Asn Asp Leu Glu Ala Phe Trp Ala Val Lys Lys Ala
                85                  90                  95

Phe Glu Gln Ala Gly Lys Glu Leu Gly Ile Lys Leu Arg Phe Gly Ala
            100                 105                 110

Asp Trp Asn Ala Ser Gly Asp Tyr His Asp Glu Ile Lys Arg Gly Thr
        115                 120                 125

Tyr Asp Gly Gly His Val Glu Leu Val
    130                 135

<210>  6
<211>  269
<212>  PRT
<213>  artificial

<220>
<223>  GS linker plus KZ144 endolysin w/o methionine plus His tag

<400>  6

Gly Ser Lys Val Leu Arg Lys Gly Asp Arg Gly Asp Glu Val Cys Gln
1               5                   10                  15

Leu Gln Thr Leu Leu Asn Leu Cys Gly Tyr Asp Val Gly Lys Pro Asp
            20                  25                  30

Gly Ile Phe Gly Asn Asn Thr Phe Asn Gln Val Val Lys Phe Gln Lys
        35                  40                  45

Asp Asn Cys Leu Asp Ser Asp Gly Ile Val Gly Lys Asn Thr Trp Ala
    50                  55                  60

Glu Leu Phe Ser Lys Tyr Ser Pro Pro Ile Pro Tyr Lys Thr Ile Pro
65                  70                  75                  80

Met Pro Thr Ala Asn Lys Ser Arg Ala Ala Ala Thr Pro Val Met Asn

85                  90                  95

Ala Val Glu Asn Ala Thr Gly Val Arg Ser Gln Leu Leu Leu Thr Phe
            100                 105                 110

Ala Ser Ile Glu Ser Ala Phe Asp Tyr Glu Ile Lys Ala Lys Thr Ser
        115                 120                 125

Ser Ala Thr Gly Trp Phe Gln Phe Leu Thr Gly Thr Trp Lys Thr Met
    130                 135                 140

Ile Glu Asn Tyr Gly Met Lys Tyr Gly Val Leu Thr Asp Pro Thr Gly
145                 150                 155                 160

Ala Leu Arg Lys Asp Pro Arg Ile Ser Ala Leu Met Gly Ala Glu Leu
                165                 170                 175

Ile Lys Glu Asn Met Asn Ile Leu Arg Pro Val Leu Lys Arg Glu Pro
            180                 185                 190

Thr Asp Thr Asp Leu Tyr Leu Ala His Phe Phe Gly Pro Gly Ala Ala
        195                 200                 205

Arg Arg Phe Leu Thr Thr Gly Gln Asn Glu Leu Ala Ala Thr His Phe
    210                 215                 220

Pro Lys Glu Ala Gln Ala Asn Pro Ser Ile Phe Tyr Asn Lys Asp Gly
225                 230                 235                 240

Ser Pro Lys Thr Ile Gln Glu Val Tyr Asn Leu Met Asp Gly Lys Val
                245                 250                 255

Ala Ala His Arg Lys Leu Glu His His His His His His
            260                 265

<210>  7
<211>  136
<212>  PRT
<213>  Escherichia virus T5

<400>  7

Ser Phe Lys Phe Gly Lys Asn Ser Glu Lys Gln Leu Ala Thr Val Lys
1               5                   10                  15

Pro Glu Leu Gln Lys Val Ala Arg Arg Ala Leu Glu Leu Ser Pro Tyr
            20                  25                  30

Asp Phe Thr Ile Val Gln Gly Ile Arg Thr Val Ala Gln Ser Ala Gln

35                  40                  45

Asn Ile Ala Asn Gly Thr Ser Phe Leu Lys Asp Pro Ser Lys Ser Lys
    50                  55                  60

His Ile Thr Gly Asp Ala Ile Asp Phe Ala Pro Tyr Ile Asn Gly Lys
65                  70                  75                  80

Ile Asp Trp Asn Asp Leu Glu Ala Phe Trp Ala Val Lys Lys Ala Phe
                85                  90                  95

Glu Gln Ala Gly Lys Glu Leu Gly Ile Lys Leu Arg Phe Gly Ala Asp
            100                 105                 110

Trp Asn Ala Ser Gly Asp Tyr His Asp Glu Ile Lys Arg Gly Thr Tyr
        115                 120                 125

Asp Gly Gly His Val Glu Leu Val
    130                 135

<210>  8
<211>  260
<212>  PRT
<213>  Artificial

<220>
<223>  Consensus Sequence


<220>
<221>  MISC_FEATURE
<222>  (1)..(1)
<223>  Xaa can be any naturally occurring amino acid or absent; in
       particular it can be methionine

<220>
<221>  MISC_FEATURE
<222>  (14)..(14)
<223>  Xaa can be any naturally occurring amino acid, in particular
       serine, arginine or aspargine

<220>
<221>  MISC_FEATURE
<222>  (23)..(23)
<223>  Xaa can be any naturally occurring amino acid, in particular
       serine, arginine or aspargine

<220>
<221>  MISC_FEATURE
<222>  (50)..(50)
<223>  Xaa can be any naturally occurring amino acid, in particular
       serine, arginine or aspargine

<220>
<221>  MISC_FEATURE
<222>  (82)..(82)

<223>  Xaa can be any naturally occurring amino acid, in particular
      threonine or isoleucine

<220>
<221>  MISC_FEATURE
<222>  (122)..(122)
<223>  Xaa can be any naturally occurring amino acid, in particular
      isoleucine or methionine

<220>
<221>  MISC_FEATURE
<222>  (149)..(149)
<223>  Xaa can be any naturally occurring amino acid, in particular
      methionine or proline

<220>
<221>  MISC_FEATURE
<222>  (154)..(154)
<223>  Xaa can be any naturally occurring amino acid, in particular
      leucine or threonine

<220>
<221>  MISC_FEATURE
<222>  (160)..(160)
<223>  Xaa can be any naturally occurring amino acid, in particular
      alanine or threonine

<220>
<221>  MISC_FEATURE
<222>  (167)..(167)
<223>  Xaa can be any naturally occurring amino acid, in particular
      isoleucine or leucine

<220>
<221>  MISC_FEATURE
<222>  (179)..(179)
<223>  Xaa can be any naturally occurring amino acid, in particular
      asparagine or phenylalanine

<220>
<221>  MISC_FEATURE
<222>  (180)..(180)
<223>  Xaa can be any naturally occurring amino acid, in particular
      methionine or glutamic acid

<220>
<221>  MISC_FEATURE
<222>  (186)..(186)
<223>  Xaa can be any naturally occurring amino acid, in particular
      valine or tyrosine

<220>
<221>  MISC_FEATURE
<222>  (206)..(206)
<223>  Xaa can be any naturally occurring amino acid, in particular
      alanine, asparagine or valine

<220>
<221>  MISC_FEATURE
<222>  (212)..(212)
<223>  Xaa can be any naturally occurring amino acid, in particular
      threonine or asparagine

<220>
<221>  MISC_FEATURE
<222>  (224)..(224)
<223>  Xaa can be any naturally occurring amino acid, in particular
       proline or glutamine

<220>
<221>  MISC_FEATURE
<222>  (230)..(230)
<223>  Xaa can be any naturally occurring amino acid, in particular
       asparagine or tyrosine

<220>
<221>  MISC_FEATURE
<222>  (232)..(232)
<223>  Xaa can be any naturally occurring amino acid, in particular
       serine or threonine

<400>  8

Xaa Lys Val Leu Arg Lys Gly Asp Arg Gly Asp Glu Val Xaa Gln Leu
1               5                   10                  15

Gln Thr Leu Leu Asn Leu Xaa Gly Tyr Asp Val Gly Lys Pro Asp Gly
            20                  25                  30

Ile Phe Gly Asn Asn Thr Phe Asn Gln Val Val Lys Phe Gln Lys Asp
        35                  40                  45

Asn Xaa Leu Asp Ser Asp Gly Ile Val Gly Lys Asn Thr Trp Ala Glu
    50                  55                  60

Leu Phe Ser Lys Tyr Ser Pro Pro Ile Pro Tyr Lys Thr Ile Pro Met
65                  70                  75                  80

Pro Xaa Ala Asn Lys Ser Arg Ala Ala Ala Thr Pro Val Met Asn Ala
                85                  90                  95

Val Glu Asn Ala Thr Gly Val Arg Ser Gln Leu Leu Leu Thr Phe Ala
            100                 105                 110

Ser Ile Glu Ser Ala Phe Asp Tyr Glu Xaa Lys Ala Lys Thr Ser Ser
        115                 120                 125

Ala Thr Gly Trp Phe Gln Phe Leu Thr Gly Thr Trp Lys Thr Met Ile
    130                 135                 140

Glu Asn Tyr Gly Xaa Lys Tyr Gly Val Xaa Thr Asp Pro Thr Gly Xaa
145                 150                 155                 160

Leu Arg Lys Asp Pro Arg Xaa Ser Ala Leu Met Gly Ala Glu Leu Ile
                165                 170                 175

Lys Glu Xaa Xaa Asn Ile Leu Arg Pro Xaa Leu Lys Arg Glu Pro Thr
            180                 185                 190

Asp Thr Asp Leu Tyr Leu Ala His Phe Phe Gly Pro Gly Xaa Ala Arg
        195                 200                 205

Arg Phe Leu Xaa Thr Gly Gln Asn Glu Leu Ala Ala Thr His Phe Xaa
    210                 215                 220

Lys Glu Ala Gln Ala Xaa Pro Xaa Ile Phe Tyr Asn Lys Asp Gly Ser
225                 230                 235                 240

Pro Lys Thr Ile Gln Glu Val Tyr Asn Leu Met Asp Gly Lys Val Ala
                245                 250                 255

Ala His Arg Lys
            260

<210>  9
<211>  259
<212>  PRT
<213>  artificial sequence

<220>
<223>  mutated KZ144 with C14S and C50S, without N-terminal methionine

<400>  9

Lys Val Leu Arg Lys Gly Asp Arg Gly Asp Glu Val Ser Gln Leu Gln
1               5                   10                  15

Thr Leu Leu Asn Leu Cys Gly Tyr Asp Val Gly Lys Pro Asp Gly Ile
            20                  25                  30

Phe Gly Asn Asn Thr Phe Asn Gln Val Val Lys Phe Gln Lys Asp Asn
        35                  40                  45

Ser Leu Asp Ser Asp Gly Ile Val Gly Lys Asn Thr Trp Ala Glu Leu
    50                  55                  60

Phe Ser Lys Tyr Ser Pro Pro Ile Pro Tyr Lys Thr Ile Pro Met Pro
65                  70                  75                  80

Thr Ala Asn Lys Ser Arg Ala Ala Ala Thr Pro Val Met Asn Ala Val
                85                  90                  95

Glu Asn Ala Thr Gly Val Arg Ser Gln Leu Leu Leu Thr Phe Ala Ser
            100                 105                 110

Ile Glu Ser Ala Phe Asp Tyr Glu Ile Lys Ala Lys Thr Ser Ser Ala
        115                 120                 125

Thr Gly Trp Phe Gln Phe Leu Thr Gly Thr Trp Lys Thr Met Ile Glu
    130                 135                 140

Asn Tyr Gly Met Lys Tyr Gly Val Leu Thr Asp Pro Thr Gly Ala Leu
145                 150                 155                 160

Arg Lys Asp Pro Arg Ile Ser Ala Leu Met Gly Ala Glu Leu Ile Lys
                165                 170                 175

Glu Asn Met Asn Ile Leu Arg Pro Val Leu Lys Arg Glu Pro Thr Asp
            180                 185                 190

Thr Asp Leu Tyr Leu Ala His Phe Phe Gly Pro Gly Ala Ala Arg Arg
        195                 200                 205

Phe Leu Thr Thr Gly Gln Asn Glu Leu Ala Ala Thr His Phe Pro Lys
    210                 215                 220

Glu Ala Gln Ala Asn Pro Ser Ile Phe Tyr Asn Lys Asp Gly Ser Pro
225                 230                 235                 240

Lys Thr Ile Gln Glu Val Tyr Asn Leu Met Asp Gly Lys Val Ala Ala
                245                 250                 255

His Arg Lys

<210>  10
<211>  259
<212>  PRT
<213>  artificial sequence

<220>
<223>  mutated KZ144 with T82I, A206V and S232, without N-terminal
       methionine

<400>  10

Lys Val Leu Arg Lys Gly Asp Arg Gly Asp Glu Val Cys Gln Leu Gln
1               5                   10                  15

Thr Leu Leu Asn Leu Cys Gly Tyr Asp Val Gly Lys Pro Asp Gly Ile
            20                  25                  30

Phe Gly Asn Asn Thr Phe Asn Gln Val Val Lys Phe Gln Lys Asp Asn
        35                  40                  45

Cys Leu Asp Ser Asp Gly Ile Val Gly Lys Asn Thr Trp Ala Glu Leu
    50                  55                  60

Phe Ser Lys Tyr Ser Pro Pro Ile Pro Tyr Lys Thr Ile Pro Met Pro
65                  70                  75                  80

Ile Ala Asn Lys Ser Arg Ala Ala Ala Thr Pro Val Met Asn Ala Val
                85                  90                  95

Glu Asn Ala Thr Gly Val Arg Ser Gln Leu Leu Leu Thr Phe Ala Ser
            100                 105                 110

Ile Glu Ser Ala Phe Asp Tyr Glu Ile Lys Ala Lys Thr Ser Ser Ala
        115                 120                 125

Thr Gly Trp Phe Gln Phe Leu Thr Gly Thr Trp Lys Thr Met Ile Glu
    130                 135                 140

Asn Tyr Gly Met Lys Tyr Gly Val Leu Thr Asp Pro Thr Gly Ala Leu
145                 150                 155                 160

Arg Lys Asp Pro Arg Ile Ser Ala Leu Met Gly Ala Glu Leu Ile Lys
                165                 170                 175

Glu Asn Met Asn Ile Leu Arg Pro Val Leu Lys Arg Glu Pro Thr Asp
            180                 185                 190

Thr Asp Leu Tyr Leu Ala His Phe Phe Gly Pro Gly Val Ala Arg Arg
        195                 200                 205

Phe Leu Thr Thr Gly Gln Asn Glu Leu Ala Ala Thr His Phe Pro Lys
    210                 215                 220

Glu Ala Gln Ala Asn Pro Thr Ile Phe Tyr Asn Lys Asp Gly Ser Pro
225                 230                 235                 240

Lys Thr Ile Gln Glu Val Tyr Asn Leu Met Asp Gly Lys Val Ala Ala
                245                 250                 255

His Arg Lys

<210>  11
<211>  259
<212>  PRT
<213>  artificial sequence

<220>
<223>  mutated KZ144 with T82I, A206V, S232T, I122M and A160T, without

N-terminal methionine

<400>  11

Lys Val Leu Arg Lys Gly Asp Arg Gly Asp Glu Val Cys Gln Leu Gln
1               5                   10                  15

Thr Leu Leu Asn Leu Cys Gly Tyr Asp Val Gly Lys Pro Asp Gly Ile
            20                  25                  30

Phe Gly Asn Asn Thr Phe Asn Gln Val Val Lys Phe Gln Lys Asp Asn
        35                  40                  45

Cys Leu Asp Ser Asp Gly Ile Val Gly Lys Asn Thr Trp Ala Glu Leu
    50                  55                  60

Phe Ser Lys Tyr Ser Pro Pro Ile Pro Tyr Lys Thr Ile Pro Met Pro
65                  70                  75                  80

Ile Ala Asn Lys Ser Arg Ala Ala Ala Thr Pro Val Met Asn Ala Val
                85                  90                  95

Glu Asn Ala Thr Gly Val Arg Ser Gln Leu Leu Leu Thr Phe Ala Ser
            100                 105                 110

Ile Glu Ser Ala Phe Asp Tyr Glu Met Lys Ala Lys Thr Ser Ser Ala
        115                 120                 125

Thr Gly Trp Phe Gln Phe Leu Thr Gly Thr Trp Lys Thr Met Ile Glu
    130                 135                 140

Asn Tyr Gly Met Lys Tyr Gly Val Leu Thr Asp Pro Thr Gly Thr Leu
145                 150                 155                 160

Arg Lys Asp Pro Arg Ile Ser Ala Leu Met Gly Ala Glu Leu Ile Lys
                165                 170                 175

Glu Asn Met Asn Ile Leu Arg Pro Val Leu Lys Arg Glu Pro Thr Asp
            180                 185                 190

Thr Asp Leu Tyr Leu Ala His Phe Phe Gly Pro Gly Val Ala Arg Arg
        195                 200                 205

Phe Leu Thr Thr Gly Gln Asn Glu Leu Ala Ala Thr His Phe Pro Lys
    210                 215                 220

Glu Ala Gln Ala Asn Pro Thr Ile Phe Tyr Asn Lys Asp Gly Ser Pro
225                 230                 235                 240

Lys Thr Ile Gln Glu Val Tyr Asn Leu Met Asp Gly Lys Val Ala Ala
                245                 250                 255

His Arg Lys

<210>  12
<211>  259
<212>  PRT
<213>  artificial sequence

<220>
<223>  mutated KZ144 with C14S, C50S, I122M and A160T, without
       N-terminal methionine

<400>  12

Lys Val Leu Arg Lys Gly Asp Arg Gly Asp Glu Val Ser Gln Leu Gln
1               5                   10                  15

Thr Leu Leu Asn Leu Cys Gly Tyr Asp Val Gly Lys Pro Asp Gly Ile
            20                  25                  30

Phe Gly Asn Asn Thr Phe Asn Gln Val Val Lys Phe Gln Lys Asp Asn
        35                  40                  45

Ser Leu Asp Ser Asp Gly Ile Val Gly Lys Asn Thr Trp Ala Glu Leu
    50                  55                  60

Phe Ser Lys Tyr Ser Pro Pro Ile Pro Tyr Lys Thr Ile Pro Met Pro
65                  70                  75                  80

Thr Ala Asn Lys Ser Arg Ala Ala Ala Thr Pro Val Met Asn Ala Val
                85                  90                  95

Glu Asn Ala Thr Gly Val Arg Ser Gln Leu Leu Leu Thr Phe Ala Ser
            100                 105                 110

Ile Glu Ser Ala Phe Asp Tyr Glu Met Lys Ala Lys Thr Ser Ser Ala
        115                 120                 125

Thr Gly Trp Phe Gln Phe Leu Thr Gly Thr Trp Lys Thr Met Ile Glu
    130                 135                 140

Asn Tyr Gly Met Lys Tyr Gly Val Leu Thr Asp Pro Thr Gly Thr Leu
145                 150                 155                 160

Arg Lys Asp Pro Arg Ile Ser Ala Leu Met Gly Ala Glu Leu Ile Lys
                165                 170                 175

Glu Asn Met Asn Ile Leu Arg Pro Val Leu Lys Arg Glu Pro Thr Asp
            180                 185                 190

Thr Asp Leu Tyr Leu Ala His Phe Phe Gly Pro Gly Ala Ala Arg Arg
        195                 200                 205

Phe Leu Thr Thr Gly Gln Asn Glu Leu Ala Ala Thr His Phe Pro Lys
    210                 215                 220

Glu Ala Gln Ala Asn Pro Ser Ile Phe Tyr Asn Lys Asp Gly Ser Pro
225                 230                 235                 240

Lys Thr Ile Gln Glu Val Tyr Asn Leu Met Asp Gly Lys Val Ala Ala
                245                 250                 255

His Arg Lys

<210>  13
<211>  259
<212>  PRT
<213>  artificial sequence

<220>
<223>  mutated KZ144 with C14S, C23S and C50S, without N-terminal
       methionine

<400>  13

Lys Val Leu Arg Lys Gly Asp Arg Gly Asp Glu Val Ser Gln Leu Gln
1               5                   10                  15

Thr Leu Leu Asn Leu Ser Gly Tyr Asp Val Gly Lys Pro Asp Gly Ile
            20                  25                  30

Phe Gly Asn Asn Thr Phe Asn Gln Val Val Lys Phe Gln Lys Asp Asn
        35                  40                  45

Ser Leu Asp Ser Asp Gly Ile Val Gly Lys Asn Thr Trp Ala Glu Leu
    50                  55                  60

Phe Ser Lys Tyr Ser Pro Pro Ile Pro Tyr Lys Thr Ile Pro Met Pro
65                  70                  75                  80

Thr Ala Asn Lys Ser Arg Ala Ala Ala Thr Pro Val Met Asn Ala Val
                85                  90                  95

Glu Asn Ala Thr Gly Val Arg Ser Gln Leu Leu Leu Thr Phe Ala Ser
            100                 105                 110

Ile Glu Ser Ala Phe Asp Tyr Glu Ile Lys Ala Lys Thr Ser Ser Ala
        115                 120                 125

Thr Gly Trp Phe Gln Phe Leu Thr Gly Thr Trp Lys Thr Met Ile Glu
    130                 135                 140

Asn Tyr Gly Met Lys Tyr Gly Val Leu Thr Asp Pro Thr Gly Ala Leu
145                 150                 155                 160

Arg Lys Asp Pro Arg Ile Ser Ala Leu Met Gly Ala Glu Leu Ile Lys
                165                 170                 175

Glu Asn Met Asn Ile Leu Arg Pro Val Leu Lys Arg Glu Pro Thr Asp
            180                 185                 190

Thr Asp Leu Tyr Leu Ala His Phe Phe Gly Pro Gly Ala Ala Arg Arg
        195                 200                 205

Phe Leu Thr Thr Gly Gln Asn Glu Leu Ala Ala Thr His Phe Pro Lys
    210                 215                 220

Glu Ala Gln Ala Asn Pro Ser Ile Phe Tyr Asn Lys Asp Gly Ser Pro
225                 230                 235                 240

Lys Thr Ile Gln Glu Val Tyr Asn Leu Met Asp Gly Lys Val Ala Ala
                245                 250                 255

His Arg Lys

<210>  14
<211>  259
<212>  PRT
<213>  artificial sequence

<220>
<223>  mutated KZ144 with T82I, A206V, S232T, I122M, A160T, C14S and
       C50S, without N-terminal methionine

<400>  14

Lys Val Leu Arg Lys Gly Asp Arg Gly Asp Glu Val Ser Gln Leu Gln
1               5                   10                  15

Thr Leu Leu Asn Leu Cys Gly Tyr Asp Val Gly Lys Pro Asp Gly Ile
            20                  25                  30

Phe Gly Asn Asn Thr Phe Asn Gln Val Val Lys Phe Gln Lys Asp Asn
        35                  40                  45

Ser Leu Asp Ser Asp Gly Ile Val Gly Lys Asn Thr Trp Ala Glu Leu
    50                  55                  60

Phe Ser Lys Tyr Ser Pro Pro Ile Pro Tyr Lys Thr Ile Pro Met Pro
65                  70                  75                  80

Ile Ala Asn Lys Ser Arg Ala Ala Ala Thr Pro Val Met Asn Ala Val
                85                  90                  95

Glu Asn Ala Thr Gly Val Arg Ser Gln Leu Leu Leu Thr Phe Ala Ser
            100                 105                 110

Ile Glu Ser Ala Phe Asp Tyr Glu Met Lys Ala Lys Thr Ser Ser Ala
        115                 120                 125

Thr Gly Trp Phe Gln Phe Leu Thr Gly Thr Trp Lys Thr Met Ile Glu
    130                 135                 140

Asn Tyr Gly Met Lys Tyr Gly Val Leu Thr Asp Pro Thr Gly Thr Leu
145                 150                 155                 160

Arg Lys Asp Pro Arg Ile Ser Ala Leu Met Gly Ala Glu Leu Ile Lys
                165                 170                 175

Glu Asn Met Asn Ile Leu Arg Pro Val Leu Lys Arg Glu Pro Thr Asp
            180                 185                 190

Thr Asp Leu Tyr Leu Ala His Phe Phe Gly Pro Gly Val Ala Arg Arg
        195                 200                 205

Phe Leu Thr Thr Gly Gln Asn Glu Leu Ala Ala Thr His Phe Pro Lys
    210                 215                 220

Glu Ala Gln Ala Asn Pro Thr Ile Phe Tyr Asn Lys Asp Gly Ser Pro
225                 230                 235                 240

Lys Thr Ile Gln Glu Val Tyr Asn Leu Met Asp Gly Lys Val Ala Ala
                245                 250                 255

His Arg Lys

<210>  15
<211>  259
<212>  PRT
<213>  artificial sequence

<220>

<223>  mutated KZ144 with T82I, A206N, S232T, I122M, A160T, C14S and
      C50S, without N-terminal methionine

<400>  15

Lys Val Leu Arg Lys Gly Asp Arg Gly Asp Glu Val Ser Gln Leu Gln
1               5                   10                  15

Thr Leu Leu Asn Leu Cys Gly Tyr Asp Val Gly Lys Pro Asp Gly Ile
            20                  25                  30

Phe Gly Asn Asn Thr Phe Asn Gln Val Val Lys Phe Gln Lys Asp Asn
        35                  40                  45

Ser Leu Asp Ser Asp Gly Ile Val Gly Lys Asn Thr Trp Ala Glu Leu
    50                  55                  60

Phe Ser Lys Tyr Ser Pro Pro Ile Pro Tyr Lys Thr Ile Pro Met Pro
65                  70                  75                  80

Ile Ala Asn Lys Ser Arg Ala Ala Ala Thr Pro Val Met Asn Ala Val
                85                  90                  95

Glu Asn Ala Thr Gly Val Arg Ser Gln Leu Leu Leu Thr Phe Ala Ser
            100                 105                 110

Ile Glu Ser Ala Phe Asp Tyr Glu Met Lys Ala Lys Thr Ser Ser Ala
        115                 120                 125

Thr Gly Trp Phe Gln Phe Leu Thr Gly Thr Trp Lys Thr Met Ile Glu
    130                 135                 140

Asn Tyr Gly Met Lys Tyr Gly Val Leu Thr Asp Pro Thr Gly Thr Leu
145                 150                 155                 160

Arg Lys Asp Pro Arg Ile Ser Ala Leu Met Gly Ala Glu Leu Ile Lys
                165                 170                 175

Glu Asn Met Asn Ile Leu Arg Pro Val Leu Lys Arg Glu Pro Thr Asp
            180                 185                 190

Thr Asp Leu Tyr Leu Ala His Phe Phe Gly Pro Gly Asn Ala Arg Arg
        195                 200                 205

Phe Leu Thr Thr Gly Gln Asn Glu Leu Ala Ala Thr His Phe Pro Lys
    210                 215                 220

Glu Ala Gln Ala Asn Pro Thr Ile Phe Tyr Asn Lys Asp Gly Ser Pro
225                 230                 235                 240

Lys Thr Ile Gln Glu Val Tyr Asn Leu Met Asp Gly Lys Val Ala Ala
                245                 250                 255

His Arg Lys

<210>  16
<211>  259
<212>  PRT
<213>  Artificial Sequence

<220>
<223>  mutated KZ144 with N230Y, T82I, A206V, S232T, I122M, A160T, C14S
       and C50S, without N-terminal methionine

<400>  16

Lys Val Leu Arg Lys Gly Asp Arg Gly Asp Glu Val Ser Gln Leu Gln
1               5                   10                  15

Thr Leu Leu Asn Leu Cys Gly Tyr Asp Val Gly Lys Pro Asp Gly Ile
            20                  25                  30

Phe Gly Asn Asn Thr Phe Asn Gln Val Val Lys Phe Gln Lys Asp Asn
        35                  40                  45

Ser Leu Asp Ser Asp Gly Ile Val Gly Lys Asn Thr Trp Ala Glu Leu
    50                  55                  60

Phe Ser Lys Tyr Ser Pro Pro Ile Pro Tyr Lys Thr Ile Pro Met Pro
65                  70                  75                  80

Ile Ala Asn Lys Ser Arg Ala Ala Ala Thr Pro Val Met Asn Ala Val
                85                  90                  95

Glu Asn Ala Thr Gly Val Arg Ser Gln Leu Leu Leu Thr Phe Ala Ser
            100                 105                 110

Ile Glu Ser Ala Phe Asp Tyr Glu Met Lys Ala Lys Thr Ser Ser Ala
        115                 120                 125

Thr Gly Trp Phe Gln Phe Leu Thr Gly Thr Trp Lys Thr Met Ile Glu
    130                 135                 140

Asn Tyr Gly Met Lys Tyr Gly Val Leu Thr Asp Pro Thr Gly Thr Leu
145                 150                 155                 160

Arg Lys Asp Pro Arg Ile Ser Ala Leu Met Gly Ala Glu Leu Ile Lys
                165                 170                 175

Glu Asn Met Asn Ile Leu Arg Pro Val Leu Lys Arg Glu Pro Thr Asp
            180                 185                 190

Thr Asp Leu Tyr Leu Ala His Phe Phe Gly Pro Gly Val Ala Arg Arg
        195                 200                 205

Phe Leu Thr Thr Gly Gln Asn Glu Leu Ala Ala Thr His Phe Pro Lys
    210                 215                 220

Glu Ala Gln Ala Tyr Pro Thr Ile Phe Tyr Asn Lys Asp Gly Ser Pro
225                 230                 235                 240

Lys Thr Ile Gln Glu Val Tyr Asn Leu Met Asp Gly Lys Val Ala Ala
                245                 250                 255

His Arg Lys

<210>  17
<211>  259
<212>  PRT
<213>  Artificial Sequence

<220>
<223>  mutated KZ144 with M180E, T82I, A206V, S232T, I122M, A160T, C14S
       and C50S, without N-terminal methionine

<400>  17

Lys Val Leu Arg Lys Gly Asp Arg Gly Asp Glu Val Ser Gln Leu Gln
1               5                   10                  15

Thr Leu Leu Asn Leu Cys Gly Tyr Asp Val Gly Lys Pro Asp Gly Ile
            20                  25                  30

Phe Gly Asn Asn Thr Phe Asn Gln Val Val Lys Phe Gln Lys Asp Asn
        35                  40                  45

Ser Leu Asp Ser Asp Gly Ile Val Gly Lys Asn Thr Trp Ala Glu Leu
    50                  55                  60

Phe Ser Lys Tyr Ser Pro Pro Ile Pro Tyr Lys Thr Ile Pro Met Pro
65                  70                  75                  80

Ile Ala Asn Lys Ser Arg Ala Ala Ala Thr Pro Val Met Asn Ala Val
                85                  90                  95

Glu Asn Ala Thr Gly Val Arg Ser Gln Leu Leu Leu Thr Phe Ala Ser
            100                 105                 110

Ile Glu Ser Ala Phe Asp Tyr Glu Met Lys Ala Lys Thr Ser Ser Ala
        115                 120                 125

Thr Gly Trp Phe Gln Phe Leu Thr Gly Thr Trp Lys Thr Met Ile Glu
    130                 135                 140

Asn Tyr Gly Met Lys Tyr Gly Val Leu Thr Asp Pro Thr Gly Thr Leu
145                 150                 155                 160

Arg Lys Asp Pro Arg Ile Ser Ala Leu Met Gly Ala Glu Leu Ile Lys
                165                 170                 175

Glu Asn Glu Asn Ile Leu Arg Pro Val Leu Lys Arg Glu Pro Thr Asp
            180                 185                 190

Thr Asp Leu Tyr Leu Ala His Phe Phe Gly Pro Gly Val Ala Arg Arg
        195                 200                 205

Phe Leu Thr Thr Gly Gln Asn Glu Leu Ala Ala Thr His Phe Pro Lys
    210                 215                 220

Glu Ala Gln Ala Asn Pro Thr Ile Phe Tyr Asn Lys Asp Gly Ser Pro
225                 230                 235                 240

Lys Thr Ile Gln Glu Val Tyr Asn Leu Met Asp Gly Lys Val Ala Ala
                245                 250                 255

His Arg Lys

<210>  18
<211>  259
<212>  PRT
<213>  Artificial Sequence

<220>
<223>  mutated KZ144 with M149P, T82I, A206V, S232T, I122M, A160T, C14S
       and C50S, without N-terminal methionine

<400>  18

Lys Val Leu Arg Lys Gly Asp Arg Gly Asp Glu Val Ser Gln Leu Gln
1               5                   10                  15

Thr Leu Leu Asn Leu Cys Gly Tyr Asp Val Gly Lys Pro Asp Gly Ile
            20                  25                  30

Phe Gly Asn Asn Thr Phe Asn Gln Val Val Lys Phe Gln Lys Asp Asn
        35                  40                  45

Ser Leu Asp Ser Asp Gly Ile Val Gly Lys Asn Thr Trp Ala Glu Leu
    50                  55                  60

Phe Ser Lys Tyr Ser Pro Pro Ile Pro Tyr Lys Thr Ile Pro Met Pro
65                  70                  75                  80

Ile Ala Asn Lys Ser Arg Ala Ala Ala Thr Pro Val Met Asn Ala Val
                85                  90                  95

Glu Asn Ala Thr Gly Val Arg Ser Gln Leu Leu Leu Thr Phe Ala Ser
            100                 105                 110

Ile Glu Ser Ala Phe Asp Tyr Glu Met Lys Ala Lys Thr Ser Ser Ala
        115                 120                 125

Thr Gly Trp Phe Gln Phe Leu Thr Gly Thr Trp Lys Thr Met Ile Glu
    130                 135                 140

Asn Tyr Gly Pro Lys Tyr Gly Val Leu Thr Asp Pro Thr Gly Thr Leu
145                 150                 155                 160

Arg Lys Asp Pro Arg Ile Ser Ala Leu Met Gly Ala Glu Leu Ile Lys
                165                 170                 175

Glu Asn Met Asn Ile Leu Arg Pro Val Leu Lys Arg Glu Pro Thr Asp
            180                 185                 190

Thr Asp Leu Tyr Leu Ala His Phe Phe Gly Pro Gly Val Ala Arg Arg
        195                 200                 205

Phe Leu Thr Thr Gly Gln Asn Glu Leu Ala Ala Thr His Phe Pro Lys
    210                 215                 220

Glu Ala Gln Ala Asn Pro Thr Ile Phe Tyr Asn Lys Asp Gly Ser Pro
225                 230                 235                 240

Lys Thr Ile Gln Glu Val Tyr Asn Leu Met Asp Gly Lys Val Ala Ala
                245                 250                 255

His Arg Lys

<210>  19
<211>  259
<212>  PRT
<213>  Artificial Sequence

<220>
<223>  mutated KZ144 with V186Y, T82I, A206V, S232T, I122M, A160T, C14S
       and C50S, without N-terminal methionine

<400>  19

Lys Val Leu Arg Lys Gly Asp Arg Gly Asp Glu Val Ser Gln Leu Gln
1               5                   10                  15

Thr Leu Leu Asn Leu Cys Gly Tyr Asp Val Gly Lys Pro Asp Gly Ile
            20                  25                  30

Phe Gly Asn Asn Thr Phe Asn Gln Val Val Lys Phe Gln Lys Asp Asn
        35                  40                  45

Ser Leu Asp Ser Asp Gly Ile Val Gly Lys Asn Thr Trp Ala Glu Leu
    50                  55                  60

Phe Ser Lys Tyr Ser Pro Pro Ile Pro Tyr Lys Thr Ile Pro Met Pro
65                  70                  75                  80

Ile Ala Asn Lys Ser Arg Ala Ala Ala Thr Pro Val Met Asn Ala Val
                85                  90                  95

Glu Asn Ala Thr Gly Val Arg Ser Gln Leu Leu Leu Thr Phe Ala Ser
            100                 105                 110

Ile Glu Ser Ala Phe Asp Tyr Glu Met Lys Ala Lys Thr Ser Ser Ala
        115                 120                 125

Thr Gly Trp Phe Gln Phe Leu Thr Gly Thr Trp Lys Thr Met Ile Glu
    130                 135                 140

Asn Tyr Gly Met Lys Tyr Gly Val Leu Thr Asp Pro Thr Gly Thr Leu
145                 150                 155                 160

Arg Lys Asp Pro Arg Ile Ser Ala Leu Met Gly Ala Glu Leu Ile Lys
                165                 170                 175

Glu Asn Met Asn Ile Leu Arg Pro Tyr Leu Lys Arg Glu Pro Thr Asp
            180                 185                 190

Thr Asp Leu Tyr Leu Ala His Phe Phe Gly Pro Gly Val Ala Arg Arg
        195                 200                 205

Phe Leu Thr Thr Gly Gln Asn Glu Leu Ala Ala Thr His Phe Pro Lys
    210                 215                 220

Glu Ala Gln Ala Asn Pro Thr Ile Phe Tyr Asn Lys Asp Gly Ser Pro

225                 230                 235                 240

Lys Thr Ile Gln Glu Val Tyr Asn Leu Met Asp Gly Lys Val Ala Ala
                245                 250                 255

His Arg Lys

<210>  20
<211>  259
<212>  PRT
<213>  Artificial Sequence

<220>
<223>  mutated KZ144 with T82I, A206V, S232T, I122M, A160T, C14R and
       C50S, without N-terminal methionine

<400>  20

Lys Val Leu Arg Lys Gly Asp Arg Gly Asp Glu Val Arg Gln Leu Gln
1               5                   10                  15

Thr Leu Leu Asn Leu Cys Gly Tyr Asp Val Gly Lys Pro Asp Gly Ile
            20                  25                  30

Phe Gly Asn Asn Thr Phe Asn Gln Val Val Lys Phe Gln Lys Asp Asn
        35                  40                  45

Ser Leu Asp Ser Asp Gly Ile Val Gly Lys Asn Thr Trp Ala Glu Leu
    50                  55                  60

Phe Ser Lys Tyr Ser Pro Pro Ile Pro Tyr Lys Thr Ile Pro Met Pro
65                  70                  75                  80

Ile Ala Asn Lys Ser Arg Ala Ala Ala Thr Pro Val Met Asn Ala Val
                85                  90                  95

Glu Asn Ala Thr Gly Val Arg Ser Gln Leu Leu Leu Thr Phe Ala Ser
            100                 105                 110

Ile Glu Ser Ala Phe Asp Tyr Glu Met Lys Ala Lys Thr Ser Ser Ala
        115                 120                 125

Thr Gly Trp Phe Gln Phe Leu Thr Gly Thr Trp Lys Thr Met Ile Glu
    130                 135                 140

Asn Tyr Gly Met Lys Tyr Gly Val Leu Thr Asp Pro Thr Gly Thr Leu
145                 150                 155                 160

Arg Lys Asp Pro Arg Ile Ser Ala Leu Met Gly Ala Glu Leu Ile Lys

165                 170                 175

Glu Asn Met Asn Ile Leu Arg Pro Val Leu Lys Arg Glu Pro Thr Asp
            180                 185                 190

Thr Asp Leu Tyr Leu Ala His Phe Phe Gly Pro Gly Val Ala Arg Arg
        195                 200                 205

Phe Leu Thr Thr Gly Gln Asn Glu Leu Ala Ala Thr His Phe Pro Lys
    210                 215                 220

Glu Ala Gln Ala Asn Pro Thr Ile Phe Tyr Asn Lys Asp Gly Ser Pro
225                 230                 235                 240

Lys Thr Ile Gln Glu Val Tyr Asn Leu Met Asp Gly Lys Val Ala Ala
                245                 250                 255

His Arg Lys

<210>  21
<211>  259
<212>  PRT
<213>  Artificial Sequence

<220>
<223>  mutated KZ144 with T82I, A206V, S232T, I122M, C14S and C50S,
       without N-terminal methionine

<400>  21

Lys Val Leu Arg Lys Gly Asp Arg Gly Asp Glu Val Cys Gln Leu Gln
1               5                   10                  15

Thr Leu Leu Asn Leu Cys Gly Tyr Asp Val Gly Lys Pro Asp Gly Ile
            20                  25                  30

Phe Gly Asn Asn Thr Phe Asn Gln Val Val Lys Phe Gln Lys Asp Asn
        35                  40                  45

Ser Leu Asp Ser Asp Gly Ile Val Gly Lys Asn Thr Trp Ala Glu Leu
    50                  55                  60

Phe Ser Lys Tyr Ser Pro Pro Ile Pro Tyr Lys Thr Ile Pro Met Pro
65                  70                  75                  80

Ile Ala Asn Lys Ser Arg Ala Ala Ala Thr Pro Val Met Asn Ala Val
                85                  90                  95

Glu Asn Ala Thr Gly Val Arg Ser Gln Leu Leu Leu Thr Phe Ala Ser

            100                 105                 110

Ile Glu Ser Ala Phe Asp Tyr Glu Met Lys Ala Lys Thr Ser Ser Ala
        115                 120                 125

Thr Gly Trp Phe Gln Phe Leu Thr Gly Thr Trp Lys Thr Met Ile Glu
    130                 135                 140

Asn Tyr Gly Met Lys Tyr Gly Val Leu Thr Asp Pro Thr Gly Ala Leu
145                 150                 155                 160

Arg Lys Asp Pro Arg Ile Ser Ala Leu Met Gly Ala Glu Leu Ile Lys
                165                 170                 175

Glu Asn Met Asn Ile Leu Arg Pro Val Leu Lys Arg Glu Pro Thr Asp
            180                 185                 190

Thr Asp Leu Tyr Leu Ala His Phe Phe Gly Pro Gly Val Ala Arg Arg
        195                 200                 205

Phe Leu Thr Thr Gly Gln Asn Glu Leu Ala Ala Thr His Phe Pro Lys
    210                 215                 220

Glu Ala Gln Ala Asn Pro Thr Ile Phe Tyr Asn Lys Asp Gly Ser Pro
225                 230                 235                 240

Lys Thr Ile Gln Glu Val Tyr Asn Leu Met Asp Gly Lys Val Ala Ala
                245                 250                 255

His Arg Lys

<210> 22
<211> 259
<212> PRT
<213> Artificial Sequence

<220>
<223> mutated KZ144 with T82I, A206V, S232T, I122M, A160T, C14S and
C50N, without N-terminal methionine

<400> 22

Lys Val Leu Arg Lys Gly Asp Arg Gly Asp Glu Val Ser Gln Leu Gln
1               5                   10                  15

Thr Leu Leu Asn Leu Cys Gly Tyr Asp Val Gly Lys Pro Asp Gly Ile
            20                  25                  30

Phe Gly Asn Asn Thr Phe Asn Gln Val Val Lys Phe Gln Lys Asp Asn

35                  40                  45

Asn Leu Asp Ser Asp Gly Ile Val Gly Lys Asn Thr Trp Ala Glu Leu
    50                  55                  60

Phe Ser Lys Tyr Ser Pro Pro Ile Pro Tyr Lys Thr Ile Pro Met Pro
65                  70                  75                  80

Ile Ala Asn Lys Ser Arg Ala Ala Ala Thr Pro Val Met Asn Ala Val
                85                  90                  95

Glu Asn Ala Thr Gly Val Arg Ser Gln Leu Leu Leu Thr Phe Ala Ser
            100                 105                 110

Ile Glu Ser Ala Phe Asp Tyr Glu Met Lys Ala Lys Thr Ser Ser Ala
        115                 120                 125

Thr Gly Trp Phe Gln Phe Leu Thr Gly Thr Trp Lys Thr Met Ile Glu
    130                 135                 140

Asn Tyr Gly Met Lys Tyr Gly Val Leu Thr Asp Pro Thr Gly Thr Leu
145                 150                 155                 160

Arg Lys Asp Pro Arg Ile Ser Ala Leu Met Gly Ala Glu Leu Ile Lys
                165                 170                 175

Glu Asn Met Asn Ile Leu Arg Pro Val Leu Lys Arg Glu Pro Thr Asp
            180                 185                 190

Thr Asp Leu Tyr Leu Ala His Phe Phe Gly Pro Gly Val Ala Arg Arg
        195                 200                 205

Phe Leu Thr Thr Gly Gln Asn Glu Leu Ala Ala Thr His Phe Pro Lys
    210                 215                 220

Glu Ala Gln Ala Asn Pro Thr Ile Phe Tyr Asn Lys Asp Gly Ser Pro
225                 230                 235                 240

Lys Thr Ile Gln Glu Val Tyr Asn Leu Met Asp Gly Lys Val Ala Ala
                245                 250                 255

His Arg Lys

<210>  23
<211>  259
<212>  PRT
<213>  Artificial Sequence

<220>
<223>  mutated KZ144 with C14R, C50S, T82I, I122M, M149P, A206V and
       S232T; without N-terminal methionine

<400>  23

Lys Val Leu Arg Lys Gly Asp Arg Gly Asp Glu Val Arg Gln Leu Gln
1               5                   10                  15

Thr Leu Leu Asn Leu Cys Gly Tyr Asp Val Gly Lys Pro Asp Gly Ile
            20                  25                  30

Phe Gly Asn Asn Thr Phe Asn Gln Val Val Lys Phe Gln Lys Asp Asn
        35                  40                  45

Ser Leu Asp Ser Asp Gly Ile Val Gly Lys Asn Thr Trp Ala Glu Leu
    50                  55                  60

Phe Ser Lys Tyr Ser Pro Pro Ile Pro Tyr Lys Thr Ile Pro Met Pro
65                  70                  75                  80

Ile Ala Asn Lys Ser Arg Ala Ala Ala Thr Pro Val Met Asn Ala Val
                85                  90                  95

Glu Asn Ala Thr Gly Val Arg Ser Gln Leu Leu Leu Thr Phe Ala Ser
            100                 105                 110

Ile Glu Ser Ala Phe Asp Tyr Glu Met Lys Ala Lys Thr Ser Ser Ala
        115                 120                 125

Thr Gly Trp Phe Gln Phe Leu Thr Gly Thr Trp Lys Thr Met Ile Glu
    130                 135                 140

Asn Tyr Gly Pro Lys Tyr Gly Val Leu Thr Asp Pro Thr Gly Ala Leu
145                 150                 155                 160

Arg Lys Asp Pro Arg Ile Ser Ala Leu Met Gly Ala Glu Leu Ile Lys
                165                 170                 175

Glu Asn Met Asn Ile Leu Arg Pro Val Leu Lys Arg Glu Pro Thr Asp
            180                 185                 190

Thr Asp Leu Tyr Leu Ala His Phe Phe Gly Pro Gly Val Ala Arg Arg
        195                 200                 205

Phe Leu Thr Thr Gly Gln Asn Glu Leu Ala Ala Thr His Phe Pro Lys
    210                 215                 220

Glu Ala Gln Ala Asn Pro Thr Ile Phe Tyr Asn Lys Asp Gly Ser Pro
225                 230                 235                 240

Lys Thr Ile Gln Glu Val Tyr Asn Leu Met Asp Gly Lys Val Ala Ala
                245                 250                 255

His Arg Lys

<210>  24
<211>  259
<212>  PRT
<213>  Artificial Sequence

<220>
<223>  mutated KZ144 with C14R, C50S, T82I, I122M, M149P, A160T, A206V
       and S232T; without N-terminal methionine

<400>  24

Lys Val Leu Arg Lys Gly Asp Arg Gly Asp Glu Val Arg Gln Leu Gln
1               5                   10                  15

Thr Leu Leu Asn Leu Cys Gly Tyr Asp Val Gly Lys Pro Asp Gly Ile
            20                  25                  30

Phe Gly Asn Asn Thr Phe Asn Gln Val Val Lys Phe Gln Lys Asp Asn
        35                  40                  45

Ser Leu Asp Ser Asp Gly Ile Val Gly Lys Asn Thr Trp Ala Glu Leu
    50                  55                  60

Phe Ser Lys Tyr Ser Pro Pro Ile Pro Tyr Lys Thr Ile Pro Met Pro
65                  70                  75                  80

Ile Ala Asn Lys Ser Arg Ala Ala Ala Thr Pro Val Met Asn Ala Val
                85                  90                  95

Glu Asn Ala Thr Gly Val Arg Ser Gln Leu Leu Leu Thr Phe Ala Ser
            100                 105                 110

Ile Glu Ser Ala Phe Asp Tyr Glu Met Lys Ala Lys Thr Ser Ser Ala
        115                 120                 125

Thr Gly Trp Phe Gln Phe Leu Thr Gly Thr Trp Lys Thr Met Ile Glu
    130                 135                 140

Asn Tyr Gly Pro Lys Tyr Gly Val Leu Thr Asp Pro Thr Gly Thr Leu
145                 150                 155                 160

Arg Lys Asp Pro Arg Ile Ser Ala Leu Met Gly Ala Glu Leu Ile Lys
                165                 170                 175

Glu Asn Met Asn Ile Leu Arg Pro Val Leu Lys Arg Glu Pro Thr Asp
            180                 185                 190

Thr Asp Leu Tyr Leu Ala His Phe Phe Gly Pro Gly Val Ala Arg Arg
        195                 200                 205

Phe Leu Thr Thr Gly Gln Asn Glu Leu Ala Ala Thr His Phe Pro Lys
    210                 215                 220

Glu Ala Gln Ala Asn Pro Thr Ile Phe Tyr Asn Lys Asp Gly Ser Pro
225                 230                 235                 240

Lys Thr Ile Gln Glu Val Tyr Asn Leu Met Asp Gly Lys Val Ala Ala
                245                 250                 255

His Arg Lys

<210>  25
<211>  259
<212>  PRT
<213>  Artificial Sequence

<220>
<223>  mutated KZ144 with T82I, I122M, M149P,  A206V, S232T, C14R and
       C50N, without N-terminal methionine

<400>  25

Lys Val Leu Arg Lys Gly Asp Arg Gly Asp Glu Val Arg Gln Leu Gln
1               5                   10                  15

Thr Leu Leu Asn Leu Cys Gly Tyr Asp Val Gly Lys Pro Asp Gly Ile
            20                  25                  30

Phe Gly Asn Asn Thr Phe Asn Gln Val Val Lys Phe Gln Lys Asp Asn
        35                  40                  45

Asn Leu Asp Ser Asp Gly Ile Val Gly Lys Asn Thr Trp Ala Glu Leu
    50                  55                  60

Phe Ser Lys Tyr Ser Pro Pro Ile Pro Tyr Lys Thr Ile Pro Met Pro
65                  70                  75                  80

Ile Ala Asn Lys Ser Arg Ala Ala Ala Thr Pro Val Met Asn Ala Val
                85                  90                  95

Glu Asn Ala Thr Gly Val Arg Ser Gln Leu Leu Leu Thr Phe Ala Ser
            100                 105                 110

Ile Glu Ser Ala Phe Asp Tyr Glu Met Lys Ala Lys Thr Ser Ser Ala
        115                 120                 125

Thr Gly Trp Phe Gln Phe Leu Thr Gly Thr Trp Lys Thr Met Ile Glu
    130                 135                 140

Asn Tyr Gly Pro Lys Tyr Gly Val Leu Thr Asp Pro Thr Gly Ala Leu
145                 150                 155                 160

Arg Lys Asp Pro Arg Ile Ser Ala Leu Met Gly Ala Glu Leu Ile Lys
                165                 170                 175

Glu Asn Met Asn Ile Leu Arg Pro Val Leu Lys Arg Glu Pro Thr Asp
            180                 185                 190

Thr Asp Leu Tyr Leu Ala His Phe Phe Gly Pro Gly Val Ala Arg Arg
        195                 200                 205

Phe Leu Thr Thr Gly Gln Asn Glu Leu Ala Ala Thr His Phe Pro Lys
    210                 215                 220

Glu Ala Gln Ala Asn Pro Thr Ile Phe Tyr Asn Lys Asp Gly Ser Pro
225                 230                 235                 240

Lys Thr Ile Gln Glu Val Tyr Asn Leu Met Asp Gly Lys Val Ala Ala
                245                 250                 255

His Arg Lys

<210>  26
<211>  259
<212>  PRT
<213>  Artificial Sequence

<220>
<223>  mutated KZ144 with T82I, I122M, M149P,  I167L, A206V, S232T, C14R
       and C50N, without N-terminal methionine

<400>  26

Lys Val Leu Arg Lys Gly Asp Arg Gly Asp Glu Val Arg Gln Leu Gln
1               5                   10                  15

Thr Leu Leu Asn Leu Cys Gly Tyr Asp Val Gly Lys Pro Asp Gly Ile
            20                  25                  30

Phe Gly Asn Asn Thr Phe Asn Gln Val Val Lys Phe Gln Lys Asp Asn
        35                  40                  45

Asn Leu Asp Ser Asp Gly Ile Val Gly Lys Asn Thr Trp Ala Glu Leu
    50                  55                  60

Phe Ser Lys Tyr Ser Pro Pro Ile Pro Tyr Lys Thr Ile Pro Met Pro
65                  70                  75                  80

Ile Ala Asn Lys Ser Arg Ala Ala Ala Thr Pro Val Met Asn Ala Val
                85                  90                  95

Glu Asn Ala Thr Gly Val Arg Ser Gln Leu Leu Leu Thr Phe Ala Ser
            100                 105                 110

Ile Glu Ser Ala Phe Asp Tyr Glu Met Lys Ala Lys Thr Ser Ser Ala
        115                 120                 125

Thr Gly Trp Phe Gln Phe Leu Thr Gly Thr Trp Lys Thr Met Ile Glu
    130                 135                 140

Asn Tyr Gly Pro Lys Tyr Gly Val Leu Thr Asp Pro Thr Gly Ala Leu
145                 150                 155                 160

Arg Lys Asp Pro Arg Leu Ser Ala Leu Met Gly Ala Glu Leu Ile Lys
                165                 170                 175

Glu Asn Met Asn Ile Leu Arg Pro Val Leu Lys Arg Glu Pro Thr Asp
            180                 185                 190

Thr Asp Leu Tyr Leu Ala His Phe Phe Gly Pro Gly Val Ala Arg Arg
        195                 200                 205

Phe Leu Thr Thr Gly Gln Asn Glu Leu Ala Ala Thr His Phe Pro Lys
    210                 215                 220

Glu Ala Gln Ala Asn Pro Thr Ile Phe Tyr Asn Lys Asp Gly Ser Pro
225                 230                 235                 240

Lys Thr Ile Gln Glu Val Tyr Asn Leu Met Asp Gly Lys Val Ala Ala
                245                 250                 255

His Arg Lys

<210>  27
<211>  259
<212>  PRT

<213>  Artificial Sequence

<220>
<223>  mutated KZ144 with T82I, I122M, M149P,  N179F, A206V, S232T, C14R
       and C50N, without N-terminal methionine

<400>  27

Lys Val Leu Arg Lys Gly Asp Arg Gly Asp Glu Val Arg Gln Leu Gln
1               5                   10                  15

Thr Leu Leu Asn Leu Cys Gly Tyr Asp Val Gly Lys Pro Asp Gly Ile
            20                  25                  30

Phe Gly Asn Asn Thr Phe Asn Gln Val Val Lys Phe Gln Lys Asp Asn
        35                  40                  45

Asn Leu Asp Ser Asp Gly Ile Val Gly Lys Asn Thr Trp Ala Glu Leu
    50                  55                  60

Phe Ser Lys Tyr Ser Pro Pro Ile Pro Tyr Lys Thr Ile Pro Met Pro
65                  70                  75                  80

Ile Ala Asn Lys Ser Arg Ala Ala Ala Thr Pro Val Met Asn Ala Val
                85                  90                  95

Glu Asn Ala Thr Gly Val Arg Ser Gln Leu Leu Leu Thr Phe Ala Ser
            100                 105                 110

Ile Glu Ser Ala Phe Asp Tyr Glu Met Lys Ala Lys Thr Ser Ser Ala
        115                 120                 125

Thr Gly Trp Phe Gln Phe Leu Thr Gly Thr Trp Lys Thr Met Ile Glu
    130                 135                 140

Asn Tyr Gly Pro Lys Tyr Gly Val Leu Thr Asp Pro Thr Gly Ala Leu
145                 150                 155                 160

Arg Lys Asp Pro Arg Leu Ser Ala Leu Met Gly Ala Glu Leu Ile Lys
                165                 170                 175

Glu Phe Met Asn Ile Leu Arg Pro Val Leu Lys Arg Glu Pro Thr Asp
            180                 185                 190

Thr Asp Leu Tyr Leu Ala His Phe Phe Gly Pro Gly Val Ala Arg Arg
        195                 200                 205

Phe Leu Thr Thr Gly Gln Asn Glu Leu Ala Ala Thr His Phe Pro Lys
    210                 215                 220

Glu Ala Gln Ala Asn Pro Thr Ile Phe Tyr Asn Lys Asp Gly Ser Pro
225                 230                 235                 240

Lys Thr Ile Gln Glu Val Tyr Asn Leu Met Asp Gly Lys Val Ala Ala
                245                 250                 255

His Arg Lys

<210>  28
<211>  259
<212>  PRT
<213>  Artificial Sequence

<220>
<223>  mutated KZ144 with T82I, I122M, M149P,  A206V, T212N, S232T, C14R
       and C50N, without N-terminal methionine

<400>  28

Lys Val Leu Arg Lys Gly Asp Arg Gly Asp Glu Val Arg Gln Leu Gln
1               5                   10                  15

Thr Leu Leu Asn Leu Cys Gly Tyr Asp Val Gly Lys Pro Asp Gly Ile
            20                  25                  30

Phe Gly Asn Asn Thr Phe Asn Gln Val Val Lys Phe Gln Lys Asp Asn
        35                  40                  45

Asn Leu Asp Ser Asp Gly Ile Val Gly Lys Asn Thr Trp Ala Glu Leu
    50                  55                  60

Phe Ser Lys Tyr Ser Pro Pro Ile Pro Tyr Lys Thr Ile Pro Met Pro
65                  70                  75                  80

Ile Ala Asn Lys Ser Arg Ala Ala Ala Thr Pro Val Met Asn Ala Val
                85                  90                  95

Glu Asn Ala Thr Gly Val Arg Ser Gln Leu Leu Leu Thr Phe Ala Ser
            100                 105                 110

Ile Glu Ser Ala Phe Asp Tyr Glu Met Lys Ala Lys Thr Ser Ser Ala
        115                 120                 125

Thr Gly Trp Phe Gln Phe Leu Thr Gly Thr Trp Lys Thr Met Ile Glu
    130                 135                 140

Asn Tyr Gly Pro Lys Tyr Gly Val Leu Thr Asp Pro Thr Gly Ala Leu
145                 150                 155                 160

Arg Lys Asp Pro Arg Ile Ser Ala Leu Met Gly Ala Glu Leu Ile Lys
                165                 170                 175

Glu Asn Met Asn Ile Leu Arg Pro Val Leu Lys Arg Glu Pro Thr Asp
            180                 185                 190

Thr Asp Leu Tyr Leu Ala His Phe Phe Gly Pro Gly Val Ala Arg Arg
        195                 200                 205

Phe Leu Asn Thr Gly Gln Asn Glu Leu Ala Ala Thr His Phe Pro Lys
    210                 215                 220

Glu Ala Gln Ala Asn Pro Thr Ile Phe Tyr Asn Lys Asp Gly Ser Pro
225                 230                 235                 240

Lys Thr Ile Gln Glu Val Tyr Asn Leu Met Asp Gly Lys Val Ala Ala
                245                 250                 255

His Arg Lys

<210>  29
<211>  259
<212>  PRT
<213>  Artificial Sequence

<220>
<223>  mutated KZ144 with T82I, I122M, M149P,  A206V, P224Q, S232T, C14R
       and C50N, without N-terminal methionine

<400>  29

Lys Val Leu Arg Lys Gly Asp Arg Gly Asp Glu Val Arg Gln Leu Gln
1               5                   10                  15

Thr Leu Leu Asn Leu Cys Gly Tyr Asp Val Gly Lys Pro Asp Gly Ile
            20                  25                  30

Phe Gly Asn Asn Thr Phe Asn Gln Val Val Lys Phe Gln Lys Asp Asn
        35                  40                  45

Asn Leu Asp Ser Asp Gly Ile Val Gly Lys Asn Thr Trp Ala Glu Leu
    50                  55                  60

Phe Ser Lys Tyr Ser Pro Pro Ile Pro Tyr Lys Thr Ile Pro Met Pro
65                  70                  75                  80

Ile Ala Asn Lys Ser Arg Ala Ala Ala Thr Pro Val Met Asn Ala Val
                85                  90                  95

Glu Asn Ala Thr Gly Val Arg Ser Gln Leu Leu Leu Thr Phe Ala Ser
            100                 105                 110

Ile Glu Ser Ala Phe Asp Tyr Glu Met Lys Ala Lys Thr Ser Ser Ala
        115                 120                 125

Thr Gly Trp Phe Gln Phe Leu Thr Gly Thr Trp Lys Thr Met Ile Glu
    130                 135                 140

Asn Tyr Gly Pro Lys Tyr Gly Val Leu Thr Asp Pro Thr Gly Ala Leu
145                 150                 155                 160

Arg Lys Asp Pro Arg Ile Ser Ala Leu Met Gly Ala Glu Leu Ile Lys
                165                 170                 175

Glu Asn Met Asn Ile Leu Arg Pro Val Leu Lys Arg Glu Pro Thr Asp
            180                 185                 190

Thr Asp Leu Tyr Leu Ala His Phe Phe Gly Pro Gly Val Ala Arg Arg
        195                 200                 205

Phe Leu Thr Thr Gly Gln Asn Glu Leu Ala Ala Thr His Phe Gln Lys
    210                 215                 220

Glu Ala Gln Ala Asn Pro Thr Ile Phe Tyr Asn Lys Asp Gly Ser Pro
225                 230                 235                 240

Lys Thr Ile Gln Glu Val Tyr Asn Leu Met Asp Gly Lys Val Ala Ala
                245                 250                 255

His Arg Lys

<210>  30
<211>  259
<212>  PRT
<213>  Artificial Sequence

<220>
<223>  mutated KZ144 with T82I, I122M, M149P, L154T, A206V, S232T, C14R
       and C50N, without N-terminal methionine

<400>  30

Lys Val Leu Arg Lys Gly Asp Arg Gly Asp Glu Val Arg Gln Leu Gln
1               5                   10                  15

Thr Leu Leu Asn Leu Cys Gly Tyr Asp Val Gly Lys Pro Asp Gly Ile
            20                  25                  30

Phe Gly Asn Asn Thr Phe Asn Gln Val Val Lys Phe Gln Lys Asp Asn
        35                  40                  45

Asn Leu Asp Ser Asp Gly Ile Val Gly Lys Asn Thr Trp Ala Glu Leu
    50                  55                  60

Phe Ser Lys Tyr Ser Pro Pro Ile Pro Tyr Lys Thr Ile Pro Met Pro
65                  70                  75                  80

Ile Ala Asn Lys Ser Arg Ala Ala Ala Thr Pro Val Met Asn Ala Val
                85                  90                  95

Glu Asn Ala Thr Gly Val Arg Ser Gln Leu Leu Leu Thr Phe Ala Ser
            100                 105                 110

Ile Glu Ser Ala Phe Asp Tyr Glu Met Lys Ala Lys Thr Ser Ser Ala
        115                 120                 125

Thr Gly Trp Phe Gln Phe Leu Thr Gly Thr Trp Lys Thr Met Ile Glu
    130                 135                 140

Asn Tyr Gly Pro Lys Tyr Gly Val Thr Thr Asp Pro Thr Gly Ala Leu
145                 150                 155                 160

Arg Lys Asp Pro Arg Ile Ser Ala Leu Met Gly Ala Glu Leu Ile Lys
                165                 170                 175

Glu Asn Met Asn Ile Leu Arg Pro Val Leu Lys Arg Glu Pro Thr Asp
            180                 185                 190

Thr Asp Leu Tyr Leu Ala His Phe Phe Gly Pro Gly Val Ala Arg Arg
        195                 200                 205

Phe Leu Thr Thr Gly Gln Asn Glu Leu Ala Ala Thr His Phe Pro Lys
    210                 215                 220

Glu Ala Gln Ala Asn Pro Thr Ile Phe Tyr Asn Lys Asp Gly Ser Pro
225                 230                 235                 240

Lys Thr Ile Gln Glu Val Tyr Asn Leu Met Asp Gly Lys Val Ala Ala
                245                 250                 255

His Arg Lys

<210>  31
<211>  137

<212>  PRT
<213>  Bacteriophage S-394

<400>  31

Met Ser Phe Lys Phe Gly Lys Asn Ser Glu Lys Gln Leu Ala Thr Val
1               5                   10                  15

Lys Pro Glu Leu Gln Lys Val Ala Arg Arg Ala Leu Glu Leu Ser Pro
            20                  25                  30

Tyr Asp Phe Thr Ile Val Gln Gly Ile Arg Thr Val Ala Gln Ser Ala
        35                  40                  45

Gln Asn Ile Ala Asn Gly Thr Ser Phe Leu Lys Asp Pro Ser Lys Ser
    50                  55                  60

Lys His Val Thr Gly Asp Ala Ile Asp Phe Ala Pro Tyr Ile Asn Gly
65                  70                  75                  80

Lys Ile Asp Trp Lys Asp Leu Glu Ala Phe Trp Ala Val Lys Lys Ala
                85                  90                  95

Phe Glu Gln Ala Gly Lys Glu Leu Gly Ile Lys Leu Arg Phe Gly Ala
            100                 105                 110

Asp Trp Asn Ser Ser Gly Asp Tyr His Asp Glu Ile Asp Arg Gly Thr
        115                 120                 125

Tyr Asp Gly Gly His Val Glu Leu Val
    130                 135


<210>  32
<211>  260
<212>  PRT
<213>  unknown

<220>
<223>  phiKZgp144

<400>  32

Met Lys Val Leu Arg Lys Gly Asp Arg Gly Asp Glu Val Cys Gln Leu
1               5                   10                  15

Gln Thr Leu Leu Asn Leu Cys Gly Tyr Asp Val Gly Lys Pro Asp Gly
            20                  25                  30

Ile Phe Gly Asn Asn Thr Phe Asn Gln Val Val Lys Phe Gln Lys Asp
        35                  40                  45

Asn Cys Leu Asp Ser Asp Gly Ile Val Gly Lys Asn Thr Trp Ala Glu
    50                  55                  60

Leu Phe Ser Lys Tyr Ser Pro Pro Ile Pro Tyr Lys Thr Ile Pro Met
65                  70                  75                  80

Pro Thr Ala Asn Lys Ser Arg Ala Ala Ala Thr Pro Val Met Asn Ala
                85                  90                  95

Val Glu Asn Ala Thr Gly Val Arg Ser Gln Leu Leu Leu Thr Phe Ala
            100                 105                 110

Ser Ile Glu Ser Ala Phe Asp Tyr Glu Ile Lys Ala Lys Thr Ser Ser
        115                 120                 125

Ala Thr Gly Trp Phe Gln Phe Leu Thr Gly Thr Trp Lys Thr Met Ile
    130                 135                 140

Glu Asn Tyr Gly Met Lys Tyr Gly Val Leu Thr Asp Pro Thr Gly Ala
145                 150                 155                 160

Leu Arg Lys Asp Pro Arg Ile Ser Ala Leu Met Gly Ala Glu Leu Ile
                165                 170                 175

Lys Glu Asn Met Asn Ile Leu Arg Pro Val Leu Lys Arg Glu Pro Thr
            180                 185                 190

Asp Thr Asp Leu Tyr Leu Ala His Phe Phe Gly Pro Gly Ala Ala Arg
        195                 200                 205

Arg Phe Leu Thr Thr Gly Gln Asn Glu Leu Ala Ala Thr His Phe Pro
    210                 215                 220

Lys Glu Ala Gln Ala Asn Pro Ser Ile Phe Tyr Asn Lys Asp Gly Ser
225                 230                 235                 240

Pro Lys Thr Ile Gln Glu Val Tyr Asn Leu Met Asp Gly Lys Val Ala
                245                 250                 255

Ala His Arg Lys
            260

<210>  33
<211>  260
<212>  PRT
<213>  artificial sequence

<220>
<223>  mutated KZ144 with C14S, C23S and C50S

<400>  33

Met Lys Val Leu Arg Lys Gly Asp Arg Gly Asp Glu Val Ser Gln Leu
1               5                   10                  15

Gln Thr Leu Leu Asn Leu Ser Gly Tyr Asp Val Gly Lys Pro Asp Gly
            20                  25                  30

Ile Phe Gly Asn Asn Thr Phe Asn Gln Val Val Lys Phe Gln Lys Asp
        35                  40                  45

Asn Ser Leu Asp Ser Asp Gly Ile Val Gly Lys Asn Thr Trp Ala Glu
    50                  55                  60

Leu Phe Ser Lys Tyr Ser Pro Pro Ile Pro Tyr Lys Thr Ile Pro Met
65                  70                  75                  80

Pro Thr Ala Asn Lys Ser Arg Ala Ala Ala Thr Pro Val Met Asn Ala
                85                  90                  95

Val Glu Asn Ala Thr Gly Val Arg Ser Gln Leu Leu Leu Thr Phe Ala
            100                 105                 110

Ser Ile Glu Ser Ala Phe Asp Tyr Glu Ile Lys Ala Lys Thr Ser Ser
        115                 120                 125

Ala Thr Gly Trp Phe Gln Phe Leu Thr Gly Thr Trp Lys Thr Met Ile
    130                 135                 140

Glu Asn Tyr Gly Met Lys Tyr Gly Val Leu Thr Asp Pro Thr Gly Ala
145                 150                 155                 160

Leu Arg Lys Asp Pro Arg Ile Ser Ala Leu Met Gly Ala Glu Leu Ile
                165                 170                 175

Lys Glu Asn Met Asn Ile Leu Arg Pro Val Leu Lys Arg Glu Pro Thr
            180                 185                 190

Asp Thr Asp Leu Tyr Leu Ala His Phe Phe Gly Pro Gly Ala Ala Arg
        195                 200                 205

Arg Phe Leu Thr Thr Gly Gln Asn Glu Leu Ala Ala Thr His Phe Pro
    210                 215                 220

Lys Glu Ala Gln Ala Asn Pro Ser Ile Phe Tyr Asn Lys Asp Gly Ser
225                 230                 235                 240

Pro Lys Thr Ile Gln Glu Val Tyr Asn Leu Met Asp Gly Lys Val Ala
                245                 250                 255

Ala His Arg Lys
            260

<210>  34
<211>  327
<212>  PRT
<213>  unknown

<220>
<223>  OBPgp279

<400>  34

Lys Asn Ser Glu Lys Asn Ala Ser Ile Ile Met Ser Ile Gln Arg Thr
1               5                   10                  15

Leu Ala Ser Leu Ser Leu Tyr Gly Gly Arg Ile Asp Gly Leu Phe Gly
            20                  25                  30

Glu Lys Cys Arg Gly Ala Ile Ile Leu Met Leu Asn Lys Val Tyr Pro
        35                  40                  45

Asn Phe Ser Thr Asn Lys Leu Pro Ser Asn Thr Tyr Glu Ala Glu Ser
    50                  55                  60

Val Phe Thr Phe Leu Gln Thr Ala Leu Ala Gly Val Gly Leu Tyr Thr
65                  70                  75                  80

Ile Thr Ile Asp Gly Lys Trp Gly Gly Thr Ser Gln Gly Ala Ile Asp
                85                  90                  95

Ala Leu Val Lys Ser Tyr Arg Gln Ile Thr Glu Ala Glu Arg Ala Gly
            100                 105                 110

Ser Thr Leu Pro Leu Gly Leu Ala Thr Val Met Ser Lys His Met Ser
        115                 120                 125

Ile Glu Gln Leu Arg Ala Met Leu Pro Thr Asp Arg Gln Gly Tyr Ala
    130                 135                 140

Glu Val Tyr Ile Asp Pro Leu Asn Glu Thr Met Asp Ile Phe Glu Ile
145                 150                 155                 160

Asn Thr Pro Leu Arg Ile Ala His Phe Met Ala Gln Ile Leu His Glu
                165                 170                 175

Thr Ala Cys Phe Lys Tyr Thr Glu Glu Leu Ala Ser Gly Lys Ala Tyr

            180                 185                 190

Glu Gly Arg Ala Asp Leu Gly Asn Thr Arg Pro Gly Asp Gly Pro Leu
        195                 200                 205

Phe Lys Gly Arg Gly Leu Leu Gln Ile Thr Gly Arg Leu Asn Tyr Val
    210                 215                 220

Lys Cys Gln Val Tyr Leu Arg Glu Lys Leu Lys Asp Pro Thr Phe Asp
225                 230                 235                 240

Ile Thr Ser Ser Val Thr Cys Ala Gln Gln Leu Ser Glu Ser Pro Leu
                245                 250                 255

Leu Ala Ala Leu Ala Ser Gly Tyr Phe Trp Arg Phe Ile Lys Pro Lys
            260                 265                 270

Leu Asn Glu Thr Ala Asp Lys Asp Asp Ile Tyr Trp Val Ser Val Tyr
        275                 280                 285

Val Asn Gly Tyr Ala Lys Gln Ala Asn Pro Tyr Tyr Pro Asn Arg Asp
    290                 295                 300

Lys Glu Pro Asn His Met Lys Glu Arg Val Gln Met Leu Ala Val Thr
305                 310                 315                 320

Lys Lys Ala Leu Gly Ile Val
                325

<210> 35
<211> 235
<212> PRT
<213> unknown

<220>
<223> PVP-SE1gp146

<400> 35

Asn Ala Ala Ile Ala Glu Ile Gln Arg Met Leu Ile Glu Gly Gly Phe
1               5                   10                  15

Ser Val Gly Lys Ser Gly Ala Asp Gly Leu Tyr Gly Pro Ala Thr Lys
            20                  25                  30

Ala Ala Leu Gln Lys Cys Ile Ala Gln Ala Thr Ser Gly Asn Asn Lys
        35                  40                  45

Gly Gly Thr Leu Lys Leu Thr Gln Ala Gln Leu Asp Lys Ile Phe Pro
    50                  55                  60

Val Gly Ala Ser Ser Gly Arg Asn Ala Lys Phe Leu Lys Pro Leu Asn
65                  70                  75                  80

Asp Leu Phe Glu Lys Thr Glu Ile Asn Thr Val Asn Arg Val Ala Gly
                85                  90                  95

Phe Leu Ser Gln Ile Gly Val Glu Ser Ala Glu Phe Arg Tyr Val Arg
            100                 105                 110

Glu Leu Gly Asn Asp Ala Tyr Phe Asp Lys Tyr Asp Thr Gly Pro Ile
        115                 120                 125

Ala Glu Arg Leu Gly Asn Thr Pro Gln Lys Asp Gly Asp Gly Ala Lys
    130                 135                 140

Tyr Lys Gly Arg Gly Leu Ile Gln Val Thr Gly Leu Ala Asn Tyr Lys
145                 150                 155                 160

Ala Cys Gly Lys Ala Leu Gly Leu Asp Leu Val Asn His Pro Glu Leu
                165                 170                 175

Leu Glu Gln Pro Glu Tyr Ala Val Ala Ser Ala Gly Trp Tyr Trp Asp
            180                 185                 190

Thr Arg Asn Ile Asn Ala Ala Cys Asp Ala Asp Asp Ile Val Lys Ile
        195                 200                 205

Thr Lys Leu Val Asn Gly Gly Thr Asn His Leu Ala Glu Arg Thr Ala
    210                 215                 220

Tyr Tyr Lys Lys Ala Lys Ser Val Leu Thr Ser
225                 230                 235

<210>  36
<211>  300
<212>  PRT
<213>  artificial

<220>
<223>  SEQ ID NO: 124 of WO2015/155244

<400>  36

Met Lys Phe Phe Arg Lys Leu Lys Lys Ser Val Lys Lys Arg Ala Lys
1               5                   10                  15

Glu Phe Phe Lys Lys Pro Arg Val Ile Gly Val Ser Ile Pro Phe Gly
            20                  25                  30

Ser Lys Val Leu Arg Lys Gly Asp Arg Gly Asp Glu Val Cys Gln Leu
        35                  40                  45

Gln Thr Leu Leu Asn Leu Cys Gly Tyr Asp Val Gly Lys Pro Asp Gly
    50                  55                  60

Ile Phe Gly Asn Asn Thr Phe Asn Gln Val Val Lys Phe Gln Lys Asp
65                  70                  75                  80

Asn Cys Leu Asp Ser Asp Gly Ile Val Gly Lys Asn Thr Trp Ala Glu
                85                  90                  95

Leu Phe Ser Lys Tyr Ser Pro Pro Ile Pro Tyr Lys Thr Ile Pro Met
            100                 105                 110

Pro Thr Ala Asn Lys Ser Arg Ala Ala Ala Thr Pro Val Met Asn Ala
        115                 120                 125

Val Glu Asn Ala Thr Gly Val Arg Ser Gln Leu Leu Leu Thr Phe Ala
    130                 135                 140

Ser Ile Glu Ser Ala Phe Asp Tyr Glu Ile Lys Ala Lys Thr Ser Ser
145                 150                 155                 160

Ala Thr Gly Trp Phe Gln Phe Leu Thr Gly Thr Trp Lys Thr Met Ile
                165                 170                 175

Glu Asn Tyr Gly Met Lys Tyr Gly Val Leu Thr Asp Pro Thr Gly Ala
            180                 185                 190

Leu Arg Lys Asp Pro Arg Ile Ser Ala Leu Met Gly Ala Glu Leu Ile
        195                 200                 205

Lys Glu Asn Met Asn Ile Leu Arg Pro Val Leu Lys Arg Glu Pro Thr
    210                 215                 220

Asp Thr Asp Leu Tyr Leu Ala His Phe Phe Gly Pro Gly Ala Ala Arg
225                 230                 235                 240

Arg Phe Leu Thr Thr Gly Gln Asn Glu Leu Ala Ala Thr His Phe Pro
                245                 250                 255

Lys Glu Ala Gln Ala Asn Pro Ser Ile Phe Tyr Asn Lys Asp Gly Ser
            260                 265                 270

Pro Lys Thr Ile Gln Glu Val Tyr Asn Leu Met Asp Gly Lys Val Ala
        275                 280                 285

Ala His Arg Lys Leu Glu His His His His His His
    290                 295                 300


<210>  37
<211>  17
<212>  PRT
<213>  Artificial Sequence

<220>
<223>  MSI-78 (4-20)  peptide

<400>  37

Lys Phe Leu Lys Lys Ala Lys Lys Phe Gly Lys Ala Phe Val Lys Ile
1               5                   10                  15


Leu




<210>  38
<211>  6
<212>  PRT
<213>  artificial

<220>
<223>  synthetic sequence

<400>  38

Lys Arg Lys Lys Arg Lys
1               5


<210>  39
<211>  5
<212>  PRT
<213>  artificial

<220>
<223>  synethtic sequence


<220>
<221>  misc_feature
<222>  (3)..(3)
<223>  Xaa can be any naturally occurring amino acid

<400>  39

Lys Arg Xaa Lys Arg
1               5


<210>  40
<211>  5
<212>  PRT
<213>  artificial

<220>
<223>  synthetic sequence

<400>  40

Lys Arg Ser Lys Arg
1               5



<210>  41
<211>  5
<212>  PRT
<213>  artificial

<220>
<223>  synthetic sequence

<400>  41

Lys Arg Gly Ser Gly
1               5



<210>  42
<211>  9
<212>  PRT
<213>  artificial

<220>
<223>  synthetic sequence

<400>  42

Lys Arg Lys Lys Arg Lys Lys Arg Lys
1               5



<210>  43
<211>  9
<212>  PRT
<213>  artificial

<220>
<223>  synthetic sequence

<400>  43

Arg Arg Arg Arg Arg Arg Arg Arg Arg
1               5



<210>  44
<211>  8
<212>  PRT
<213>  artificial

<220>
<223>  synthetic sequence

<400>  44

Lys Lys Lys Lys Lys Lys Lys Lys
1               5

<210>  45
<211>  10
<212>  PRT
<213>  artificial

<220>
<223>  synthetic sequence

<400>  45

Lys Arg Lys Lys Arg Lys Lys Arg Lys Lys
1               5                   10


<210>  46
<211>  12
<212>  PRT
<213>  artificial

<220>
<223>  synthetic sequence

<400>  46

Lys Arg Lys Lys Arg Lys Lys Arg Lys Lys Arg Lys
1               5                   10


<210>  47
<211>  14
<212>  PRT
<213>  artificial

<220>
<223>  synthetic sequence

<400>  47

Lys Arg Lys Lys Arg Lys Lys Arg Lys Lys Arg Lys Lys Arg
1               5                   10


<210>  48
<211>  16
<212>  PRT
<213>  artificial

<220>
<223>  synthetic sequence

<400>  48

Lys Lys Lys Lys Lys Lys Lys Lys Lys Lys Lys Lys Lys Lys Lys Lys
1               5                   10                  15


<210>  49
<211>  18
<212>  PRT
<213>  artificial

<220>
<223>  synthetic sequence

<400>  49

Lys Arg Lys Lys Arg Lys Lys Arg Lys Lys Arg Lys Lys Arg Lys Lys
1               5                   10                  15

Arg Lys

<210>  50
<211>  19
<212>  PRT
<213>  artificial

<220>
<223>  synthetic sequence

<400>  50

Lys Arg Lys Lys Arg Lys Lys Arg Lys Lys Arg Lys Lys Arg Lys Lys
1               5                   10                  15

Arg Lys Lys

<210>  51
<211>  19
<212>  PRT
<213>  artificial

<220>
<223>  synthetic sequence

<400>  51

Arg Arg Arg Arg Arg Arg Arg Arg Arg Arg Arg Arg Arg Arg Arg Arg
1               5                   10                  15

Arg Arg Arg

<210>  52
<211>  19
<212>  PRT
<213>  artificial

<220>
<223>  synthetic sequence

<400>  52

Lys Lys Lys Lys Lys Lys Lys Lys Lys Lys Lys Lys Lys Lys Lys Lys
1               5                   10                  15

Lys Lys Lys

<210> 53
<211> 20
<212> PRT
<213> artificial

<220>
<223> synthetic sequence

<400> 53

Lys Arg Lys Lys Arg Lys Lys Arg Lys Arg Ser Lys Arg Lys Lys Arg
1               5                   10                  15

Lys Lys Arg Lys
            20

<210> 54
<211> 21
<212> PRT
<213> artificial

<220>
<223> synthetic sequence

<400> 54

Lys Arg Lys Lys Arg Lys Lys Arg Lys Arg Ser Lys Arg Lys Lys Arg
1               5                   10                  15

Lys Lys Arg Lys Lys
            20

<210> 55
<211> 21
<212> PRT
<213> artificial

<220>
<223> synthetic sequence

<400> 55

Lys Arg Lys Lys Arg Lys Lys Arg Lys Lys Arg Lys Lys Arg Lys Lys
1               5                   10                  15

Arg Lys Lys Arg Lys
            20

<210> 56
<211> 22
<212> PRT
<213> artificial

<220>
<223>  synthetic sequence

<400>  56

Lys Arg Lys Lys Arg Lys Lys Arg Lys Arg Gly Ser Gly Lys Arg Lys
1               5                   10                  15

Lys Arg Lys Lys Arg Lys
            20

<210>  57
<211>  24
<212>  PRT
<213>  artificial

<220>
<223>  synthetic sequence

<400>  57

Lys Arg Lys Lys Arg Lys Lys Arg Lys Arg Gly Ser Gly Ser Gly Lys
1               5                   10                  15

Arg Lys Lys Arg Lys Lys Arg Lys
            20

<210>  58
<211>  25
<212>  PRT
<213>  artificial

<220>
<223>  synthetic sequence

<400>  58

Lys Arg Lys Lys Arg Lys Lys Arg Lys Lys Arg Lys Lys Arg Lys Lys
1               5                   10                  15

Arg Lys Lys Arg Lys Lys Arg Lys Lys
            20                  25

<210>  59
<211>  31
<212>  PRT
<213>  artificial

<220>
<223>  synthetic sequence

<400>  59

Lys Arg Lys Lys Arg Lys Lys Arg Lys Arg Ser Lys Arg Lys Lys Arg
1               5                   10                  15

Lys Lys Arg Lys Arg Ser Lys Arg Lys Lys Arg Lys Lys Arg Lys
            20                  25                  30

<210>  60
<211>  38
<212>  PRT
<213>  artificial

<220>
<223>  synthetic sequence

<400>  60

Lys Arg Lys Lys Arg Lys Lys Arg Lys Arg Gly Ser Gly Ser Gly Lys
1               5                   10                  15

Arg Lys Lys Arg Lys Lys Arg Lys Gly Ser Gly Ser Gly Lys Arg Lys
            20                  25                  30

Lys Arg Lys Lys Arg Lys
        35

<210>  61
<211>  39
<212>  PRT
<213>  artificial

<220>
<223>  synthetic sequence

<400>  61

Lys Arg Lys Lys Arg Lys Lys Arg Lys Lys Arg Lys Lys Arg Lys Lys
1               5                   10                  15

Arg Lys Lys Arg Lys Lys Arg Lys Lys Arg Lys Lys Arg Lys Lys Arg
            20                  25                  30

Lys Lys Arg Lys Lys Arg Lys
        35

<210>  62
<211>  42
<212>  PRT
<213>  artificial

<220>
<223>  synthetic sequence

<400>  62

Lys Arg Lys Lys Arg Lys Lys Arg Lys Arg Ser Lys Arg Lys Lys Arg
1               5                   10                  15

Lys Lys Arg Lys Arg Ser Lys Arg Lys Lys Arg Lys Lys Arg Lys Arg

20                  25                  30

Ser Lys Arg Lys Lys Arg Lys Lys Arg Lys
        35                  40

<210>  63
<211>  37
<212>  PRT
<213>  Homo sapiens

<400>  63

Leu Leu Gly Asp Phe Phe Arg Lys Ser Lys Glu Lys Ile Gly Lys Glu
1               5                   10                  15

Phe Lys Arg Ile Val Gln Arg Ile Lys Asp Phe Leu Arg Asn Leu Val
            20                  25                  30

Pro Arg Thr Glu Ser
        35

<210>  64
<211>  29
<212>  PRT
<213>  unknown

<220>
<223>  SMAP-29 sheep

<400>  64

Arg Gly Leu Arg Arg Leu Gly Arg Lys Ile Ala His Gly Val Lys Lys
1               5                   10                  15

Tyr Gly Pro Thr Val Leu Arg Ile Ile Arg Ile Ala Gly
            20                  25

<210>  65
<211>  13
<212>  PRT
<213>  unknown

<220>
<223>  Indolicidine bovine

<400>  65

Ile Leu Pro Trp Lys Trp Pro Trp Trp Pro Trp Arg Arg
1               5                   10

<210>  66
<211>  18
<212>  PRT
<213>  unknown

<220>
<223>  Protegrin Porcine

<400>  66

Arg Gly Gly Arg Leu Cys Tyr Cys Arg Arg Arg Phe Cys Val Cys Val
1               5                   10                  15

Gly Arg

<210>  67
<211>  31
<212>  PRT
<213>  unknown

<220>
<223>  Cecropin P1 Mammal (pig)

<400>  67

Ser Trp Leu Ser Lys Thr Ala Lys Lys Leu Glu Asn Ser Ala Lys Lys
1               5                   10                  15

Arg Ile Ser Glu Gly Ile Ala Ile Ala Ile Gln Gly Gly Pro Arg
            20                  25                  30

<210>  68
<211>  23
<212>  PRT
<213>  unknown

<220>
<223>  Magainin frog

<400>  68

Gly Ile Gly Lys Phe Leu His Ser Ala Lys Lys Phe Gly Lys Ala Phe
1               5                   10                  15

Val Gly Glu Ile Met Asn Ser
            20

<210>  69
<211>  25
<212>  PRT
<213>  unknown

<220>
<223>  Pleurocidin fish

<400>  69

Gly Trp Gly Ser Phe Phe Lys Lys Ala Ala His Val Gly Lys His Val
1               5                   10                  15

Gly Lys Ala Ala Leu Thr His Tyr Leu
                20                  25

<210>  70
<211>  36
<212>  PRT
<213>  Aedes aegypti

<400>  70

Gly Gly Leu Lys Lys Leu Gly Lys Lys Leu Glu Gly Ala Gly Lys Arg
1               5                   10                  15

Val Phe Asn Ala Ala Glu Lys Ala Leu Pro Val Val Ala Gly Ala Lys
            20                  25                  30

Ala Leu Arg Lys
        35

<210>  71
<211>  40
<212>  PRT
<213>  Drosophila melanogaster

<400>  71

Gly Trp Leu Lys Lys Ile Gly Lys Lys Ile Glu Arg Val Gly Gln His
1               5                   10                  15

Thr Arg Asp Ala Thr Ile Gln Gly Leu Gly Ile Pro Gln Gln Ala Ala
            20                  25                  30

Asn Val Ala Ala Thr Ala Arg Gly
        35                  40

<210>  72
<211>  21
<212>  PRT
<213>  unknown

<220>
<223>  Buforin II vertebrate

<400>  72

Thr Arg Ser Ser Arg Ala Gly Leu Gln Phe Pro Val Gly Arg Val His
1               5                   10                  15

Arg Leu Leu Arg Lys
            20

<210>  73
<211>  39
<212>  PRT

<213>  unknown

<220>
<223>  Sarcotoxin IA Fly

<400>  73

Gly Trp Leu Lys Lys Ile Gly Lys Lys Ile Glu Arg Val Gly Gln His
1               5                   10                  15

Thr Arg Asp Ala Thr Ile Gln Gly Leu Gly Ile Ala Gln Gln Ala Ala
            20                  25                  30

Asn Val Ala Ala Thr Ala Arg
        35

<210>  74
<211>  17
<212>  PRT
<213>  Apis mellifera

<400>  74

Ala Asn Arg Pro Val Tyr Ile Pro Pro Pro Arg Pro Pro His Pro Arg
1               5                   10                  15

Leu

<210>  75
<211>  24
<212>  PRT
<213>  unknown

<220>
<223>  Ascaphine 5 Frog

<400>  75

Gly Ile Lys Asp Trp Ile Lys Gly Ala Ala Lys Lys Leu Ile Lys Thr
1               5                   10                  15

Val Ala Ser His Ile Ala Asn Gln
            20

<210>  76
<211>  22
<212>  PRT
<213>  unknown

<220>
<223>  Nigrocine 2 Frog

<400>  76

Gly Leu Leu Ser Lys Val Leu Gly Val Gly Lys Lys Val Leu Cys Gly

1               5                   10                  15

Val Ser Gly Leu Val Cys
            20

<210>  77
<211>  24
<212>  PRT
<213>  unknown

<220>
<223>  Pseudin 1 Rana Frog

<400>  77

Gly Leu Asn Thr Leu Lys Lys Val Phe Gln Gly Leu His Glu Ala Ile
1               5                   10                  15

Lys Leu Ile Asn Asn His Val Gln
            20

<210>  78
<211>  18
<212>  PRT
<213>  unknown

<220>
<223>  Ranalexin Frog

<400>  78

Phe Leu Gly Gly Leu Ile Val Pro Ala Met Ile Cys Ala Val Thr Lys
1               5                   10                  15

Lys Cys

<210>  79
<211>  26
<212>  PRT
<213>  unknown

<220>
<223>  Melittin bee

<400>  79

Gly Ile Gly Ala Val Leu Lys Val Leu Thr Thr Gly Leu Pro Ala Leu
1               5                   10                  15

Ile Ser Trp Ile Lys Arg Lys Arg Gln Gln
            20                  25

<210>  80
<211>  25

<212>  PRT
<213>  unknown

<220>
<223>  Lycotoxin 1 Spider

<400>  80

Ile Trp Leu Thr Ala Leu Lys Phe Leu Gly Lys His Ala Ala Lys Lys
1               5                   10                  15

Leu Ala Lys Gln Gln Leu Ser Lys Leu
            20                  25

<210>  81
<211>  19
<212>  PRT
<213>  unknown

<220>
<223>  Parasin 1 Fish

<400>  81

Lys Gly Arg Gly Lys Gln Gly Gly Lys Val Arg Ala Lys Ala Lys Thr
1               5                   10                  15

Arg Ser Ser

<210>  82
<211>  39
<212>  PRT
<213>  unknown

<220>
<223>  Buforin I Toad

<400>  82

Ala Gly Arg Gly Lys Gln Gly Gly Lys Val Arg Ala Lys Ala Lys Thr
1               5                   10                  15

Arg Ser Ser Arg Ala Gly Leu Gln Phe Pro Val Gly Arg Val His Arg
            20                  25                  30

Leu Leu Arg Lys Gly Asn Tyr
        35

<210>  83
<211>  34
<212>  PRT
<213>  unknown

<220>
<223>  Dermaseptin 1 Frog

<400>  83

Ala Leu Trp Lys Thr Met Leu Lys Lys Leu Gly Thr Met Ala Leu His
1               5                   10                  15

Ala Gly Lys Ala Ala Leu Gly Ala Ala Ala Asp Thr Ile Ser Gln Gly
            20                  25                  30

Thr Gln

<210>  84
<211>  12
<212>  PRT
<213>  unknown

<220>
<223>  Bactenecin 1 Cow

<400>  84

Arg Leu Cys Arg Ile Val Val Ile Arg Val Cys Arg
1               5                   10

<210>  85
<211>  21
<212>  PRT
<213>  unknown

<220>
<223>  Thanatin Insect

<400>  85

Gly Ser Lys Lys Pro Val Pro Ile Ile Tyr Cys Asn Arg Arg Thr Gly
1               5                   10                  15

Lys Cys Gln Arg Met
            20

<210>  86
<211>  19
<212>  PRT
<213>  unknown

<220>
<223>  Brevinin 1T Rana frogs

<400>  86

Val Asn Pro Ile Ile Leu Gly Val Leu Pro Lys Val Cys Leu Ile Thr
1               5                   10                  15

Lys Lys Cys

<210>  87
<211>  26
<212>  PRT
<213>  unknown

<220>
<223>  Ranateurin 1 Rana frog

<400>  87

Ser Met Leu Ser Val Leu Lys Asn Leu Gly Lys Val Gly Leu Gly Phe
1               5                   10                  15

Val Ala Cys Lys Ile Asn Ile Lys Gln Cys
            20                  25

<210>  88
<211>  46
<212>  PRT
<213>  unknown

<220>
<223>  Esculentin 1 Rana frogs

<400>  88

Gly Ile Phe Ser Lys Leu Gly Arg Lys Lys Ile Lys Asn Leu Leu Ile
1               5                   10                  15

Ser Gly Leu Lys Asn Val Gly Lys Glu Val Gly Met Asp Val Val Arg
            20                  25                  30

Thr Gly Ile Lys Ile Ala Gly Cys Lys Ile Lys Gly Glu Cys
        35                  40                  45

<210>  89
<211>  17
<212>  PRT
<213>  Limulus polyphemus

<400>  89

Arg Trp Cys Phe Arg Val Cys Tyr Arg Gly Ile Cys Tyr Arg Lys Cys
1               5                   10                  15

Arg

<210>  90
<211>  25
<212>  PRT
<213>  unknown

<220>

<223>  Androctonin Scorpion

<400>  90

Arg Ser Val Cys Arg Gln Ile Lys Ile Cys Arg Arg Arg Gly Gly Cys
1               5                   10                  15

Tyr Tyr Lys Cys Thr Asn Arg Pro Tyr
            20                  25

<210>  91
<211>  30
<212>  PRT
<213>  Homo sapiens

<400>  91

Asp Cys Tyr Cys Arg Ile Pro Ala Cys Ile Ala Gly Glu Arg Arg Tyr
1               5                   10                  15

Gly Thr Cys Ile Tyr Gln Gly Arg Leu Trp Ala Phe Cys Cys
            20                  25                  30

<210>  92
<211>  38
<212>  PRT
<213>  unknown

<220>
<223>  beta-defensin cow

<400>  92

Asn Pro Val Ser Cys Val Arg Asn Lys Gly Ile Cys Val Pro Ile Arg
1               5                   10                  15

Cys Pro Gly Ser Met Lys Gln Ile Gly Thr Cys Val Gly Arg Ala Val
            20                  25                  30

Lys Cys Cys Arg Lys Lys
        35

<210>  93
<211>  18
<212>  PRT
<213>  unknown

<220>
<223>  theta-defensin monkey

<400>  93

Gly Phe Cys Arg Cys Leu Cys Arg Arg Gly Val Cys Arg Cys Ile Cys
1               5                   10                  15

Thr Arg

<210> 94
<211> 40
<212> PRT
<213> unknown

<220>
<223> defensin (sapecin A) insect

<400> 94

Ala Thr Cys Asp Leu Leu Ser Gly Thr Gly Ile Asn His Ser Ala Cys
1               5                   10                  15

Ala Ala His Cys Leu Leu Arg Gly Asn Arg Gly Gly Tyr Cys Asn Gly
            20                  25                  30

Lys Ala Val Cys Val Cys Arg Asn
        35                  40

<210> 95
<211> 46
<212> PRT
<213> unknown

<220>
<223> Thionin (crambin) plant

<400> 95

Thr Thr Cys Cys Pro Ser Ile Val Ala Arg Ser Asn Phe Asn Val Cys
1               5                   10                  15

Arg Ile Pro Gly Thr Pro Glu Ala Ile Cys Ala Thr Tyr Thr Gly Cys
            20                  25                  30

Ile Ile Ile Pro Gly Ala Thr Cys Pro Gly Asp Tyr Ala Asn
        35                  40                  45

<210> 96
<211> 50
<212> PRT
<213> unknown

<220>
<223> defensin from radish

<400> 96

Gln Lys Leu Cys Gln Arg Pro Ser Gly Thr Trp Ser Gly Val Cys Gly
1               5                   10                  15

Asn Asn Asn Ala Cys Lys Asn Gln Cys Ile Arg Leu Glu Lys Ala Arg

20                  25                  30

His Gly Ser Cys Asn Tyr Val Phe Pro Ala His Cys Ile Cys Tyr Phe
        35                  40                  45

Pro Cys
    50

<210>  97
<211>  44
<212>  PRT
<213>  Drosophila melanogaster

<400>  97

Asp Cys Leu Ser Gly Arg Tyr Lys Gly Pro Cys Ala Val Trp Asp Asn
1               5                   10                  15

Glu Thr Cys Arg Arg Val Cys Lys Glu Glu Gly Arg Ser Ser Gly His
            20                  25                  30

Cys Ser Pro Ser Leu Lys Cys Trp Cys Glu Gly Cys
        35                  40

<210>  98
<211>  25
<212>  PRT
<213>  Homo sapiens

<400>  98

Asp Thr His Phe Pro Ile Cys Ile Phe Cys Cys Gly Cys Cys His Arg
1               5                   10                  15

Ser Lys Cys Gly Met Cys Cys Lys Thr
            20                  25

<210>  99
<211>  44
<212>  PRT
<213>  unknown

<220>
<223>  Bac 5 Cow

<400>  99

Arg Phe Arg Pro Pro Ile Arg Arg Pro Pro Ile Arg Pro Pro Phe Tyr
1               5                   10                  15

Pro Pro Phe Arg Pro Pro Ile Arg Pro Pro Ile Phe Pro Pro Ile Arg
            20                  25                  30

Pro Pro Phe Arg Pro Pro Leu Gly Arg Pro Phe Pro
        35                  40

<210>  100
<211>  39
<212>  PRT
<213>  unknown

<220>
<223>  PR-39 Pig

<400>  100

Arg Arg Arg Pro Arg Pro Pro Tyr Leu Pro Arg Pro Arg Pro Pro Pro
1               5                   10                  15

Phe Phe Pro Pro Arg Leu Pro Pro Arg Ile Pro Pro Gly Phe Pro Pro
            20                  25                  30

Arg Phe Pro Pro Arg Phe Pro
        35

<210>  101
<211>  20
<212>  PRT
<213>  unknown

<220>
<223>  Pyrrhocoricin Insect

<400>  101

Val Asp Lys Gly Ser Tyr Leu Pro Arg Pro Thr Pro Pro Arg Pro Ile
1               5                   10                  15

Tyr Asn Arg Asn
            20

<210>  102
<211>  24
<212>  PRT
<213>  Homo sapiens

<400>  102

Asp Ser His Ala Lys Arg His His Gly Tyr Lys Arg Lys Phe His Glu
1               5                   10                  15

Lys His His Ser His Arg Gly Tyr
            20

<210>  103
<211>  19
<212>  PRT
<213>  Unknown

<220>
<223>  ECP19

<400>  103

Arg Pro Pro Gln Phe Thr Arg Ala Gln Trp Phe Ala Ile Gln His Ile
1               5                   10                  15

Ser Leu Asn

<210>  104
<211>  23
<212>  PRT
<213>  Unknown

<220>
<223>  MSI-594

<400>  104

Gly Ile Gly Lys Phe Leu Lys Lys Ala Lys Lys Gly Ile Gly Ala Val
1               5                   10                  15

Leu Lys Val Leu Thr Thr Gly
            20

<210>  105
<211>  35
<212>  PRT
<213>  Unknown

<220>
<223>  TL-ColM

<400>  105

Met Glu Thr Leu Thr Val His Ala Pro Ser Pro Ser Thr Asn Leu Pro
1               5                   10                  15

Ser Tyr Gly Asn Gly Ala Phe Ser Leu Ser Ala Pro His Val Pro Gly
            20                  25                  30

Ala Gly Pro
        35

<210>  106
<211>  18
<212>  PRT
<213>  Unknown

<220>
<223>  SBO

<400>  106

Lys Leu Lys Lys Ile Ala Gln Lys Ile Lys Asn Phe Phe Ala Lys Leu
1               5                   10                  15

Val Ala

<210>  107
<211>  26
<212>  PRT
<213>  unknown

<220>
<223>  Macedocin

<400>  107

Gly Lys Asn Gly Val Phe Lys Thr Ile Ser His Glu Cys His Leu Asn
1               5                   10                  15

Thr Trp Ala Phe Leu Ala Thr Cys Cys Ser
            20                  25

<210>  108
<211>  22
<212>  PRT
<213>  unknown

<220>
<223>  Macedocin (Trunc)

<400>  108

Gly Lys Asn Gly Val Phe Lys Thr Ile Ser His Glu Cys His Leu Asn
1               5                   10                  15

Thr Trp Ala Phe Leu Ala
            20

<210>  109
<211>  28
<212>  PRT
<213>  unknown

<220>
<223>  D16

<400>  109

Ala Cys Lys Leu Lys Ser Leu Leu Lys Thr Leu Ser Lys Ala Lys Lys
1               5                   10                  15

Lys Lys Leu Lys Thr Leu Leu Lys Ala Leu Ser Lys
            20                  25

<210>  110
<211>  17
<212>  PRT
<213>  unknown

<220>
<223>  CPF-C1

<400>  110

Gly Phe Gly Ser Leu Leu Gly Lys Ala Leu Arg Leu Gly Ala Asn Val
1               5                   10                  15

Leu

<210>  111
<211>  34
<212>  PRT
<213>  unknown

<220>
<223>  TL-ColM

<400>  111

Glu Thr Leu Thr Val His Ala Pro Ser Pro Ser Thr Asn Leu Pro Ser
1               5                   10                  15

Tyr Gly Asn Gly Ala Phe Ser Leu Ser Ala Pro His Val Pro Gly Ala
            20                  25                  30

Gly Pro

<210>  112
<211>  26
<212>  PRT
<213>  unknown

<220>
<223>  TM-174E

<400>  112

Leu Ile Ser Lys Gly Trp Pro Tyr Leu Leu Val Val Val Leu Gly Ala
1               5                   10                  15

Thr Ile Tyr Phe Trp Gly Asn Ser Asn Gly
            20                  25

<210>  113
<211>  45
<212>  PRT
<213>  unknown

<220>
<223>  ECP45

<400>  113

Arg Pro Pro Gln Phe Thr Arg Ala Gln Trp Phe Ala Ile Gln His Ile
1               5                   10                  15

Ser Leu Asn Pro Pro Arg Cys Thr Ile Ala Met Arg Ala Ile Asn Asn
            20                  25                  30

Tyr Arg Trp Arg Cys Lys Asn Gln Asn Thr Phe Leu Arg
        35                  40                  45

<210>  114
<211>  50
<212>  PRT
<213>  unknown

<220>
<223>  ColicinE3_1-51 (S37F)

<400>  114

Ser Gly Gly Asp Gly Arg Gly His Asn Thr Gly Ala His Ser Thr Ser
1               5                   10                  15

Gly Asn Ile Asn Gly Gly Pro Thr Gly Leu Gly Val Gly Gly Gly Ala
            20                  25                  30

Ser Asp Gly Phe Gly Trp Ser Ser Glu Asn Asn Pro Trp Gly Gly Gly
        35                  40                  45

Ser Gly
    50

<210>  115
<211>  68
<212>  PRT
<213>  unknown

<220>
<223>  ColicinE3_1-69 (S37F)

<400>  115

Ser Gly Gly Asp Gly Arg Gly His Asn Thr Gly Ala His Ser Thr Ser
1               5                   10                  15

Gly Asn Ile Asn Gly Gly Pro Thr Gly Leu Gly Val Gly Gly Gly Ala
            20                  25                  30

Ser Asp Gly Phe Gly Trp Ser Ser Glu Asn Asn Pro Trp Gly Gly Gly
        35                  40                  45

Ser Gly Ser Gly Ile His Trp Gly Gly Gly Ser Gly His Gly Asn Gly
    50                  55                  60

Gly Gly Asn Gly
65

<210>  116
<211>  52
<212>  PRT
<213>  unknown

<220>
<223>  ColicinD_1-53

<400>  116

Ser Asp Tyr Glu Gly Ser Gly Pro Thr Glu Gly Ile Asp Tyr Gly His
1               5                   10                  15

Ser Met Val Val Trp Pro Ser Thr Gly Leu Ile Ser Gly Gly Asp Val
            20                  25                  30

Lys Pro Gly Gly Ser Ser Gly Ile Ala Pro Ser Met Pro Pro Gly Trp
        35                  40                  45

Gly Asp Tyr Ser
    50

<210>  117
<211>  34
<212>  PRT
<213>  Limulus polyphemus

<400>  117

Gly Phe Lys Leu Lys Gly Met Ala Arg Ile Ser Cys Leu Pro Asn Gly
1               5                   10                  15

Gln Trp Ser Asn Phe Pro Pro Lys Cys Ile Arg Glu Cys Ala Met Val
            20                  25                  30

Ser Ser

<210>  118
<211>  18
<212>  PRT
<213>  artificial

<220>
<223>  synthetic sequence

<400>  118

Gly Phe Phe Ile Pro Ala Val Ile Leu Pro Ser Ile Ala Phe Leu Ile
1               5                   10                  15

Val Pro

<210>  119
<211>  5
<212>  PRT
<213>  artificial

<220>
<223>  synthetic sequence

<400>  119

Phe Phe Val Ala Pro
1               5

<210>  120
<211>  13
<212>  PRT
<213>  unknown

<220>
<223>  alpha4-helix of T4 lysozyme

<400>  120

Pro Asn Arg Ala Lys Arg Val Ile Thr Thr Phe Arg Thr
1               5                   10

<210>  121
<211>  27
<212>  PRT
<213>  artificial

<220>
<223>  synthetic sequence

<400>  121

Lys Arg Trp Val Lys Arg Val Lys Arg Val Lys Arg Trp Val Lys Arg
1               5                   10                  15

Val Val Arg Val Val Lys Arg Trp Val Lys Arg
            20                  25

<210>  122
<211>  25
<212>  PRT
<213>  Artificial Sequence

<220>
<223>  synthetic sequence; MW2

<400>  122

Gly Lys Pro Gly Trp Leu Ile Lys Val Ala Leu Lys Phe Lys Lys Leu
1               5                   10                  15

Ile Arg Arg Pro Leu Lys Arg Leu Ala
            20                  25

<210>  123
<211>  5
<212>  PRT
<213>  Artificial

<220>
<223>  Linker sequence

<400>  123

Gly Gly Gly Gly Ser
1               5

<210>  124
<211>  6
<212>  PRT
<213>  artificial sequence

<220>
<223>  His-Tag (6x)

<400>  124

His His His His His His
1               5

<210>  125
<211>  166
<212>  PRT
<213>  Artificial sequence

<220>
<223>  Fusion MSI-78-GGGGS-S394 w/o methionine

<400>  125

Gly Ile Gly Lys Phe Leu Lys Lys Ala Lys Lys Phe Gly Lys Ala Phe
1               5                   10                  15

Val Lys Ile Leu Lys Lys Gly Gly Gly Gly Ser Gly Ser Met Ser Phe
            20                  25                  30

Lys Phe Gly Lys Asn Ser Glu Lys Gln Leu Ala Thr Val Lys Pro Glu
        35                  40                  45

Leu Gln Lys Val Ala Arg Arg Ala Leu Glu Leu Ser Pro Tyr Asp Phe
    50                  55                  60

Thr Ile Val Gln Gly Ile Arg Thr Val Ala Gln Ser Ala Gln Asn Ile
65                  70                  75                  80

Ala Asn Gly Thr Ser Phe Leu Lys Asp Pro Ser Lys Ser Lys His Val
                85                  90                  95

Thr Gly Asp Ala Ile Asp Phe Ala Pro Tyr Ile Asn Gly Lys Ile Asp
            100                 105                 110

Trp Lys Asp Leu Glu Ala Phe Trp Ala Val Lys Lys Ala Phe Glu Gln
        115                 120                 125

Ala Gly Lys Glu Leu Gly Ile Lys Leu Arg Phe Gly Ala Asp Trp Asn
    130                 135                 140

Ser Ser Gly Asp Tyr His Asp Glu Ile Asp Arg Gly Thr Tyr Asp Gly
145                 150                 155                 160

Gly His Val Glu Leu Val
                165

<210>  126
<211>  167
<212>  PRT
<213>  Artificial sequence

<220>
<223>  Fusion MSI-78-GGGGS-S394

<400>  126

Met Gly Ile Gly Lys Phe Leu Lys Lys Ala Lys Lys Phe Gly Lys Ala
1               5                   10                  15

Phe Val Lys Ile Leu Lys Lys Gly Gly Gly Gly Ser Gly Ser Met Ser
            20                  25                  30

Phe Lys Phe Gly Lys Asn Ser Glu Lys Gln Leu Ala Thr Val Lys Pro
        35                  40                  45

Glu Leu Gln Lys Val Ala Arg Arg Ala Leu Glu Leu Ser Pro Tyr Asp
    50                  55                  60

Phe Thr Ile Val Gln Gly Ile Arg Thr Val Ala Gln Ser Ala Gln Asn
65                  70                  75                  80

Ile Ala Asn Gly Thr Ser Phe Leu Lys Asp Pro Ser Lys Ser Lys His
                85                  90                  95

Val Thr Gly Asp Ala Ile Asp Phe Ala Pro Tyr Ile Asn Gly Lys Ile
            100                 105                 110

Asp Trp Lys Asp Leu Glu Ala Phe Trp Ala Val Lys Lys Ala Phe Glu
        115                 120                 125

Gln Ala Gly Lys Glu Leu Gly Ile Lys Leu Arg Phe Gly Ala Asp Trp
    130                 135                 140

Asn Ser Ser Gly Asp Tyr His Asp Glu Ile Asp Arg Gly Thr Tyr Asp
145                 150                 155                 160

Gly Gly His Val Glu Leu Val
                165

<210>  127
<211>  175
<212>  PRT
<213>  Artificial sequence

<220>
<223>  Fusion MSI-78-GGGGS-S394-His6

<400>  127

Met Gly Ile Gly Lys Phe Leu Lys Lys Ala Lys Lys Phe Gly Lys Ala
1               5                   10                  15

Phe Val Lys Ile Leu Lys Lys Gly Gly Gly Gly Ser Gly Ser Met Ser
            20                  25                  30

Phe Lys Phe Gly Lys Asn Ser Glu Lys Gln Leu Ala Thr Val Lys Pro
        35                  40                  45

Glu Leu Gln Lys Val Ala Arg Arg Ala Leu Glu Leu Ser Pro Tyr Asp
    50                  55                  60

Phe Thr Ile Val Gln Gly Ile Arg Thr Val Ala Gln Ser Ala Gln Asn
65                  70                  75                  80

Ile Ala Asn Gly Thr Ser Phe Leu Lys Asp Pro Ser Lys Ser Lys His
                85                  90                  95

Val Thr Gly Asp Ala Ile Asp Phe Ala Pro Tyr Ile Asn Gly Lys Ile
            100                 105                 110

Asp Trp Lys Asp Leu Glu Ala Phe Trp Ala Val Lys Lys Ala Phe Glu
        115                 120                 125

Gln Ala Gly Lys Glu Leu Gly Ile Lys Leu Arg Phe Gly Ala Asp Trp
    130                 135                 140

Asn Ser Ser Gly Asp Tyr His Asp Glu Ile Asp Arg Gly Thr Tyr Asp
145                 150                 155                 160

Gly Gly His Val Glu Leu Val Leu Glu His His His His His His
                165                 170                 175

<210>  128
<211>  291
<212>  PRT
<213>  Artificial Sequence

<220>
<223>  Fusion Cathelicidin-BF-KZ144(C14S, C23S, C50S) w/o methionine

<400>  128

Lys Phe Phe Arg Lys Leu Lys Lys Ser Val Lys Lys Arg Ala Lys Glu
1               5                   10                  15

Phe Phe Lys Lys Pro Arg Val Ile Gly Val Ser Ile Pro Phe Gly Ser
            20                  25                  30

Lys Val Leu Arg Lys Gly Asp Arg Gly Asp Glu Val Ser Gln Leu Gln
        35                  40                  45

Thr Leu Leu Asn Leu Ser Gly Tyr Asp Val Gly Lys Pro Asp Gly Ile
    50                  55                  60

Phe Gly Asn Asn Thr Phe Asn Gln Val Val Lys Phe Gln Lys Asp Asn
65                  70                  75                  80

Ser Leu Asp Ser Asp Gly Ile Val Gly Lys Asn Thr Trp Ala Glu Leu
                85                  90                  95

Phe Ser Lys Tyr Ser Pro Pro Ile Pro Tyr Lys Thr Ile Pro Met Pro
            100                 105                 110

Thr Ala Asn Lys Ser Arg Ala Ala Ala Thr Pro Val Met Asn Ala Val
        115                 120                 125

Glu Asn Ala Thr Gly Val Arg Ser Gln Leu Leu Leu Thr Phe Ala Ser
    130                 135                 140

Ile Glu Ser Ala Phe Asp Tyr Glu Ile Lys Ala Lys Thr Ser Ser Ala
145                 150                 155                 160

Thr Gly Trp Phe Gln Phe Leu Thr Gly Thr Trp Lys Thr Met Ile Glu

165                 170                 175

Asn Tyr Gly Met Lys Tyr Gly Val Leu Thr Asp Pro Thr Gly Ala Leu
            180                 185                 190

Arg Lys Asp Pro Arg Ile Ser Ala Leu Met Gly Ala Glu Leu Ile Lys
        195                 200                 205

Glu Asn Met Asn Ile Leu Arg Pro Val Leu Lys Arg Glu Pro Thr Asp
    210                 215                 220

Thr Asp Leu Tyr Leu Ala His Phe Phe Gly Pro Gly Ala Ala Arg Arg
225                 230                 235                 240

Phe Leu Thr Thr Gly Gln Asn Glu Leu Ala Ala Thr His Phe Pro Lys
                245                 250                 255

Glu Ala Gln Ala Asn Pro Ser Ile Phe Tyr Asn Lys Asp Gly Ser Pro
            260                 265                 270

Lys Thr Ile Gln Glu Val Tyr Asn Leu Met Asp Gly Lys Val Ala Ala
        275                 280                 285

His Arg Lys
    290

<210>  129
<211>  292
<212>  PRT
<213>  Artificial Sequence

<220>
<223>  Fusion Cathelicidin-BF-KZ144(C14S, C23S, C50S)

<400>  129

Met Lys Phe Phe Arg Lys Leu Lys Lys Ser Val Lys Lys Arg Ala Lys
1               5                   10                  15

Glu Phe Phe Lys Lys Pro Arg Val Ile Gly Val Ser Ile Pro Phe Gly
            20                  25                  30

Ser Lys Val Leu Arg Lys Gly Asp Arg Gly Asp Glu Val Ser Gln Leu
        35                  40                  45

Gln Thr Leu Leu Asn Leu Ser Gly Tyr Asp Val Gly Lys Pro Asp Gly
    50                  55                  60

Ile Phe Gly Asn Asn Thr Phe Asn Gln Val Val Lys Phe Gln Lys Asp
65                  70                  75                  80

Asn Ser Leu Asp Ser Asp Gly Ile Val Gly Lys Asn Thr Trp Ala Glu
                85                  90                  95

Leu Phe Ser Lys Tyr Ser Pro Pro Ile Pro Tyr Lys Thr Ile Pro Met
            100                 105                 110

Pro Thr Ala Asn Lys Ser Arg Ala Ala Ala Thr Pro Val Met Asn Ala
        115                 120                 125

Val Glu Asn Ala Thr Gly Val Arg Ser Gln Leu Leu Leu Thr Phe Ala
    130                 135                 140

Ser Ile Glu Ser Ala Phe Asp Tyr Glu Ile Lys Ala Lys Thr Ser Ser
145                 150                 155                 160

Ala Thr Gly Trp Phe Gln Phe Leu Thr Gly Thr Trp Lys Thr Met Ile
                165                 170                 175

Glu Asn Tyr Gly Met Lys Tyr Gly Val Leu Thr Asp Pro Thr Gly Ala
            180                 185                 190

Leu Arg Lys Asp Pro Arg Ile Ser Ala Leu Met Gly Ala Glu Leu Ile
        195                 200                 205

Lys Glu Asn Met Asn Ile Leu Arg Pro Val Leu Lys Arg Glu Pro Thr
    210                 215                 220

Asp Thr Asp Leu Tyr Leu Ala His Phe Phe Gly Pro Gly Ala Ala Arg
225                 230                 235                 240

Arg Phe Leu Thr Thr Gly Gln Asn Glu Leu Ala Ala Thr His Phe Pro
                245                 250                 255

Lys Glu Ala Gln Ala Asn Pro Ser Ile Phe Tyr Asn Lys Asp Gly Ser
            260                 265                 270

Pro Lys Thr Ile Gln Glu Val Tyr Asn Leu Met Asp Gly Lys Val Ala
        275                 280                 285

Ala His Arg Lys
    290

<210>  130
<211>  300
<212>  PRT
<213>  Artificial Sequence

<220>
<223>  Fusion Cathelicidin-BF-KZ144(C14S, C23S, C50S)-His6

<400>  130

Met Lys Phe Phe Arg Lys Leu Lys Lys Ser Val Lys Lys Arg Ala Lys
1               5                   10                  15

Glu Phe Phe Lys Lys Pro Arg Val Ile Gly Val Ser Ile Pro Phe Gly
            20                  25                  30

Ser Lys Val Leu Arg Lys Gly Asp Arg Gly Asp Glu Val Ser Gln Leu
        35                  40                  45

Gln Thr Leu Leu Asn Leu Ser Gly Tyr Asp Val Gly Lys Pro Asp Gly
    50                  55                  60

Ile Phe Gly Asn Asn Thr Phe Asn Gln Val Val Lys Phe Gln Lys Asp
65                  70                  75                  80

Asn Ser Leu Asp Ser Asp Gly Ile Val Gly Lys Asn Thr Trp Ala Glu
                85                  90                  95

Leu Phe Ser Lys Tyr Ser Pro Pro Ile Pro Tyr Lys Thr Ile Pro Met
            100                 105                 110

Pro Thr Ala Asn Lys Ser Arg Ala Ala Ala Thr Pro Val Met Asn Ala
        115                 120                 125

Val Glu Asn Ala Thr Gly Val Arg Ser Gln Leu Leu Leu Thr Phe Ala
    130                 135                 140

Ser Ile Glu Ser Ala Phe Asp Tyr Glu Ile Lys Ala Lys Thr Ser Ser
145                 150                 155                 160

Ala Thr Gly Trp Phe Gln Phe Leu Thr Gly Thr Trp Lys Thr Met Ile
                165                 170                 175

Glu Asn Tyr Gly Met Lys Tyr Gly Val Leu Thr Asp Pro Thr Gly Ala
            180                 185                 190

Leu Arg Lys Asp Pro Arg Ile Ser Ala Leu Met Gly Ala Glu Leu Ile
        195                 200                 205

Lys Glu Asn Met Asn Ile Leu Arg Pro Val Leu Lys Arg Glu Pro Thr
    210                 215                 220

Asp Thr Asp Leu Tyr Leu Ala His Phe Phe Gly Pro Gly Ala Ala Arg
225                 230                 235                 240

Arg Phe Leu Thr Thr Gly Gln Asn Glu Leu Ala Ala Thr His Phe Pro
                245                 250                 255

Lys Glu Ala Gln Ala Asn Pro Ser Ile Phe Tyr Asn Lys Asp Gly Ser
            260                 265                 270

Pro Lys Thr Ile Gln Glu Val Tyr Asn Leu Met Asp Gly Lys Val Ala
        275                 280                 285

Ala His Arg Lys Leu Glu His His His His His His
    290                 295                 300

<210>  131
<211>  169
<212>  PRT
<213>  Artificial Sequence

<220>
<223>  Fusion Cathelicidin-BF-S394 w/o methionine

<400>  131

Lys Phe Phe Arg Lys Leu Lys Lys Ser Val Lys Lys Arg Ala Lys Glu
1               5                   10                  15

Phe Phe Lys Lys Pro Arg Val Ile Gly Val Ser Ile Pro Phe Gly Ser
            20                  25                  30

Met Ser Phe Lys Phe Gly Lys Asn Ser Glu Lys Gln Leu Ala Thr Val
        35                  40                  45

Lys Pro Glu Leu Gln Lys Val Ala Arg Arg Ala Leu Glu Leu Ser Pro
    50                  55                  60

Tyr Asp Phe Thr Ile Val Gln Gly Ile Arg Thr Val Ala Gln Ser Ala
65                  70                  75                  80

Gln Asn Ile Ala Asn Gly Thr Ser Phe Leu Lys Asp Pro Ser Lys Ser
                85                  90                  95

Lys His Val Thr Gly Asp Ala Ile Asp Phe Ala Pro Tyr Ile Asn Gly
            100                 105                 110

Lys Ile Asp Trp Lys Asp Leu Glu Ala Phe Trp Ala Val Lys Lys Ala
        115                 120                 125

Phe Glu Gln Ala Gly Lys Glu Leu Gly Ile Lys Leu Arg Phe Gly Ala
    130                 135                 140

Asp Trp Asn Ser Ser Gly Asp Tyr His Asp Glu Ile Asp Arg Gly Thr
145                 150                 155                 160

Tyr Asp Gly Gly His Val Glu Leu Val
                165

<210>  132
<211>  170
<212>  PRT
<213>  Artificial Sequence

<220>
<223>  Fusion Cathelicidin-BF-S394

<400>  132

Met Lys Phe Phe Arg Lys Leu Lys Lys Ser Val Lys Lys Arg Ala Lys
1               5                   10                  15

Glu Phe Phe Lys Lys Pro Arg Val Ile Gly Val Ser Ile Pro Phe Gly
            20                  25                  30

Ser Met Ser Phe Lys Phe Gly Lys Asn Ser Glu Lys Gln Leu Ala Thr
        35                  40                  45

Val Lys Pro Glu Leu Gln Lys Val Ala Arg Arg Ala Leu Glu Leu Ser
    50                  55                  60

Pro Tyr Asp Phe Thr Ile Val Gln Gly Ile Arg Thr Val Ala Gln Ser
65                  70                  75                  80

Ala Gln Asn Ile Ala Asn Gly Thr Ser Phe Leu Lys Asp Pro Ser Lys
                85                  90                  95

Ser Lys His Val Thr Gly Asp Ala Ile Asp Phe Ala Pro Tyr Ile Asn
            100                 105                 110

Gly Lys Ile Asp Trp Lys Asp Leu Glu Ala Phe Trp Ala Val Lys Lys
        115                 120                 125

Ala Phe Glu Gln Ala Gly Lys Glu Leu Gly Ile Lys Leu Arg Phe Gly
    130                 135                 140

Ala Asp Trp Asn Ser Ser Gly Asp Tyr His Asp Glu Ile Asp Arg Gly
145                 150                 155                 160

Thr Tyr Asp Gly Gly His Val Glu Leu Val
                165                 170

<210>  133
<211>  178
<212>  PRT
<213>  Artificial Sequence

<220>
<223>  Fusion Cathelicidin-BF-S394-His6

<400>  133

Met Lys Phe Phe Arg Lys Leu Lys Lys Ser Val Lys Lys Arg Ala Lys
1               5                   10                  15

Glu Phe Phe Lys Lys Pro Arg Val Ile Gly Val Ser Ile Pro Phe Gly
            20                  25                  30

Ser Met Ser Phe Lys Phe Gly Lys Asn Ser Glu Lys Gln Leu Ala Thr
        35                  40                  45

Val Lys Pro Glu Leu Gln Lys Val Ala Arg Arg Ala Leu Glu Leu Ser
    50                  55                  60

Pro Tyr Asp Phe Thr Ile Val Gln Gly Ile Arg Thr Val Ala Gln Ser
65                  70                  75                  80

Ala Gln Asn Ile Ala Asn Gly Thr Ser Phe Leu Lys Asp Pro Ser Lys
                85                  90                  95

Ser Lys His Val Thr Gly Asp Ala Ile Asp Phe Ala Pro Tyr Ile Asn
            100                 105                 110

Gly Lys Ile Asp Trp Lys Asp Leu Glu Ala Phe Trp Ala Val Lys Lys
        115                 120                 125

Ala Phe Glu Gln Ala Gly Lys Glu Leu Gly Ile Lys Leu Arg Phe Gly
    130                 135                 140

Ala Asp Trp Asn Ser Ser Gly Asp Tyr His Asp Glu Ile Asp Arg Gly
145                 150                 155                 160

Thr Tyr Asp Gly Gly His Val Glu Leu Val Leu Glu His His His His
                165                 170                 175

His His

<210>  134
<211>  288
<212>  PRT
<213>  Artificial Sequence

<220>
<223>  Fusion MSI-78--KZ144(C14S, C23S, C50S) w/o methionine

<400>  134

Gly Ile Gly Lys Phe Leu Lys Lys Ala Lys Lys Phe Gly Lys Ala Phe
1               5                   10                  15

Val Lys Ile Leu Lys Lys Gly Gly Gly Gly Ser Gly Ser Lys Val Leu
            20                  25                  30

Arg Lys Gly Asp Arg Gly Asp Glu Val Ser Gln Leu Gln Thr Leu Leu
        35                  40                  45

Asn Leu Ser Gly Tyr Asp Val Gly Lys Pro Asp Gly Ile Phe Gly Asn
    50                  55                  60

Asn Thr Phe Asn Gln Val Val Lys Phe Gln Lys Asp Asn Ser Leu Asp
65                  70                  75                  80

Ser Asp Gly Ile Val Gly Lys Asn Thr Trp Ala Glu Leu Phe Ser Lys
                85                  90                  95

Tyr Ser Pro Pro Ile Pro Tyr Lys Thr Ile Pro Met Pro Thr Ala Asn
            100                 105                 110

Lys Ser Arg Ala Ala Ala Thr Pro Val Met Asn Ala Val Glu Asn Ala
        115                 120                 125

Thr Gly Val Arg Ser Gln Leu Leu Leu Thr Phe Ala Ser Ile Glu Ser
    130                 135                 140

Ala Phe Asp Tyr Glu Ile Lys Ala Lys Thr Ser Ser Ala Thr Gly Trp
145                 150                 155                 160

Phe Gln Phe Leu Thr Gly Thr Trp Lys Thr Met Ile Glu Asn Tyr Gly
                165                 170                 175

Met Lys Tyr Gly Val Leu Thr Asp Pro Thr Gly Ala Leu Arg Lys Asp
            180                 185                 190

Pro Arg Ile Ser Ala Leu Met Gly Ala Glu Leu Ile Lys Glu Asn Met
        195                 200                 205

Asn Ile Leu Arg Pro Val Leu Lys Arg Glu Pro Thr Asp Thr Asp Leu
    210                 215                 220

Tyr Leu Ala His Phe Phe Gly Pro Gly Ala Ala Arg Arg Phe Leu Thr
225                 230                 235                 240

Thr Gly Gln Asn Glu Leu Ala Ala Thr His Phe Pro Lys Glu Ala Gln
                245                 250                 255

Ala Asn Pro Ser Ile Phe Tyr Asn Lys Asp Gly Ser Pro Lys Thr Ile
            260                 265                 270

Gln Glu Val Tyr Asn Leu Met Asp Gly Lys Val Ala Ala His Arg Lys
        275                 280                 285

<210>  135
<211>  289
<212>  PRT
<213>  Artificial Sequence

<220>
<223>  Fusion MSI-78--KZ144(C14S, C23S, C50S)

<400>  135

Met Gly Ile Gly Lys Phe Leu Lys Lys Ala Lys Lys Phe Gly Lys Ala
1               5                   10                  15

Phe Val Lys Ile Leu Lys Lys Gly Gly Gly Gly Ser Gly Ser Lys Val
            20                  25                  30

Leu Arg Lys Gly Asp Arg Gly Asp Glu Val Ser Gln Leu Gln Thr Leu
        35                  40                  45

Leu Asn Leu Ser Gly Tyr Asp Val Gly Lys Pro Asp Gly Ile Phe Gly
    50                  55                  60

Asn Asn Thr Phe Asn Gln Val Val Lys Phe Gln Lys Asp Asn Ser Leu
65                  70                  75                  80

Asp Ser Asp Gly Ile Val Gly Lys Asn Thr Trp Ala Glu Leu Phe Ser
                85                  90                  95

Lys Tyr Ser Pro Pro Ile Pro Tyr Lys Thr Ile Pro Met Pro Thr Ala
            100                 105                 110

Asn Lys Ser Arg Ala Ala Ala Thr Pro Val Met Asn Ala Val Glu Asn
        115                 120                 125

Ala Thr Gly Val Arg Ser Gln Leu Leu Leu Thr Phe Ala Ser Ile Glu
    130                 135                 140

Ser Ala Phe Asp Tyr Glu Ile Lys Ala Lys Thr Ser Ser Ala Thr Gly
145                 150                 155                 160

Trp Phe Gln Phe Leu Thr Gly Thr Trp Lys Thr Met Ile Glu Asn Tyr
                165                 170                 175

Gly Met Lys Tyr Gly Val Leu Thr Asp Pro Thr Gly Ala Leu Arg Lys
            180                 185                 190

Asp Pro Arg Ile Ser Ala Leu Met Gly Ala Glu Leu Ile Lys Glu Asn
        195                 200                 205

Met Asn Ile Leu Arg Pro Val Leu Lys Arg Glu Pro Thr Asp Thr Asp
    210                 215                 220

Leu Tyr Leu Ala His Phe Phe Gly Pro Gly Ala Ala Arg Arg Phe Leu
225                 230                 235                 240

Thr Thr Gly Gln Asn Glu Leu Ala Ala Thr His Phe Pro Lys Glu Ala
                245                 250                 255

Gln Ala Asn Pro Ser Ile Phe Tyr Asn Lys Asp Gly Ser Pro Lys Thr
            260                 265                 270

Ile Gln Glu Val Tyr Asn Leu Met Asp Gly Lys Val Ala Ala His Arg
        275                 280                 285

Lys

<210>  136
<211>  297
<212>  PRT
<213>  Artificial Sequence

<220>
<223>  Fusion MSI-78-KZ144(C14S, C23S, C50S)-His6

<400>  136

Met Gly Ile Gly Lys Phe Leu Lys Lys Ala Lys Lys Phe Gly Lys Ala
1               5                   10                  15

Phe Val Lys Ile Leu Lys Lys Gly Gly Gly Gly Ser Gly Ser Lys Val
            20                  25                  30

Leu Arg Lys Gly Asp Arg Gly Asp Glu Val Ser Gln Leu Gln Thr Leu
        35                  40                  45

Leu Asn Leu Ser Gly Tyr Asp Val Gly Lys Pro Asp Gly Ile Phe Gly
    50                  55                  60

Asn Asn Thr Phe Asn Gln Val Val Lys Phe Gln Lys Asp Asn Ser Leu
65                  70                  75                  80

Asp Ser Asp Gly Ile Val Gly Lys Asn Thr Trp Ala Glu Leu Phe Ser
                85                  90                  95

Lys Tyr Ser Pro Pro Ile Pro Tyr Lys Thr Ile Pro Met Pro Thr Ala
            100                 105                 110

Asn Lys Ser Arg Ala Ala Ala Thr Pro Val Met Asn Ala Val Glu Asn
        115                 120                 125

Ala Thr Gly Val Arg Ser Gln Leu Leu Leu Thr Phe Ala Ser Ile Glu
    130                 135                 140

Ser Ala Phe Asp Tyr Glu Ile Lys Ala Lys Thr Ser Ser Ala Thr Gly
145                 150                 155                 160

Trp Phe Gln Phe Leu Thr Gly Thr Trp Lys Thr Met Ile Glu Asn Tyr
                165                 170                 175

Gly Met Lys Tyr Gly Val Leu Thr Asp Pro Thr Gly Ala Leu Arg Lys
            180                 185                 190

Asp Pro Arg Ile Ser Ala Leu Met Gly Ala Glu Leu Ile Lys Glu Asn
        195                 200                 205

Met Asn Ile Leu Arg Pro Val Leu Lys Arg Glu Pro Thr Asp Thr Asp
    210                 215                 220

Leu Tyr Leu Ala His Phe Phe Gly Pro Gly Ala Ala Arg Arg Phe Leu
225                 230                 235                 240

Thr Thr Gly Gln Asn Glu Leu Ala Ala Thr His Phe Pro Lys Glu Ala
                245                 250                 255

Gln Ala Asn Pro Ser Ile Phe Tyr Asn Lys Asp Gly Ser Pro Lys Thr
            260                 265                 270

Ile Gln Glu Val Tyr Asn Leu Met Asp Gly Lys Val Ala Ala His Arg
        275                 280                 285

Lys Leu Glu His His His His His His
    290                 295

<210>  137
<211>  359
<212>  PRT

<213>  Artificial Sequence

<220>
<223>  Fusion Cathelicidin-BF-OBPgp279 w/o methionine

<400>  137

Lys Phe Phe Arg Lys Leu Lys Lys Ser Val Lys Lys Arg Ala Lys Glu
1               5                   10                  15

Phe Phe Lys Lys Pro Arg Val Ile Gly Val Ser Ile Pro Phe Gly Ser
            20                  25                  30

Lys Asn Ser Glu Lys Asn Ala Ser Ile Ile Met Ser Ile Gln Arg Thr
        35                  40                  45

Leu Ala Ser Leu Ser Leu Tyr Gly Gly Arg Ile Asp Gly Leu Phe Gly
    50                  55                  60

Glu Lys Cys Arg Gly Ala Ile Ile Leu Met Leu Asn Lys Val Tyr Pro
65                  70                  75                  80

Asn Phe Ser Thr Asn Lys Leu Pro Ser Asn Thr Tyr Glu Ala Glu Ser
                85                  90                  95

Val Phe Thr Phe Leu Gln Thr Ala Leu Ala Gly Val Gly Leu Tyr Thr
            100                 105                 110

Ile Thr Ile Asp Gly Lys Trp Gly Gly Thr Ser Gln Gly Ala Ile Asp
        115                 120                 125

Ala Leu Val Lys Ser Tyr Arg Gln Ile Thr Glu Ala Glu Arg Ala Gly
    130                 135                 140

Ser Thr Leu Pro Leu Gly Leu Ala Thr Val Met Ser Lys His Met Ser
145                 150                 155                 160

Ile Glu Gln Leu Arg Ala Met Leu Pro Thr Asp Arg Gln Gly Tyr Ala
                165                 170                 175

Glu Val Tyr Ile Asp Pro Leu Asn Glu Thr Met Asp Ile Phe Glu Ile
            180                 185                 190

Asn Thr Pro Leu Arg Ile Ala His Phe Met Ala Gln Ile Leu His Glu
        195                 200                 205

Thr Ala Cys Phe Lys Tyr Thr Glu Glu Leu Ala Ser Gly Lys Ala Tyr
    210                 215                 220

Glu Gly Arg Ala Asp Leu Gly Asn Thr Arg Pro Gly Asp Gly Pro Leu
225                 230                 235                 240

Phe Lys Gly Arg Gly Leu Leu Gln Ile Thr Gly Arg Leu Asn Tyr Val
                245                 250                 255

Lys Cys Gln Val Tyr Leu Arg Glu Lys Leu Lys Asp Pro Thr Phe Asp
            260                 265                 270

Ile Thr Ser Ser Val Thr Cys Ala Gln Gln Leu Ser Glu Ser Pro Leu
        275                 280                 285

Leu Ala Ala Leu Ala Ser Gly Tyr Phe Trp Arg Phe Ile Lys Pro Lys
    290                 295                 300

Leu Asn Glu Thr Ala Asp Lys Asp Asp Ile Tyr Trp Val Ser Val Tyr
305                 310                 315                 320

Val Asn Gly Tyr Ala Lys Gln Ala Asn Pro Tyr Tyr Pro Asn Arg Asp
                325                 330                 335

Lys Glu Pro Asn His Met Lys Glu Arg Val Gln Met Leu Ala Val Thr
            340                 345                 350

Lys Lys Ala Leu Gly Ile Val
        355

<210>  138
<211>  360
<212>  PRT
<213>  Artificial Sequence

<220>
<223>  Fusion Cathelicidin-BF-OBPgp279

<400>  138

Met Lys Phe Phe Arg Lys Leu Lys Lys Ser Val Lys Lys Arg Ala Lys
1               5                   10                  15

Glu Phe Phe Lys Lys Pro Arg Val Ile Gly Val Ser Ile Pro Phe Gly
            20                  25                  30

Ser Lys Asn Ser Glu Lys Asn Ala Ser Ile Ile Met Ser Ile Gln Arg
        35                  40                  45

Thr Leu Ala Ser Leu Ser Leu Tyr Gly Gly Arg Ile Asp Gly Leu Phe
    50                  55                  60

Gly Glu Lys Cys Arg Gly Ala Ile Ile Leu Met Leu Asn Lys Val Tyr

65                  70                  75                  80

Pro Asn Phe Ser Thr Asn Lys Leu Pro Ser Asn Thr Tyr Glu Ala Glu
                85                  90                  95

Ser Val Phe Thr Phe Leu Gln Thr Ala Leu Ala Gly Val Gly Leu Tyr
            100                 105                 110

Thr Ile Thr Ile Asp Gly Lys Trp Gly Gly Thr Ser Gln Gly Ala Ile
        115                 120                 125

Asp Ala Leu Val Lys Ser Tyr Arg Gln Ile Thr Glu Ala Glu Arg Ala
    130                 135                 140

Gly Ser Thr Leu Pro Leu Gly Leu Ala Thr Val Met Ser Lys His Met
145                 150                 155                 160

Ser Ile Glu Gln Leu Arg Ala Met Leu Pro Thr Asp Arg Gln Gly Tyr
                165                 170                 175

Ala Glu Val Tyr Ile Asp Pro Leu Asn Glu Thr Met Asp Ile Phe Glu
            180                 185                 190

Ile Asn Thr Pro Leu Arg Ile Ala His Phe Met Ala Gln Ile Leu His
        195                 200                 205

Glu Thr Ala Cys Phe Lys Tyr Thr Glu Glu Leu Ala Ser Gly Lys Ala
    210                 215                 220

Tyr Glu Gly Arg Ala Asp Leu Gly Asn Thr Arg Pro Gly Asp Gly Pro
225                 230                 235                 240

Leu Phe Lys Gly Arg Gly Leu Leu Gln Ile Thr Gly Arg Leu Asn Tyr
                245                 250                 255

Val Lys Cys Gln Val Tyr Leu Arg Glu Lys Leu Lys Asp Pro Thr Phe
            260                 265                 270

Asp Ile Thr Ser Ser Val Thr Cys Ala Gln Gln Leu Ser Glu Ser Pro
        275                 280                 285

Leu Leu Ala Ala Leu Ala Ser Gly Tyr Phe Trp Arg Phe Ile Lys Pro
    290                 295                 300

Lys Leu Asn Glu Thr Ala Asp Lys Asp Asp Ile Tyr Trp Val Ser Val
305                 310                 315                 320

Tyr Val Asn Gly Tyr Ala Lys Gln Ala Asn Pro Tyr Tyr Pro Asn Arg
                325                 330                 335

Asp Lys Glu Pro Asn His Met Lys Glu Arg Val Gln Met Leu Ala Val
            340                 345                 350

Thr Lys Lys Ala Leu Gly Ile Val
        355                 360

<210>  139
<211>  368
<212>  PRT
<213>  Artificial Sequence

<220>
<223>  Fusion Cathelicidin-BF-OBPgp279-His6

<400>  139

Met Lys Phe Phe Arg Lys Leu Lys Lys Ser Val Lys Lys Arg Ala Lys
1               5                   10                  15

Glu Phe Phe Lys Lys Pro Arg Val Ile Gly Val Ser Ile Pro Phe Gly
            20                  25                  30

Ser Lys Asn Ser Glu Lys Asn Ala Ser Ile Ile Met Ser Ile Gln Arg
        35                  40                  45

Thr Leu Ala Ser Leu Ser Leu Tyr Gly Gly Arg Ile Asp Gly Leu Phe
    50                  55                  60

Gly Glu Lys Cys Arg Gly Ala Ile Ile Leu Met Leu Asn Lys Val Tyr
65                  70                  75                  80

Pro Asn Phe Ser Thr Asn Lys Leu Pro Ser Asn Thr Tyr Glu Ala Glu
                85                  90                  95

Ser Val Phe Thr Phe Leu Gln Thr Ala Leu Ala Gly Val Gly Leu Tyr
            100                 105                 110

Thr Ile Thr Ile Asp Gly Lys Trp Gly Gly Thr Ser Gln Gly Ala Ile
        115                 120                 125

Asp Ala Leu Val Lys Ser Tyr Arg Gln Ile Thr Glu Ala Glu Arg Ala
    130                 135                 140

Gly Ser Thr Leu Pro Leu Gly Leu Ala Thr Val Met Ser Lys His Met
145                 150                 155                 160

Ser Ile Glu Gln Leu Arg Ala Met Leu Pro Thr Asp Arg Gln Gly Tyr

                165                 170                 175

Ala Glu Val Tyr Ile Asp Pro Leu Asn Glu Thr Met Asp Ile Phe Glu
            180                 185                 190

Ile Asn Thr Pro Leu Arg Ile Ala His Phe Met Ala Gln Ile Leu His
        195                 200                 205

Glu Thr Ala Cys Phe Lys Tyr Thr Glu Glu Leu Ala Ser Gly Lys Ala
    210                 215                 220

Tyr Glu Gly Arg Ala Asp Leu Gly Asn Thr Arg Pro Gly Asp Gly Pro
225                 230                 235                 240

Leu Phe Lys Gly Arg Gly Leu Leu Gln Ile Thr Gly Arg Leu Asn Tyr
                245                 250                 255

Val Lys Cys Gln Val Tyr Leu Arg Glu Lys Leu Lys Asp Pro Thr Phe
            260                 265                 270

Asp Ile Thr Ser Ser Val Thr Cys Ala Gln Gln Leu Ser Glu Ser Pro
        275                 280                 285

Leu Leu Ala Ala Leu Ala Ser Gly Tyr Phe Trp Arg Phe Ile Lys Pro
    290                 295                 300

Lys Leu Asn Glu Thr Ala Asp Lys Asp Asp Ile Tyr Trp Val Ser Val
305                 310                 315                 320

Tyr Val Asn Gly Tyr Ala Lys Gln Ala Asn Pro Tyr Tyr Pro Asn Arg
                325                 330                 335

Asp Lys Glu Pro Asn His Met Lys Glu Arg Val Gln Met Leu Ala Val
            340                 345                 350

Thr Lys Lys Ala Leu Gly Ile Val Leu Glu His His His His His His
        355                 360                 365

<210> 140
<211> 268
<212> PRT
<213> Artificial Sequence

<220>
<223> Fusion Cathelicidin-BF-PVP-SE1gp146 w/o methionine

<400> 140

Lys Phe Phe Arg Lys Leu Lys Lys Ser Val Lys Lys Arg Ala Lys Glu
1               5                   10                  15

Phe Phe Lys Lys Pro Arg Val Ile Gly Val Ser Ile Pro Phe Gly Ser
            20                  25                  30

Met Asn Ala Ala Ile Ala Glu Ile Gln Arg Met Leu Ile Glu Gly Gly
        35                  40                  45

Phe Ser Val Gly Lys Ser Gly Ala Asp Gly Leu Tyr Gly Pro Ala Thr
    50                  55                  60

Lys Ala Ala Leu Gln Lys Cys Ile Ala Gln Ala Thr Ser Gly Asn Asn
65                  70                  75                  80

Lys Gly Gly Thr Leu Lys Leu Thr Gln Ala Gln Leu Asp Lys Ile Phe
                85                  90                  95

Pro Val Gly Ala Ser Ser Gly Arg Asn Ala Lys Phe Leu Lys Pro Leu
            100                 105                 110

Asn Asp Leu Phe Glu Lys Thr Glu Ile Asn Thr Val Asn Arg Val Ala
        115                 120                 125

Gly Phe Leu Ser Gln Ile Gly Val Glu Ser Ala Glu Phe Arg Tyr Val
    130                 135                 140

Arg Glu Leu Gly Asn Asp Ala Tyr Phe Asp Lys Tyr Asp Thr Gly Pro
145                 150                 155                 160

Ile Ala Glu Arg Leu Gly Asn Thr Pro Gln Lys Asp Gly Asp Gly Ala
                165                 170                 175

Lys Tyr Lys Gly Arg Gly Leu Ile Gln Val Thr Gly Leu Ala Asn Tyr
            180                 185                 190

Lys Ala Cys Gly Lys Ala Leu Gly Leu Asp Leu Val Asn His Pro Glu
        195                 200                 205

Leu Leu Glu Gln Pro Glu Tyr Ala Val Ala Ser Ala Gly Trp Tyr Trp
    210                 215                 220

Asp Thr Arg Asn Ile Asn Ala Ala Cys Asp Ala Asp Asp Ile Val Lys
225                 230                 235                 240

Ile Thr Lys Leu Val Asn Gly Gly Thr Asn His Leu Ala Glu Arg Thr
                245                 250                 255

Ala Tyr Tyr Lys Lys Ala Lys Ser Val Leu Thr Ser

260                 265

<210>  141
<211>  269
<212>  PRT
<213>  Artificial Sequence

<220>
<223>  Fusion Cathelicidin-BF-PVP-SE1gp146

<400>  141

Met Lys Phe Phe Arg Lys Leu Lys Lys Ser Val Lys Lys Arg Ala Lys
1               5                   10                  15

Glu Phe Phe Lys Lys Pro Arg Val Ile Gly Val Ser Ile Pro Phe Gly
            20                  25                  30

Ser Met Asn Ala Ala Ile Ala Glu Ile Gln Arg Met Leu Ile Glu Gly
        35                  40                  45

Gly Phe Ser Val Gly Lys Ser Gly Ala Asp Gly Leu Tyr Gly Pro Ala
    50                  55                  60

Thr Lys Ala Ala Leu Gln Lys Cys Ile Ala Gln Ala Thr Ser Gly Asn
65                  70                  75                  80

Asn Lys Gly Gly Thr Leu Lys Leu Thr Gln Ala Gln Leu Asp Lys Ile
                85                  90                  95

Phe Pro Val Gly Ala Ser Ser Gly Arg Asn Ala Lys Phe Leu Lys Pro
            100                 105                 110

Leu Asn Asp Leu Phe Glu Lys Thr Glu Ile Asn Thr Val Asn Arg Val
        115                 120                 125

Ala Gly Phe Leu Ser Gln Ile Gly Val Glu Ser Ala Glu Phe Arg Tyr
    130                 135                 140

Val Arg Glu Leu Gly Asn Asp Ala Tyr Phe Asp Lys Tyr Asp Thr Gly
145                 150                 155                 160

Pro Ile Ala Glu Arg Leu Gly Asn Thr Pro Gln Lys Asp Gly Asp Gly
                165                 170                 175

Ala Lys Tyr Lys Gly Arg Gly Leu Ile Gln Val Thr Gly Leu Ala Asn
            180                 185                 190

Tyr Lys Ala Cys Gly Lys Ala Leu Gly Leu Asp Leu Val Asn His Pro
        195                 200                 205

Glu Leu Leu Glu Gln Pro Glu Tyr Ala Val Ala Ser Ala Gly Trp Tyr
    210                 215                 220

Trp Asp Thr Arg Asn Ile Asn Ala Ala Cys Asp Ala Asp Asp Ile Val
225                 230                 235                 240

Lys Ile Thr Lys Leu Val Asn Gly Gly Thr Asn His Leu Ala Glu Arg
                245                 250                 255

Thr Ala Tyr Tyr Lys Lys Ala Lys Ser Val Leu Thr Ser
            260                 265

<210>  142
<211>  277
<212>  PRT
<213>  Artificial Sequence

<220>
<223>  Fusion Cathelicidin-BF-PVP-SE1gp146-His6

<400>  142

Met Lys Phe Phe Arg Lys Leu Lys Lys Ser Val Lys Lys Arg Ala Lys
1               5                   10                  15

Glu Phe Phe Lys Lys Pro Arg Val Ile Gly Val Ser Ile Pro Phe Gly
            20                  25                  30

Ser Met Asn Ala Ala Ile Ala Glu Ile Gln Arg Met Leu Ile Glu Gly
        35                  40                  45

Gly Phe Ser Val Gly Lys Ser Gly Ala Asp Gly Leu Tyr Gly Pro Ala
    50                  55                  60

Thr Lys Ala Ala Leu Gln Lys Cys Ile Ala Gln Ala Thr Ser Gly Asn
65                  70                  75                  80

Asn Lys Gly Gly Thr Leu Lys Leu Thr Gln Ala Gln Leu Asp Lys Ile
                85                  90                  95

Phe Pro Val Gly Ala Ser Ser Gly Arg Asn Ala Lys Phe Leu Lys Pro
            100                 105                 110

Leu Asn Asp Leu Phe Glu Lys Thr Glu Ile Asn Thr Val Asn Arg Val
        115                 120                 125

Ala Gly Phe Leu Ser Gln Ile Gly Val Glu Ser Ala Glu Phe Arg Tyr
    130                 135                 140

Val Arg Glu Leu Gly Asn Asp Ala Tyr Phe Asp Lys Tyr Asp Thr Gly
145                 150                 155                 160

Pro Ile Ala Glu Arg Leu Gly Asn Thr Pro Gln Lys Asp Gly Asp Gly
                165                 170                 175

Ala Lys Tyr Lys Gly Arg Gly Leu Ile Gln Val Thr Gly Leu Ala Asn
            180                 185                 190

Tyr Lys Ala Cys Gly Lys Ala Leu Gly Leu Asp Leu Val Asn His Pro
        195                 200                 205

Glu Leu Leu Glu Gln Pro Glu Tyr Ala Val Ala Ser Ala Gly Trp Tyr
    210                 215                 220

Trp Asp Thr Arg Asn Ile Asn Ala Ala Cys Asp Ala Asp Asp Ile Val
225                 230                 235                 240

Lys Ile Thr Lys Leu Val Asn Gly Gly Thr Asn His Leu Ala Glu Arg
                245                 250                 255

Thr Ala Tyr Tyr Lys Lys Ala Lys Ser Val Leu Thr Ser Leu Glu His
            260                 265                 270

His His His His His
        275

<210>  143
<211>  164
<212>  PRT
<213>  Artificial Sequence

<220>
<223>  Fusion Lycotoxin1-S394 w/o methionine

<400>  143

Ile Trp Leu Thr Ala Leu Lys Phe Leu Gly Lys His Ala Ala Lys Lys
1               5                   10                  15

Leu Ala Lys Gln Gln Leu Ser Lys Leu Gly Ser Met Ser Phe Lys Phe
            20                  25                  30

Gly Lys Asn Ser Glu Lys Gln Leu Ala Thr Val Lys Pro Glu Leu Gln
        35                  40                  45

Lys Val Ala Arg Arg Ala Leu Glu Leu Ser Pro Tyr Asp Phe Thr Ile
    50                  55                  60

Val Gln Gly Ile Arg Thr Val Ala Gln Ser Ala Gln Asn Ile Ala Asn
65                  70                  75                  80

Gly Thr Ser Phe Leu Lys Asp Pro Ser Lys Ser Lys His Val Thr Gly
                85                  90                  95

Asp Ala Ile Asp Phe Ala Pro Tyr Ile Asn Gly Lys Ile Asp Trp Lys
            100                 105                 110

Asp Leu Glu Ala Phe Trp Ala Val Lys Lys Ala Phe Glu Gln Ala Gly
        115                 120                 125

Lys Glu Leu Gly Ile Lys Leu Arg Phe Gly Ala Asp Trp Asn Ser Ser
    130                 135                 140

Gly Asp Tyr His Asp Glu Ile Asp Arg Gly Thr Tyr Asp Gly Gly His
145                 150                 155                 160

Val Glu Leu Val

<210>  144
<211>  165
<212>  PRT
<213>  Artificial Sequence

<220>
<223>  Fusion Lycotoxin1-S394

<400>  144

Met Ile Trp Leu Thr Ala Leu Lys Phe Leu Gly Lys His Ala Ala Lys
1               5                   10                  15

Lys Leu Ala Lys Gln Gln Leu Ser Lys Leu Gly Ser Met Ser Phe Lys
            20                  25                  30

Phe Gly Lys Asn Ser Glu Lys Gln Leu Ala Thr Val Lys Pro Glu Leu
        35                  40                  45

Gln Lys Val Ala Arg Arg Ala Leu Glu Leu Ser Pro Tyr Asp Phe Thr
    50                  55                  60

Ile Val Gln Gly Ile Arg Thr Val Ala Gln Ser Ala Gln Asn Ile Ala
65                  70                  75                  80

Asn Gly Thr Ser Phe Leu Lys Asp Pro Ser Lys Ser Lys His Val Thr
                85                  90                  95

Gly Asp Ala Ile Asp Phe Ala Pro Tyr Ile Asn Gly Lys Ile Asp Trp

100                 105                 110

Lys Asp Leu Glu Ala Phe Trp Ala Val Lys Lys Ala Phe Glu Gln Ala
        115                 120                 125

Gly Lys Glu Leu Gly Ile Lys Leu Arg Phe Gly Ala Asp Trp Asn Ser
    130                 135                 140

Ser Gly Asp Tyr His Asp Glu Ile Asp Arg Gly Thr Tyr Asp Gly Gly
145                 150                 155                 160

His Val Glu Leu Val
                165

<210>  145
<211>  173
<212>  PRT
<213>  Artificial Sequence

<220>
<223>  Fusion Lycotoxin1-S394-His6

<400>  145

Met Ile Trp Leu Thr Ala Leu Lys Phe Leu Gly Lys His Ala Ala Lys
1               5                   10                  15

Lys Leu Ala Lys Gln Gln Leu Ser Lys Leu Gly Ser Met Ser Phe Lys
            20                  25                  30

Phe Gly Lys Asn Ser Glu Lys Gln Leu Ala Thr Val Lys Pro Glu Leu
        35                  40                  45

Gln Lys Val Ala Arg Arg Ala Leu Glu Leu Ser Pro Tyr Asp Phe Thr
    50                  55                  60

Ile Val Gln Gly Ile Arg Thr Val Ala Gln Ser Ala Gln Asn Ile Ala
65                  70                  75                  80

Asn Gly Thr Ser Phe Leu Lys Asp Pro Ser Lys Ser Lys His Val Thr
                85                  90                  95

Gly Asp Ala Ile Asp Phe Ala Pro Tyr Ile Asn Gly Lys Ile Asp Trp
            100                 105                 110

Lys Asp Leu Glu Ala Phe Trp Ala Val Lys Lys Ala Phe Glu Gln Ala
        115                 120                 125

Gly Lys Glu Leu Gly Ile Lys Leu Arg Phe Gly Ala Asp Trp Asn Ser
    130                 135                 140

Ser Gly Asp Tyr His Asp Glu Ile Asp Arg Gly Thr Tyr Asp Gly Gly
145                 150                 155                 160

His Val Glu Leu Val Leu Glu His His His His His His
                165                 170

<210>  146
<211>  364
<212>  PRT
<213>  Artificial Sequence

<220>
<223>  Fusion MSI-78-OBPgp279 w/o methionine

<400>  146

Gly Ile Gly Lys Phe Leu Lys Lys Ala Lys Lys Phe Gly Lys Ala Phe
1               5                   10                  15

Val Lys Ile Leu Lys Lys Gly Gly Gly Gly Ser Gly Ser Lys Asn Ser
            20                  25                  30

Glu Lys Asn Ala Ser Ile Ile Met Ser Ile Gln Arg Thr Leu Ala Ser
        35                  40                  45

Leu Ser Leu Tyr Gly Gly Arg Ile Asp Gly Leu Phe Gly Glu Lys Cys
    50                  55                  60

Arg Gly Ala Ile Ile Leu Met Leu Asn Lys Val Tyr Pro Asn Phe Ser
65                  70                  75                  80

Thr Asn Lys Leu Pro Ser Asn Thr Tyr Glu Ala Glu Ser Val Phe Thr
                85                  90                  95

Phe Leu Gln Thr Ala Leu Ala Gly Val Gly Leu Tyr Thr Ile Thr Ile
            100                 105                 110

Asp Gly Lys Trp Gly Gly Thr Ser Gln Gly Ala Ile Asp Ala Leu Val
        115                 120                 125

Lys Ser Tyr Arg Gln Ile Thr Glu Ala Glu Arg Ala Gly Ser Thr Leu
    130                 135                 140

Pro Leu Gly Leu Ala Thr Val Met Ser Lys His Met Ser Ile Glu Gln
145                 150                 155                 160

Leu Arg Ala Met Leu Pro Thr Asp Arg Gln Gly Tyr Ala Glu Val Tyr
                165                 170                 175

Ile Asp Pro Leu Asn Glu Thr Met Asp Ile Phe Glu Ile Asn Thr Pro
            180                 185                 190

Leu Arg Ile Ala His Phe Met Ala Gln Ile Leu His Glu Thr Ala Cys
        195                 200                 205

Phe Lys Tyr Thr Glu Glu Leu Ala Ser Gly Lys Ala Tyr Glu Gly Arg
    210                 215                 220

Ala Asp Leu Gly Asn Thr Arg Pro Gly Asp Gly Pro Leu Phe Lys Gly
225                 230                 235                 240

Arg Gly Leu Leu Gln Ile Thr Gly Arg Leu Asn Tyr Val Lys Cys Gln
                245                 250                 255

Val Tyr Leu Arg Glu Lys Leu Lys Asp Pro Thr Phe Asp Ile Thr Ser
            260                 265                 270

Ser Val Thr Cys Ala Gln Gln Leu Ser Glu Ser Pro Leu Leu Ala Ala
        275                 280                 285

Leu Ala Ser Gly Tyr Phe Trp Arg Phe Ile Lys Pro Lys Leu Asn Glu
    290                 295                 300

Thr Ala Asp Lys Asp Asp Ile Tyr Trp Val Ser Val Tyr Val Asn Gly
305                 310                 315                 320

Tyr Ala Lys Gln Ala Asn Pro Tyr Tyr Pro Asn Arg Asp Lys Glu Pro
                325                 330                 335

Asn His Met Lys Glu Arg Val Gln Met Leu Ala Val Thr Lys Lys Ala
            340                 345                 350

Leu Gly Ile Val Leu Glu His His His His His His
        355                 360

<210>  147
<211>  357
<212>  PRT
<213>  Artificial Sequence

<220>
<223>  Fusion MSI-78-OBPgp279

<400>  147

Met Gly Ile Gly Lys Phe Leu Lys Lys Ala Lys Lys Phe Gly Lys Ala
1               5                   10                  15

Phe Val Lys Ile Leu Lys Lys Gly Gly Gly Gly Ser Gly Ser Lys Asn
            20                  25                  30

Ser Glu Lys Asn Ala Ser Ile Ile Met Ser Ile Gln Arg Thr Leu Ala
        35                  40                  45

Ser Leu Ser Leu Tyr Gly Gly Arg Ile Asp Gly Leu Phe Gly Glu Lys
    50                  55                  60

Cys Arg Gly Ala Ile Ile Leu Met Leu Asn Lys Val Tyr Pro Asn Phe
65                  70                  75                  80

Ser Thr Asn Lys Leu Pro Ser Asn Thr Tyr Glu Ala Glu Ser Val Phe
                85                  90                  95

Thr Phe Leu Gln Thr Ala Leu Ala Gly Val Gly Leu Tyr Thr Ile Thr
            100                 105                 110

Ile Asp Gly Lys Trp Gly Gly Thr Ser Gln Gly Ala Ile Asp Ala Leu
        115                 120                 125

Val Lys Ser Tyr Arg Gln Ile Thr Glu Ala Glu Arg Ala Gly Ser Thr
    130                 135                 140

Leu Pro Leu Gly Leu Ala Thr Val Met Ser Lys His Met Ser Ile Glu
145                 150                 155                 160

Gln Leu Arg Ala Met Leu Pro Thr Asp Arg Gln Gly Tyr Ala Glu Val
                165                 170                 175

Tyr Ile Asp Pro Leu Asn Glu Thr Met Asp Ile Phe Glu Ile Asn Thr
            180                 185                 190

Pro Leu Arg Ile Ala His Phe Met Ala Gln Ile Leu His Glu Thr Ala
        195                 200                 205

Cys Phe Lys Tyr Thr Glu Glu Leu Ala Ser Gly Lys Ala Tyr Glu Gly
    210                 215                 220

Arg Ala Asp Leu Gly Asn Thr Arg Pro Gly Asp Gly Pro Leu Phe Lys
225                 230                 235                 240

Gly Arg Gly Leu Leu Gln Ile Thr Gly Arg Leu Asn Tyr Val Lys Cys
                245                 250                 255

Gln Val Tyr Leu Arg Glu Lys Leu Lys Asp Pro Thr Phe Asp Ile Thr
            260                 265                 270

Ser Ser Val Thr Cys Ala Gln Gln Leu Ser Glu Ser Pro Leu Leu Ala
        275                 280                 285

Ala Leu Ala Ser Gly Tyr Phe Trp Arg Phe Ile Lys Pro Lys Leu Asn
    290                 295                 300

Glu Thr Ala Asp Lys Asp Asp Ile Tyr Trp Val Ser Val Tyr Val Asn
305                 310                 315                 320

Gly Tyr Ala Lys Gln Ala Asn Pro Tyr Tyr Pro Asn Arg Asp Lys Glu
                325                 330                 335

Pro Asn His Met Lys Glu Arg Val Gln Met Leu Ala Val Thr Lys Lys
            340                 345                 350

Ala Leu Gly Ile Val
        355

<210>  148
<211>  365
<212>  PRT
<213>  Artificial Sequence

<220>
<223>  Fusion MSI-78-OBPgp279-His6

<400>  148

Met Gly Ile Gly Lys Phe Leu Lys Lys Ala Lys Lys Phe Gly Lys Ala
1               5                   10                  15

Phe Val Lys Ile Leu Lys Lys Gly Gly Gly Gly Ser Gly Ser Lys Asn
            20                  25                  30

Ser Glu Lys Asn Ala Ser Ile Ile Met Ser Ile Gln Arg Thr Leu Ala
        35                  40                  45

Ser Leu Ser Leu Tyr Gly Gly Arg Ile Asp Gly Leu Phe Gly Glu Lys
    50                  55                  60

Cys Arg Gly Ala Ile Ile Leu Met Leu Asn Lys Val Tyr Pro Asn Phe
65                  70                  75                  80

Ser Thr Asn Lys Leu Pro Ser Asn Thr Tyr Glu Ala Glu Ser Val Phe
                85                  90                  95

Thr Phe Leu Gln Thr Ala Leu Ala Gly Val Gly Leu Tyr Thr Ile Thr
            100                 105                 110

Ile Asp Gly Lys Trp Gly Gly Thr Ser Gln Gly Ala Ile Asp Ala Leu
        115                 120                 125

Val Lys Ser Tyr Arg Gln Ile Thr Glu Ala Glu Arg Ala Gly Ser Thr
    130                 135                 140

Leu Pro Leu Gly Leu Ala Thr Val Met Ser Lys His Met Ser Ile Glu
145                 150                 155                 160

Gln Leu Arg Ala Met Leu Pro Thr Asp Arg Gln Gly Tyr Ala Glu Val
                165                 170                 175

Tyr Ile Asp Pro Leu Asn Glu Thr Met Asp Ile Phe Glu Ile Asn Thr
            180                 185                 190

Pro Leu Arg Ile Ala His Phe Met Ala Gln Ile Leu His Glu Thr Ala
        195                 200                 205

Cys Phe Lys Tyr Thr Glu Glu Leu Ala Ser Gly Lys Ala Tyr Glu Gly
    210                 215                 220

Arg Ala Asp Leu Gly Asn Thr Arg Pro Gly Asp Gly Pro Leu Phe Lys
225                 230                 235                 240

Gly Arg Gly Leu Leu Gln Ile Thr Gly Arg Leu Asn Tyr Val Lys Cys
                245                 250                 255

Gln Val Tyr Leu Arg Glu Lys Leu Lys Asp Pro Thr Phe Asp Ile Thr
            260                 265                 270

Ser Ser Val Thr Cys Ala Gln Gln Leu Ser Glu Ser Pro Leu Leu Ala
        275                 280                 285

Ala Leu Ala Ser Gly Tyr Phe Trp Arg Phe Ile Lys Pro Lys Leu Asn
    290                 295                 300

Glu Thr Ala Asp Lys Asp Asp Ile Tyr Trp Val Ser Val Tyr Val Asn
305                 310                 315                 320

Gly Tyr Ala Lys Gln Ala Asn Pro Tyr Tyr Pro Asn Arg Asp Lys Glu
                325                 330                 335

Pro Asn His Met Lys Glu Arg Val Gln Met Leu Ala Val Thr Lys Lys
            340                 345                 350

Ala Leu Gly Ile Val Leu Glu His His His His His His
        355                 360                 365

<210>  149
<211>  408
<212>  DNA
<213>  Bacteriophage S394

<400>  149
agcttcaaat tcggcaaaaa cagcgaaaaa cagctggcaa ccgttaaacc ggaactgcag      60

aaagttgcac gtcgtgcact ggaactgagc ccgtatgatt ttaccattgt tcagggtatt     120

cgtaccgttg cacagagcgc acagaatatt gcaaatggca ccagctttct gaaagatccg     180

agcaaaagca aacatgttac cggtgatgca attgattttg caccgtatat taacggcaaa     240

atcgattgga aagatctgga agcattttgg gcagtgaaaa aagcatttga acaggcaggt     300

aaagaactgg gtattaaact gcgttttggt gcagattgga atagcagcgg tgattatcat     360

gatgaaattg atcgtggcac ctatgatggt ggtcatgttg aactggtt                  408


<210>  150
<211>  66
<212>  DNA
<213>  Artificial sequence

<220>
<223>  MSI-78 DNA

<400>  150
ggcattggca aatttctgaa aaaagcgaaa aaatttggca aagcgtttgt gaaaattctg      60

aaaaaa                                                                  66


<210>  151
<211>  90
<212>  DNA
<213>  Artificial Sequence

<220>
<223>  nucleic acid encoding Cathelicidin-BF peptide

<400>  151
aaattttttc gcaaactgaa aaaaagcgtg aaaaaacgcg cgaaagaatt ttttaaaaaa      60

ccgcgcgtga ttggcgtgag cattccgttt                                        90


<210>  152
<211>  777
<212>  DNA
<213>  Artificial Sequence

<220>
<223>  mutated KZ144 with C14S, C23S and C50S, without N-terminal
       methionine

<400>  152
aaagtattac gcaaaggcga taggggtgat gaggtaagtc aactccagac actcttaaat      60

ttaagtggct atgatgttgg aaagccagat ggtatttttg gaaataacac ctttaatcag     120

gtagttaaat ttcaaaaaga taatagtcta gatagtgatg gtattgtagg taagaatact 180

tgggctgaat tattcagtaa atattctcca cctattcctt ataaaactat ccctatgcca 240

actgcaaata aatcacgtgc agctgcaact ccagttatga atgcagtaga aaatgctact 300

ggcgttcgta gccagttgct actaacattt gcttctattg aatcagcatt cgattacgaa 360

ataaaagcta agacttcatc agctactggt tggttccaat tccttactgg aacatggaaa 420

acaatgattg aaaattatgg catgaagtat ggcgtactta ctgatccaac tggggcatta 480

cgtaaagatc cacgtataag tgctttaatg ggtgccgaac taattaaaga gaatatgaat 540

attcttcgtc ctgtccttaa acgtgaacca actgatactg atctttattt agctcacttc 600

tttgggcctg gtgcagcccg tcgtttcctg accactggcc agaatgaatt agctgctacc 660

catttcccaa aagaagctca ggcaaaccca tctatttttt ataacaaaga tgggtcacct 720

aaaaccattc aagaagttta taacttaatg gatggtaaag ttgcagcaca tagaaaa 777

<210> 153
<211> 531
<212> DNA
<213> Artificial Sequence

<220>
<223> Fusion MSI-78-GGGGS-S394-His6

<400> 153
catatgggca ttggcaaatt tctgaaaaaa gcgaaaaaat ttggcaaagc gtttgtgaaa 60

attctgaaaa aaggcggcgg cggcagcgga tccatgagct tcaaattcgg caaaaacagc 120

gaaaaacagc tggcaaccgt taaaccggaa ctgcagaaag ttgcacgtcg tgcactggaa 180

ctgagcccgt atgattttac cattgttcag ggtattcgta ccgttgcaca gagcgcacag 240

aatattgcaa atggcaccag ctttctgaaa gatccgagca aaagcaaaca tgttaccggt 300

gatgcaattg attttgcacc gtatattaac ggcaaaatcg attggaaaga tctggaagca 360

ttttgggcag tgaaaaaagc atttgaacag gcaggtaaag aactgggtat taaactgcgt 420

tttggtgcag attggaatag cagcggtgat tatcatgatg aaattgatcg tggcacctat 480

gatggtggtc atgttgaact ggttctcgag caccaccacc accaccactg a 531

<210> 154
<211> 906
<212> DNA
<213> Artificial sequence

<220>
<223> Fusion Cathelicidin-BF-KZ144(C14S, C23S, C50S)-His6

<400> 154
catatgaaat tttttcgcaa actgaaaaaa agcgtgaaaa aacgcgcgaa agaatttttt 60

aaaaaaccgc gcgtgattgg cgtgagcatt ccgtttggat ccaaagtatt acgcaaaggc 120

gataggggtg atgaggtaag tcaactccag acactcttaa atttaagtgg ctatgatgtt 180

ggaaagccag atggtatttt tggaaataac acctttaatc aggtagttaa atttcaaaaa 240

gataatagtc tagatagtga tggtattgta ggtaagaata cttgggctga attattcagt 300

aaatattctc cacctattcc ttataaaact atccctatgc caactgcaaa taaatcacgt 360

gcagctgcaa ctccagttat gaatgcagta gaaaatgcta ctggcgttcg tagccagttg 420

ctactaacat ttgcttctat tgaatcagca ttcgattacg aaataaaagc taagacttca 480

tcagctactg gttggttcca attccttact ggaacatgga aaacaatgat tgaaaattat 540

ggcatgaagt atggcgtact tactgatcca actggggcat tacgtaaaga tccacgtata 600

agtgctttaa tgggtgccga actaattaaa gagaatatga atattcttcg tcctgtcctt 660

aaacgtgaac caactgatac tgatctttat ttagctcact tctttgggcc tggtgcagcc 720

cgtcgtttcc tgaccactgg ccagaatgaa ttagctgcta cccatttccc aaaagaagct 780

caggcaaacc catctatttt ttataacaaa gatgggtcac ctaaaaccat tcaagaagtt 840

tataacttaa tggatggtaa agttgcagca catagaaaac tcgagcacca ccaccaccac 900

cactga 906